# EUROPEAN PATENT SPECIFICATION

(11) **EP 0 976 734 B1**
(45) Date of publication and mention of the grant of the patent: **28.09.2005**
(21) Application number: 99117934.2
(22) Date of filing: 26.02.1996
(51) Int. Cl.: C07D 211/68, C07D 211/78, C07D 309/16, C07D 309/28, C07D 335/02, A61K 31/435, A61K 31/35, A61K 31/38, C07C 233/52, A61K 31/16, A61K 31/55

(54) **Selective inhibitors of viral or bacterial neuraminidase**
Selektive Inhibitoren viraler oder bakterieller Neuraminidase
Inhibiteurs sélectifs de neuraminidase virale ou bactérienne

(30) Priority: 27.02.1995 US 395245; 06.06.1995 US 476946; 29.12.1995 US 580567
(43) Date of publication of application: 02.02.2000
(62) Divisional of application: 96912404.9
(73) Proprietor: GILEAD SCIENCES, INC., Foster City, CA 94404 (US)
(72) Inventor: Bischofberger, Norbert W., San Carlos, CA 94070 (US); Kim, Choung U., San Carlos, CA 94070 (US); Lew, Willard, San Mateo, CA 94402 (US); Liu, Hongtao, Foster City, CA 94404 (US); Williams, Matthew A., Foster City, CA 94404 (US)
(74) Representative: Kinzebach, Werner

(56) References cited:
- EP-A- 0 539 204
- WO-A-91/16320
- WO-A-92/06691
- WO-A-93/12105
- WO-A-95/20583
- WO-A-96/04265
- ITZSTEIN VON M ET AL: "Rational design of potent sialidase-based inhibitors of inluenza virus replication" NATURE, vol. 363, LONDON, page 418-423 XP000574955
- NISHIMURA Y ET AL: "SYNTHESIS OF 3-EPISIASTATIN B ANALOGUES HAVING ANTI-INFLUENZA VIRUS ACTIVITY" JOURNAL OF ANTIBIOTICS, vol. 46, no. 12, December 1993, page 1883-1889 XP002055958
- MACK H ET AL: "SYNTHESIS OF 6-THIOSIALIC ACIDS AND 6-THIO-N-ACETYL-D-NEURAMINIC ACID" TETRAHEDRON LETTERS, vol. 28, no. 2, 1987, pages 191-194, XP000645386
- NISHIMURA Y ET AL: "TOTALLY SYNTHETIC ANALOGUES OF SIASTATIN B III. TRIFLUOROACETAMIDE ANALOGUES HAVING INHIBITORY ACTIVITY FOR TUMOR METASTASIS" JOURNAL OF ANTIBIOTICS, vol. 47, no. 1, January 1994, page 101-107 XP002055959
- BAUMBERGER F ET AL: "Synthesis of new sialidase inhibitors, 6-amino-6-deoxysialic acids" HELVETICA CHIMICA ACTA, vol. 71, no. 2, 16 March 1988, pages 429-445, XP002127639

## Description

### Background of the Invention

### Field of the Invention

Neuraminidase (also known as sialidase, acylneuraminyl hydrolase, and EC 3.2.1.18) is an enzyme common among animals and a number of microorganisms. It is a glycohydrolase that cleaves terminal alpha-ketosidically linked sialic acids from glycoproteins, glycolipids and oligiosaccharides. Many of the microorganisms containing neuraminidase are pathogenic to man and other animals including fowl, horses, swine and seals. These pathogenic organisms include influenza virus.

Neuraminidase has been implicated in the pathogenicity of influenza viruses. It is thought to help the elution of newly synthesized virons from infected cells and assist in the movement of the virus (through its hydrolase activity) through the mucus of the respiratory tract.

### Brief Description of Related-Art

Itzstein, M. von et al.; "Nature", 363(6428):418-423 (1993), discloses the rational design of sialidase-based inhibitors of influenza virus replication.

Colman, P. M. et al.; International Patent Publication No. WO 92/06691 (Int. App. No. PCT/AU90/00501, publication date April 30,1992), Itzstein, L. M. von et al.; European Patent Publication No. 0 539 204 A1 (EP App. No. 92309684.6, publication date April 28, 1993), and Itzstein, L. M. von et al.; International Publication No. WO 91/16320 (Int. App. No. PCT/AU91/00161, publication date October 31, 1991) disclose compounds that bind neuraminidase and are asserted to exhibited antiviral activity *in vivo*.

### Objects of the Invention

A principal object of the invention is inhibition of viruses, in particular influenza viruses. In particular, an object is inhibition of glycolytic enzymes such as neuraminidase, in particular the selective inhibition of viral or bacterial neuraminidases.

An additional object of the invention is to provide neuraminidase inhibitors that have a retarded rate of urinary excretion, that enter into nasal or pulmonary secretions from the systemic circulation, that have sufficient oral bioavailability to be therapeutically effective, that possess elevated potency, that exhibit clinically acceptable toxicity profiles and have other desirable pharmacologic properties.

Another object is to provide improved and less costly methods for synthesis of neuraminidase inhibitors.

A still further object is to provide improved methods for administration of known and novel neuraminidase inhibitors.

An additional object is to provide compositions useful in preparing polymers, surfactants or immunogens and for use in other industrial processes and articles

These and other objects will be readily apparent to the ordinary artisan from consideration of the invention as a whole.

### Summary of the Invention

The invention provides the compounds of formula I: 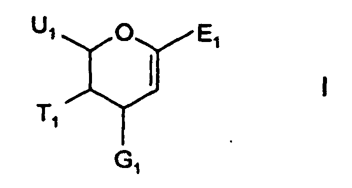 wherein
E₁ is COOH
G₁ is guanidino, amino, or guanidino or amino substituted with
C₁-C₆alkyl;
T₁ is -NHCOCH₃, -NHCOCH₂F, -NHCOCHF₂, or -NHCOCF₃;
U₁ is -O-CH₂CH(R₁)W₇;
W₇ is CH₂OR₁; and
R₁ is C₄-C₁₂alkyl;
and the salts, solvates, resolved enantiomers and purified diastereomers thereof.

In another embodiment of the invention a composition is provided that comprises a compound of formula I and a pharmaceutically-acceptable carrier.

Another embodiment of the invention is a method of inhibiting the activity of neuraminidase comprising the step of contacting a sample suspected of containing neuraminidase with a compound of the invention.

Another embodiment of the invention is the use of a compound of the invention for preparing a pharmaceutical composition for the treatment or prophylaxis of influenza infection.

### Stereoisomers

The compounds of the invention may be enriched or resolved optical isomers at any or all asymmetric atoms. For example, the chiral centers apparent from the depictions are provided as the chiral isomers or racemic mixtures. Both racemic and diasteromeric mixtures, as well as the individual optical isomers isolated or synthesized, substantially free of their enantiomeric or diastereomeric partners, are all within the scope of the invention. The racemic mixtures are separated into their individual, substantially optically pure isomers through well-known techniques such as, for example, the separation of diastereomeric salts formed with optically active adjuncts, e.g., acids or bases followed by conversion back to the optically active substances. In most instances, the desired optical isomer is synthesized by means of stereospecific reactions, beginning with the appropriate stereoisomer of the desired starting material.

The compounds of the invention can also exist as tautomeric isomers in case G₁ is guanidino (ene-amine tautomers). All their possible tautomeric forms are within the scope of the invention.

### Salts and Hydrates

The invention includes salts of the compounds herein, especially pharmaceutically acceptable non-toxic salts containing, for example, Na⁺, Li⁺, K⁺, Ca⁺⁺ and Mg⁺⁺. Such salts may include those derived by combination of appropriate cations such as alkali and alkaline earth metal ions or ammonium and quaternary amino ions with an acid anion moiety, typically the E₁ group carboxylic acid. Monovalent salts are preferred if a water soluble salt is desired.

Metal salts typically are prepared by reacting the metal hydroxide with a compound of this invention. Examples of metal salts which are prepared in this way are salts containing Li⁺, Na⁺, and K⁺. A less soluble metal salt can be precipitated from the solution of a more soluble salt by addition of the suitable metal compound.

In addition, salts may be formed from acid addition of certain organic and inorganic acids, e.g., HCl, HBr, H₂SO₄, or organic sulfonic acids, to basic centers, typically amines of group G₁, or to acidic groups such as E₁. Finally, it is to be understood that the invention includes compounds of formula I in their un-ionized, as well as zwitterionic form, and combinations with stoiochimetric amounts of water as in hydrates.

Also included within the scope of this invention are the salts of the parental compounds with one or more amino acids. Any of the amino acids described above are suitable, especially the naturally-occuring amino acids found as protein components, although the amino acid typically is one bearing a side chain with a basic or acidic group, e.g., lysine, arginine or glutamic acid, or a neutral group such as glycine, serine, threonine, alanine, isoleucine, or leucine.

### Methods of Inhibition of Neuraminidase.

Another aspect of the invention relates to methods of inhibiting the activity of neuraminidase comprising the step of treating a sample suspected of containing neuraminidase with a compound of the invention.

Compounds of the invention act as inhibitors of neuraminidase, as intermediates for such inhibitors or have other utilities as described below. The inhibitors will bind to locations on the surface or in a cavity of neuraminidase having a geometry unique to neuraminidase. Compounds binding neuraminidase may bind with varying degrees of reversibility. Those compounds binding substantially irreversibly are ideal candidates for use in this method of the invention. Once labeled, the substantially irreversibly binding compounds are useful as probes for the detection of neuraminidase. Accordingly, the invention relates to methods of detecting neuraminidase in a sample suspected of containing neuraminidase comprising the steps of: treating a sample suspected of containing neuraminidase with a composition comprising a compound of the invention bound to a label; and observing the effect of the sample on the activity of the label. Suitable labels are well known in the diagnostics field and include stable free radicals, fluorophores, radioisotopes, enzymes, chemiluminescent groups and chromogens. The compounds herein are labeled in conventional fashion using functional groups such as hydroxyl or amino.

Within the context of the invention samples suspected of containing neuraminidase include natural or man-made materials such as living organisms; tissue or cell cultures; biological samples such as biological material samples (blood, serum, urine, cerebrospinal fluid, tears, sputum, saliva, tissue samples, and the like); laboratory samples; food, water, or air samples; bioproduct samples such as extracts of cells, particularly recombinant cells synthesizing a desired glycoprotein; and the like. Typically the sample will be suspected of containing an organism which produces neuraminidase, frequently a pathogenic organism such as a virus. Samples can be contained in any medium including water and organic solvent\water mixtures. Samples include living organisms such as humans, and man made materials such as cell cultures.

The treating step of the invention comprises adding the compound of the invention to the sample or it comprises adding a precursor of the compound to the sample. The addition step comprises any method of administration as described above.

If desired, the activity of neuraminidase after application of the composition can be observed by any method including direct and indirect methods of detecting neuraminidase activity. Quantitative, qualitative, and semiquantitative methods of determining neuraminidase activity are all contemplated. Typically one of the screening methods described above are applied, however, any other method such as observation of the physiological properties of a living organism are also applicable.

Organisms that contain neuraminidase include bacteria (Vibrio cholerae, Clostridium perfringens, Streptococcus pneumoniae, and Arthrobacter sialophilus) and viruses (especially orthomyxoviruses or paramyxoviruses such as influenza virus A and B, parainfluenza virus, mumps virus, Newcastle disease virus, fowl plague virus, and sendai virus). Inhibition of neuraminidase activity obtained from or found within any of these organisms is within the objects of this invention. The virology of influenza viruses is described in "Fundamental Virology" (Raven Press, New York, 1986), Chapter 24. The compounds of this invention are useful in the treatment or prophylaxis of such infections in animals, e.g. duck, rodents, or swine, or in man.

However, in screening compounds capable of inhibiting influenza viruses it should be kept in mind that the results of enzyme assays may not correlate with cell culture assays, as shown Table 1 of Chandler et al., supra. Thus, a plaque reduction assay should be the primary screening tool.

### Screens for Neuraminidase Inhibitors.

Compounds of the invention are screened for inhibitory activity against neuraminidase by any of the conventional techniques for evaluating enzyme activity. Within the context of the invention, typically compounds are first screened for inhibition of neuraminidase *in vitro* and compounds showing inhibitory activity are then screened for activity *in vivo.* Compounds having *in vitro* Ki (inhibitory constants) of less then about 5 X 10⁻⁶ M, typically less than about 1 X 10⁻⁷ M and preferably less than about 5 X 10⁻⁸ M are preferred for *in vivo* use.

Useful *in vitro* screens have been described in detail and will not be elaborated here. However, Itzstein, M. von et al.; ''Nature'', 363(6428):418-423 (1993), in particular page 420, column 2, full paragraph 3, to page 421, column 2, first partial paragraph, describes a suitable *in vitro* assay of Potier, M.; et al.; "Analyt. Biochem.", 94:287-296 (1979), as modified by Chong, A.K.J.; et al.; "Biochem. Biophys. Acta", 1077:65-71 (1991); and Colman, P. M.; et al.; International Publication No. WO 92/06691 (Int. App. No. PCT/AU90/00501, publication date April 30, 1992) page 34, line 13, to page 35, line 16, describes another useful *in vitro* screen.

*In vivo* screens have also been described in detail, see Itzstein, M. von et al.; *op. cit.,* in particular page 421, column 2, first full paragraph, to page 423, column 2, first partial paragraph, and Colman, P. M.; et al.; *op. cit*. page 36, lines 1-38, describe suitable *in vivo* screens.

### Pharmaceutical Formulations and Routes of Administration.

The compounds of this invention are formulated with conventional carriers and excipients, which will be selected in accord with ordinary practice. Tablets will contain excipients, glidants, fillers, binders and the like. Aqueous formulations are prepared in sterile form, and when intended for delivery by other than oral administration generally will be isotonic. All formulations will optionally contain excipients such as those set forth in the "Handbook of Pharmaceutical Excipients" (1986). Excipients include ascorbic acid and other antioxidants, chelating agents such as EDTA, carbohydrates such as dextrin, hydroxyalkylcellulose, hydroxyalkylmethylcellulose, stearic acid and the like. The pH of the formulations ranges from about 3 to about 11, but is ordinarily about 7 to 10.

One or more compounds of the invention (herein referred to as the active ingredients) are administered by any route appropriate to the condition to be treated. Suitable routes include oral, rectal, nasal, topical (including buccal and sublingual), vaginal and parenteral (including subcutaneous, intramuscular, intravenous, intradermal, intrathecal and epidural), and the like. It will be appreciated that the preferred route may vary with for example the condition of the recipient. An advantage of the compounds of this invention is that they are orally bioavailable and can be dosed orally; it is not necessary to administer them by intrapulmonary or intranasal routes.

While it is possible for the active ingredients to be administered alone it may be preferable to present them as pharmaceutical formulations. The formulations, both for veterinary and for human use, of the invention comprise at least one active ingredient, as above defined, together with one or more acceptable carriers therefor and optionally other therapeutic ingredients. The carrier(s) must be "acceptable" in the sense of being compatible with the other ingredients of the formulation and physiologically innocuous to the recipient thereof.

The formulations include those suitable for the foregoing administration routes. The formulations may conveniently be presented in unit dosage form and may be prepared by any of the methods well known in the art of pharmacy. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the invention suitable for oral administration are prepared as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

A tablet is made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom.

For infections of the eye or other external tissues e.g. mouth and skin, the formulations are preferably applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc.), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG 400) and mixtures thereof. The topical formulations may desirably include a compound which enhances absorption or penetration of the active ingredient through the skin or other affected areas. Examples of such dermal penetration enhancers include dimethyl sulphoxide and related analogs.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations.

Emulgents and emulsion stabilizers suitable for use in the formulation of the invention include Tween® 60, Span® 80, cetostearyl alcohol, benzyl alcohol, myristyl alcohol, glyceryl mono-stearate and sodium lauryl sulfate.

The choice of suitable oils or fats for the formulation is based on achieving the desired cosmetic properties. The cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils are used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for intrapulmonary or nasal administration have a particle size for example in the range of 0.1 to 500 microns (including particle sizes in a range between 0.1 and 500 microns in increments microns such as 0.5, 1, 30 microns, 35 microns, etc.), which is administered by rapid inhalation through the nasal passage or by inhalation through the mouth so as to reach the alveolar sacs. Suitable formulations include aqueous or oily solutions of the active ingredient. Formulations suitable for aerosol or dry powder administration may be prepared according to conventional methods and may be delivered with other therapeutic agents such as compounds heretofore used in the treatment or prophylaxis of influenza A or B infections as described below.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The formulations are presented in unit-dose or multi-dose containers, for example sealed ampoules and vials, and may be stored in a freeze-dried (lyophilized) condition requiring only the addition of the sterile liquid carrier, for example water for injection, immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

It should be understood that in addition to the ingredients particularly mentioned above the formulations of this invention may include other agents conventional in the art having regard to the type of formulation in question, for example those suitable for oral administration may include flavoring agents.

The invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

Compounds of the invention are used to provide controlled release pharmaceutical formulations containing as active ingredient one or more compounds of the invention ("controlled release formulations") in which the release of the active ingredient are controlled and regulated to allow less frequency dosing or to improve the pharmacokinetic or toxicity profile of a given active ingredient.

Effective dose of active ingredient depends at least on the nature of the condition being treated, toxicity, whether the compound is being used prophylactically (lower doses) or against an active influenza infection, the method of delivery, and the pharmaceutical formulation, and will be determined by the clinician using conventional dose escalation studies. It can be expected to be from about 0.0001 to about 100 mg/kg body weight per day. Typically, from about 0.01 to about 10 mg/kg body weight per day. More typically, from about .01 to about 5 mg/kg body weight per day. More typically, from about .05 to about 0.5 mg/kg body weight per day. For example, for inhalation the daily candidate dose for an adult human of approximately 70 kg body weight will range from 1 mg to 1000 mg, preferably between 5 mg and 500 mg, and may take the form of single or multiple doses.

Active ingredients of the invention are also used in combination with other active ingredients. Such combinations are selected based on the condition to be treated, cross-reactivities of ingredients and pharmaco-properties of the combination. For example, when treating viral infections of the respiratory system, in particular influenza infection, the compositions of the invention are combined with antivirals (such as amantidine, rimantadine and ribavirin), mucolytics, expectorants, bronchialdilators, antibiotics, antipyretics, or analgesics. Ordinarily, antibiotics, antipyretics, and analgesics are administered together with the compounds of this invention.

### Metabolites of the Compounds of the Invention

Also falling within the scope of this invention are the *in vivo* metabolic products of the compounds described herein, to the extent such products are novel and unobvious over the prior art. Such products may result for example from the oxidation, reduction, hydrolysis, amidation, esterification and the like of the administered compound, primarily due to enzymatic processes. Accordingly, the invention includes novel and unobvious compounds produced by a process comprising contacting a compound of this invention with a mammal for a period of time sufficient to yield a metabolic product thereof. Such products typically are identified by preparing a radiolabelled (e.g. C¹⁴ or H³) compound of the invention, administering it parenterally in a detectable dose (e.g. greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g. by MS or NMR analysis. In general, analysis of metabolites is done in the same way as conventional drug metabolism studies well-known to those skilled in the art. The conversion products, so long as they are not otherwise found *in vivo,* are useful in diagnostic assays for therapeutic dosing of the compounds of the invention even if they possess no neuraminidase inhibitory activity of their own.

### Additional Uses for the Compounds of This Invention.

The compounds of this invention, or the biologically active substances produced from these compounds by hydrolysis or metabolism *in vivo,* are used as immunogens or for conjugation to proteins, whereby they serve as components of immunogenic compositions to prepare antibodies capable of binding specifically to the protein, to the compounds or to their metabolic products which retain immunologically recognized epitopes (sites of antibody binding). The immunogenic compositions therefore are useful as intermediates in the preparation of antibodies for use in diagnostic, quality control, or the like, methods or in assays for the compounds or their novel metabolic products. The compounds are useful for raising antibodies against otherwise non-immunogenic polypeptides, in that the compounds serve as haptenic sites stimulating an immune response that cross-reacts with the unmodified conjugated protein.

The hydrolysis products of interest include products of the hydrolysis of the protected acidic and basic groups discussed above. As noted above, the acidic or basic amides comprising immunogenic polypeptides such as albumin or keyhole limpet hemocyanin generally are useful as immunogens. The metabolic products described above may retain a substantial degree of immunological cross reactivity with the compounds of the invention. Thus, the antibodies of this invention will be capable of binding to the unprotected compounds of the invention without binding to the protected compounds; alternatively the metabolic products, will be capable of binding to the protected compounds and/or the metabolitic products without binding to the protected compounds of the invention, or will be capable of binding specifically to any one or all three. The antibodies desirably will not substantially cross-react with naturally-occurring materials. Substantial cross-reactivity is reactivity under specific assay conditions for specific analytes sufficient to interfere with the assay results.

The immunogens of this invention contain the compound of this invention presenting the desired epitope in association with an immunogenic substance. Within the context of the invention such association means covalent bonding to form an immunogenic conjugate (when applicable) or a mixture of non-covalently bonded materials, or a combination of the above. Immunogenic substances include adjuvants such as Freund's adjuvant, immunogenic proteins such as viral, bacterial, yeast, plant and animal polypeptides, in particular keyhole limpet hemocyanin, serum albumin, bovine thyroglobulin or soybean trypsin inhibitor, and immunogenic polysaccharides. Typically, the compound having the structure of the desired epitope is covalently conjugated to an immunogenic polypeptide or polysaccharide by the use of a polyfunctional (ordinarily bifunctional) cross-linking agent. Methods for the manufacture of hapten immunogens are conventional per se, and any of the methods used heretofore for conjugating haptens to immunogenic polypeptides or the like are suitably employed here as well, taking into account the functional groups on the precursors or hydrolytic products which are available for cross-linking and the likelihood of producing antibodies specific to the epitope in question as opposed to the immunogenic substance.

Typically the polypeptide is conjugated to a site on the compound of the invention distant from the epitope to be recognized.

The conjugates are prepared in conventional fashion. For example, the cross-linking agents N-hydroxysuccinimide, succinic anhydride or alkN=C=Nalk are useful in preparing the conjugates of this invention. The conjugates comprise a compound of the invention attached by a bond or a linking group of 1-100, typically, 1-25, more typically 1-10 carbon atoms to the immunogenic substance. The conjugates are separated from starting materials and by products using chromatography or the like, and then are sterile filtered and vialed for storage.

The compounds of this invention are cross-linked for example through any one or more of the following groups: a hydroxyl group of U₁; a carboxyl group of E₁; a carbon atom of U₁, E₁, G₁, or T₁, in substitution of H; and an amine group of G₁. Included within such compounds are amides of polypeptides where the polypeptide serves as an above-described R_{6c} or R_{6b} groups.

Animals are typically immunized against the immunogenic conjugates or derivatives and antisera or monoclonal antibodies prepared in conventional fashion.

The compounds of the invention are useful for maintaining the structural integrity of glycoproteins in recombinant cell culture, i.e., they are added to fermentations in which glycoproteins are being produced for recovery so as to inhibit neuraminidase-catalyzed cleavage of the desired glycoproteins. This is of particular value in the recombinant synthesis of proteins in heterologous host cells that may disadvantageously degrade the carbohydrate portion of the protein being synthesized.

The compounds of the invention are polyfunctional. As such they represent a unique class of monomers for the synthesis of polymers. By way of example and not limitation, the polymers prepared from the compounds of this invention include polyamides and polyesters.

The present compounds are used as monomers to provide access to polymers having unique pendent functionalities. The compounds of this invention are useful in homopolymers, or as comonomers with monomers which do not fall within the scope of the invention. Homopolymers of the compounds of this invention will have utility as cation exchange agents (polyesters or polyamides) in the preparation of molecular sieves (polyamides), textiles, fibers, films, formed articles and the like where the acid functionality E₁ is esterified to a hydroxyl group in U₁, for example, whereby the pendant basic group G₁ is capable of binding acidic functionalities such as are found in polypeptides whose purification is desired. Polyamides are prepared by cross-linking E₁ and G₁, with U₁ and the adjacent portion of the ring remaining free to function as a hydrophilic or hydrophobic affinity group, depending up the selection of the U₁ group. The preparation of these polymers from the compounds of the invention is conventional per se.

The compounds of the invention are also useful as a unique class of polyfunctional surfactants. Particularly when U₁ does not contain a hydrophilic substituent and is, for example, alkyl or alkoxy, the compounds have the properties of bi-functional surfactants. As such they have useful surfactant, surface coating, emulsion modifying, rheology modifying and surface wetting properties.

As polyfunctional compounds with defined geometry and carrying simultaneously polar and non-polar moieties, the compounds of the invention are useful as a unique class of phase transfer agents. By way of example and not limitation, the compounds of the invention are useful in phase transfer catalysis and liquid/liquid ion extraction (LIX).

The compounds of the invention optionally contain asymmetric carbon atoms in groups U₁, E₁, G₁, and T₁. As such, they are a unique class of chiral auxiliaries for use in the synthesis or resolution of other optically active materials. For example, a racemic mixture of carboxylic acids can be resolved into its component enantiomers by: 1) forming a mixture of diastereomeric esters or amides with a compound of the invention wherein U₁ is an asymmetric hydroxyalkane or amino alkane group; 2) separating the diastereomers; and 3) hydrolyzing the ester structure. Racemic alcohols are separated by ester formation with an acid group of E₁. Further, such a method can be used to resolve the compounds of the invention themselves if optically active acids or alcohols are used instead of racemic starting materials.

The compounds of this invention are useful as linkers or spacers in preparing affinity absorption matrices, immobilized enzymes for process control, or immunoassay reagents. The compounds herein contain a multiplicity of functional groups that are suitable as sites for cross-linking desired substances. For example, it is conventional to link affinity reagents such as hormones, peptides, antibodies, drugs, and the like to insoluble substrates. These insolublized reagents are employed in known fashion to absorb binding partners for the affinity reagents from manufactured preparations, diagnostic samples and other impure mixtures. Similarly, immobilized enzymes are used to perform catalytic conversions with facile recovery of enzyme. Bifunctional compounds are commonly used to link analytes to detectable groups in preparing diagnostic reagents.

Many functional groups in the compounds of this invention are suitable for use in cross-linking. For example, the carboxylic or phosphonic acid of group E₁ is used to form esters with alcohols or amides with amines of the reagent to be cross-linked. The G₁ sites substituted with OH, NHR₁, SH, azido (which is reduced to amino if desired before cross-linking), CN, NO₂, amino, guanidino, halo and the like are suitable sites. Suitable protection of reactive groups will be used where necessary while assembling the cross-linked reagent to prevent polymerization of the bifunctional compound of this invention. In general, the compounds here are used by linking them through carboxylic or phosphonic acid to the hydroxyl or amino groups of the first linked partner, then covalently bonded to the other binding partner through a T₁ or G₁ group. For example a first binding partner such as a steroid hormone is esterified to the carboxylic acid of a compound of this invention and then this conjugate is cross-linked through a G₁ hydroxyl to cyanogen bromide activated Sepaharose, whereby immobilized steroid is obtained. Other chemistries for conjugation are well known. See for example Maggio, "Enzyme-Immunoassay" (CRC, 1988, pp 71-135) and references cited therein.

As noted above, the therapeutically useful compounds of this invention in which the W₁, or G₁ carboxyl, hydroxyl or amino groups are protected are useful as oral or sustained release forms. In these uses the protecting group is removed *in vivo*, e.g., hydrolyzed or oxidized, so as to yield the free carboxyl, amino or hydroxyl. Suitable esters or amides for this utility are selected based on the substrate specificity of esterases and/or carboxypeptidases expected to be found within cells where precursor hydrolysis is desired. To the extent that the specificity of these enzymes is unknown, one will screen a plurality of the compounds of this invention until the desired substrate specificity is found. This will be apparent from the appearance of free compound or of antiviral activity. One generally selects amides or esters of the invention compound that are (i) not hydrolyzed or hydrolyzed comparatively slowly in the upper gut, (ii) gut and cell permeable and (iii) hydrolyzed in the cell cytoplasm and/or systemic circulation. Screening assays preferably use cells from particular tissues that are susceptible to influenza infection, e.g. the mucous membranes of the bronchopulmonary tract. Assays known in the art are suitable for determining *in vivo* bioavailability including intestinal lumen stability, cell permeation, liver homogenate stability and plasma stability assays. However, even if the ester, amide or other protected derivatives are not converted *in vivo* to the free carboxyl, amino or hydroxyl groups, they remain useful as chemical intermediates.

### Exemplary Methods of Making the Compounds of the Invention

The invention also relates to methods of making the compounds of the invention. The compounds are prepared by any of the applicable techniques of organic synthesis. Many such techniques are well known in the art. However, many of the known techniques are elaborated in "Compendium of Organic Synthetic Methods" (John Wiley & Sons, New York), Vol. 1, Ian T. Harrison and Shuyen Harrison, 1971; Vol. 2, Ian T. Harrison and Shuyen Harrison, 1974; Vol. 3, Louis S. Hegedus and Leroy Wade, 1977; Vol. 4, Leroy G. Wade, jr., 1980; Vol. 5, Leroy G. Wade, Jr., 1984; and Vol. 6, Michael B. Smith; as well as March, J., "Advanced Organic Chemistry, Third Edition", (John Wiley & Sons, New York, 1985), "Comprehensive Organic Synthesis. Selectivity, Strategy & Efficiency in Modem Organic Chemistry. In 9 Volumes", Barry M. Trost, Editor-in-Chief (Pergamon Press, New York, 1993 printing).

A number of exemplary methods for the preparation of the compounds of the invention are provided below. These methods are intended to illustrate the nature of such preparations are not intended to limit the scope of applicable methods.

Generally, the reaction conditions such as temperature, reaction time, solvents, workup procedures, and the like, will be those common in the art for the particular reaction to be performed. The cited reference material, together with material cited therein, contains detailed descriptions of such conditions. Typically the temperatures will be -100°C to 200°C, solvents will be aprotic or protic, and reaction times will be 10 seconds to 10 days. Workup typically consists of quenching any unreacted reagents followed by partition between a water/organic layer system (extraction) and separating the layer containing the product.

Oxidation and reduction reactions are typically carried out at temperatures near room temperature (about 20°C), although for metal hydride reductions frequently the temperature is reduced to 0°C to -100°C, solvents are typically aprotic for reductions and may be either protic or aprotic for oxidations. Reaction times are adjusted to achieve desired conversions.

Condensation reactions are typically carried out at temperatures near room temperature, although for non-equilibrating, kinetically controlled condensations reduced temperatures (0°C to -100°C) are also common. Solvents can be either protic (common in equilibrating reactions) or aprotic (common in kinetically controlled reactions).

Standard synthetic techniques such as azeotropic removal of reaction by-products and use of anhydrous reaction conditions (e.g. inert gas environments) are common in the art and will be applied when applicable.

One exemplary method of preparing the compounds of the invention is shown in **Scheme 1** below by reference. A detailed description of the methods is found in the Experimental section below.

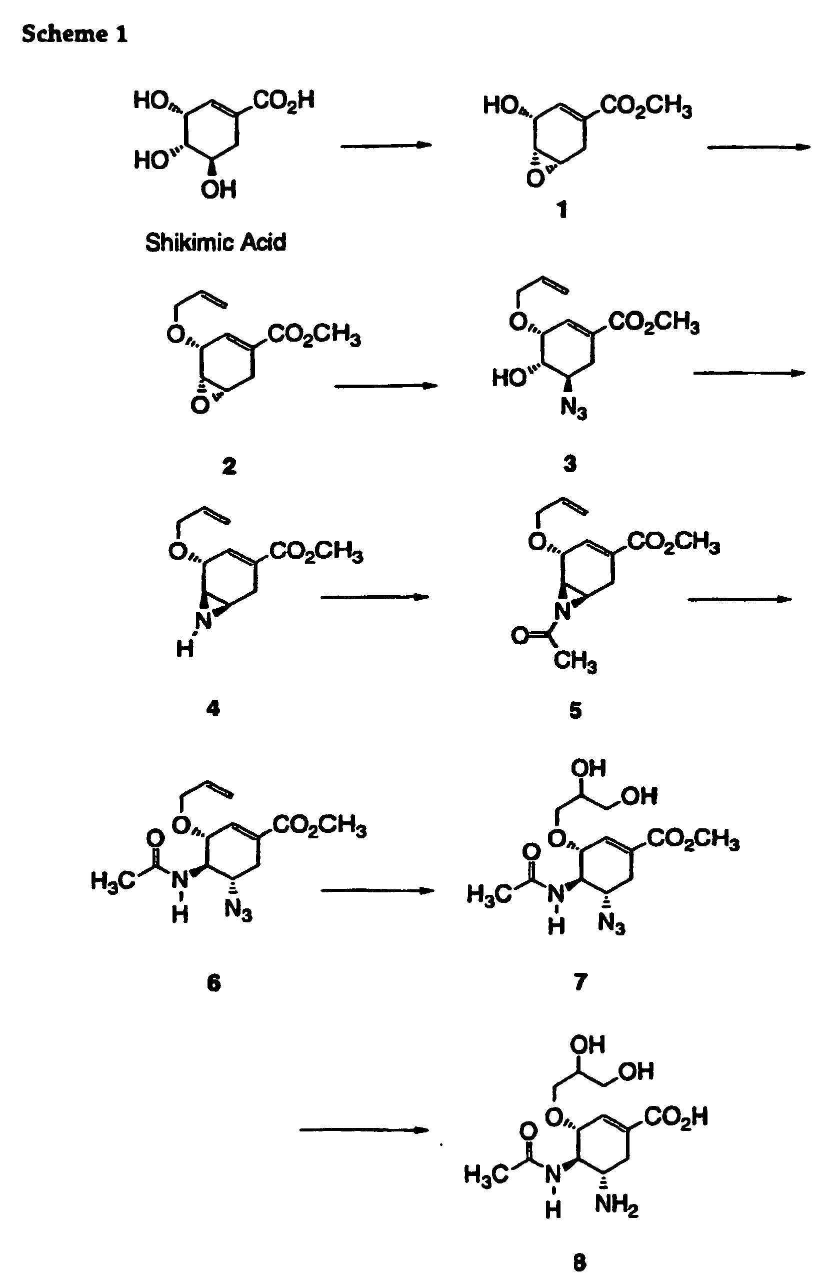

Modifications of Scheme **1** to form additional embodiments is shown in **Schemes 2-4**.

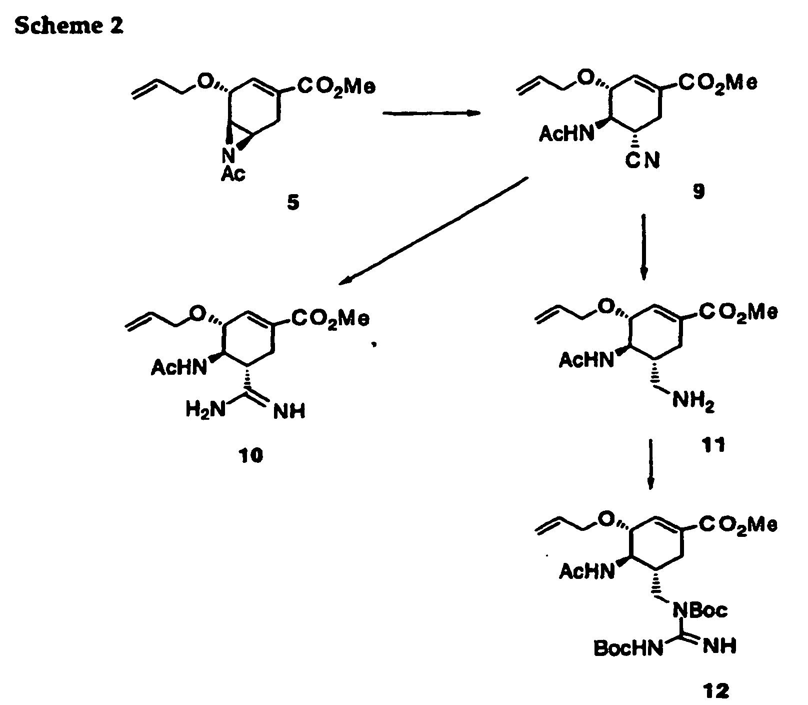

### Scheme 2

Aziridine **5** is converted to the amino nitrile **9** by Yb(CN)₃ catalyzed addition of TMSCN according to the procedure of Utimoto and co-workers, "Tetrahedron Lett.", 31:6379 (1990).

Conversion of nitrile **9** to the corresponding amidine **10** is accomplished using a standard three step sequence: i) H₂S; ii) CH₃I; iii) NH₄OAc. A typical conversion is found in "J. Med. Chem.", 36:1811 (1993).

Nitrile **9** is converted to the amino methyl compound **11** by reduction using any of the available methods found in "Modern Synthetic Reactions" 2nd ed. H.O. House, Benjamin/Cummings Publishing Co., 1972..

Amino methyl compound **11** is converted to the bis-Boc protected guanidino compound **12** by treating **11** with N,N'-bis-Boc-1H-pyrazole-1-carboxamidine according to the method found in "Tetrahedron Lett.", 36:299 (1995).

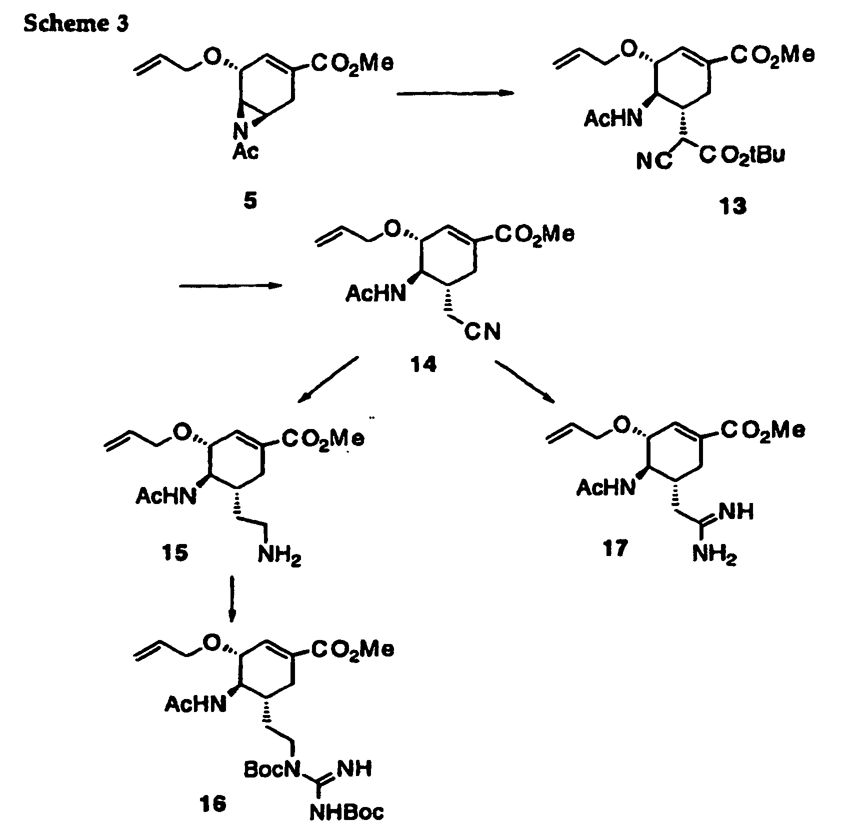

### Scheme 3

The aziridine **5** is opened with α-cyano acetic acid t-butyl ester to give **13**. Aziridine openings of this type are found in "Tetrahedron Lett.", 23:5021 (1982). Selective hydrolysis of the t-butyl ester moiety under acidic condtions followed by decarboxylation gives nitrile **14**.

Reduction of **14** to the amino ethyl derivative **15** is accomplished in the same fashion as the conversion of **9** to **11.** The amine **15** is then converted into the guanidino derivative **16** with N,N'-bis-Boc-1H-pyrazole-1-carboxamidine according to the method found in "Tetrahedron Lett.", 36:299 (1995).

The nitrile **14** is converted to the corresponding amidine **17** using the same sequence described above for the conversion of **9** to **10.**

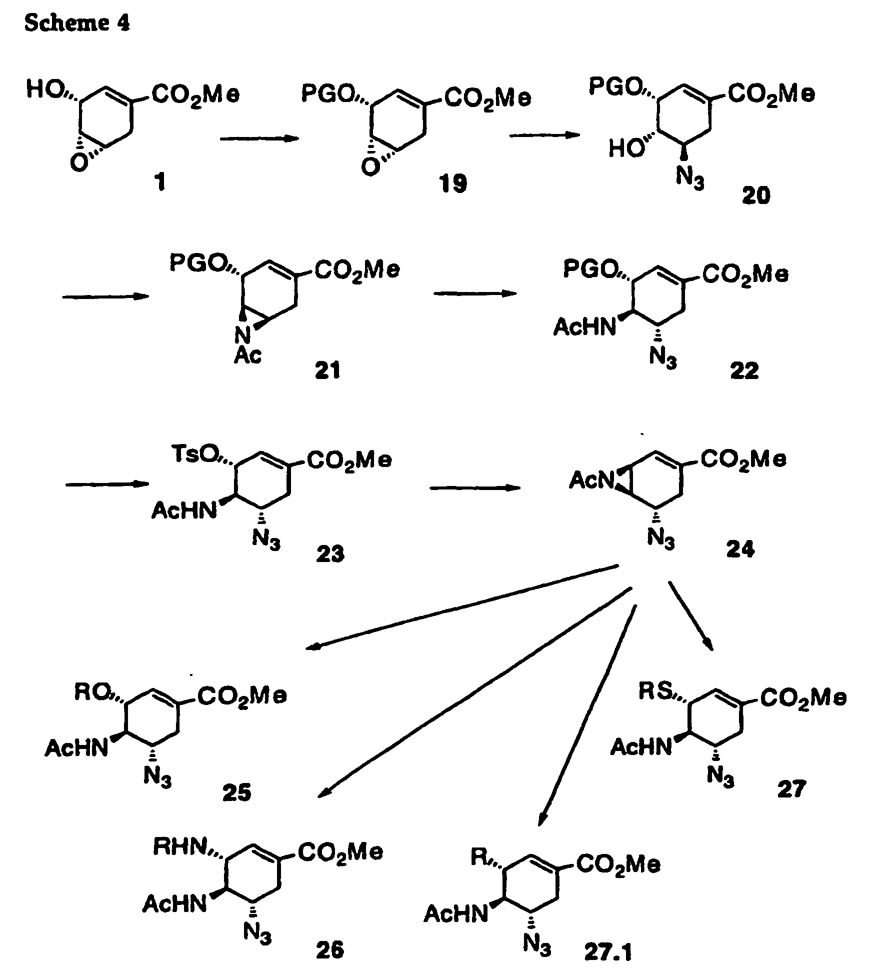

### Scheme 4

The epoxy alcohol **1** is protected (PG=protecting group), for example with MOMCl. Typical conditions are found in "Protective Groups in Organic Synthesis" 2nd ed.,T.W. Greene and P.G.M. Wuts, John Wiley & Sons, New York, NY, 1991.

The epoxide **19** is opened with NaN₃/NH₄Cl to the amino alcohol **20** according to the procedure of Sharpless and co-workers, "J. Org. Chem.", 50:1557 (1985).

Reduction of **20** to the N-acetyl aziridine **21** is accomplished in a three step sequence: 1) MsCl/triethyl amine; 2) H₂/Pd; 3) AcCl/pyridine. Such transformations can be found in "Angew. Chem. Int. Ed. Engl.", 33:599 (1994).

Aziridine **21** is converted to the azido amide **22** by opening with NaN₃/NH₄Cl in DMF at 65 °C as described in "J. Chem. Soc. Perkin Trans I", 801 (1976).

Removal of the MOM protecting group of **22** is accomplished using the methods described in "Protective Groups in Organic Synthesis" 2nd ed.,T.W. Greene and P.G.M. Wuts, John Wiley & Sons, New York, NY, 1991. The resulting alcohol is converted directly to aziridine **24** with TsCl in pyridine. Such transformations are found in "Angew. Chem. Int. Ed. Engl.", 33:599 (1994).

Aziridine **24** is then reacted with ROH, RNH₂, RSH or an organometallic (metal-R) to give the corresponding ring opened derivatives **25, 26, 27** and **27.1** respectively. Aziridine openings of this type are found in "Tetrahedron Lett.", 23:5021 (1982) and "Angew. Chem. Int. Ed. EngL", 33:599 (1994).

### Scheme 5

Another class of compounds of the invention are prepared by the method of **Schemes 5a** and **5b.** Quinic acid is converted to **28** by the method of Shing, T.K.M.; et al.; "Tetrahedron", 47(26):4571 (1991). Mesylation with MsCl in TEA/CH₂Cl₂ will give **29** which is reacted with NaN₃ in DMF to give **30**. Reaction of **30** with TFA in CH₂Cl₂ will give **31** which is mesylated with MsCl in TEA/CH₂Cl₂ to give **32**. Reaction with triphenylphosphine in water will give **33** which is converted to **35** by sequential application of: 1) CH₃C(O)Cl in pyridine, 2) NaN₃ in DMF, and 3) NaH in THF. Alkylation of **35** with a wide variety of nucleophiles common in the art will provide a number of compounds such as **36**. Methods for elaboration of the compounds such as **36** to other embodiments of the invention will be similar to those described above.

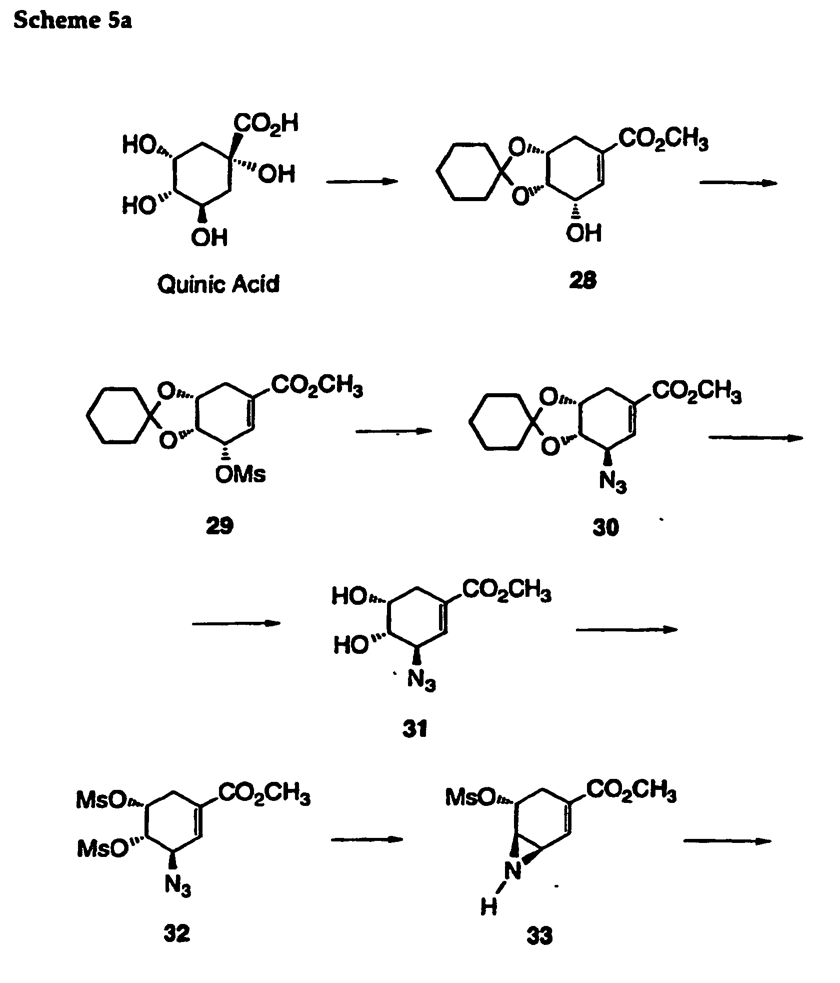

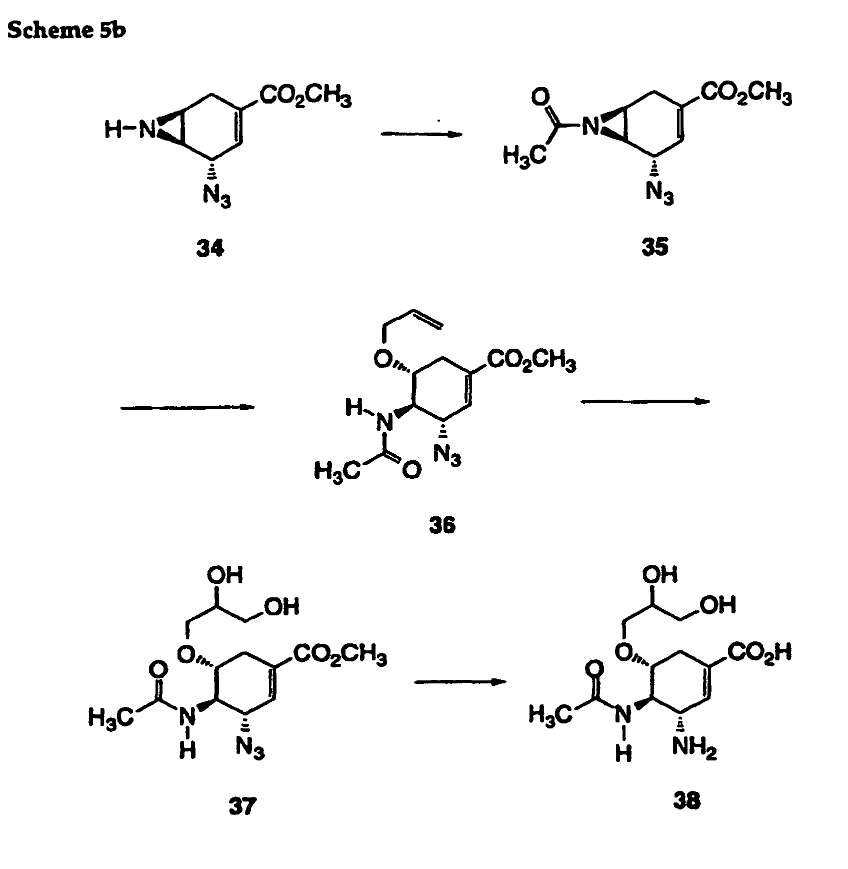

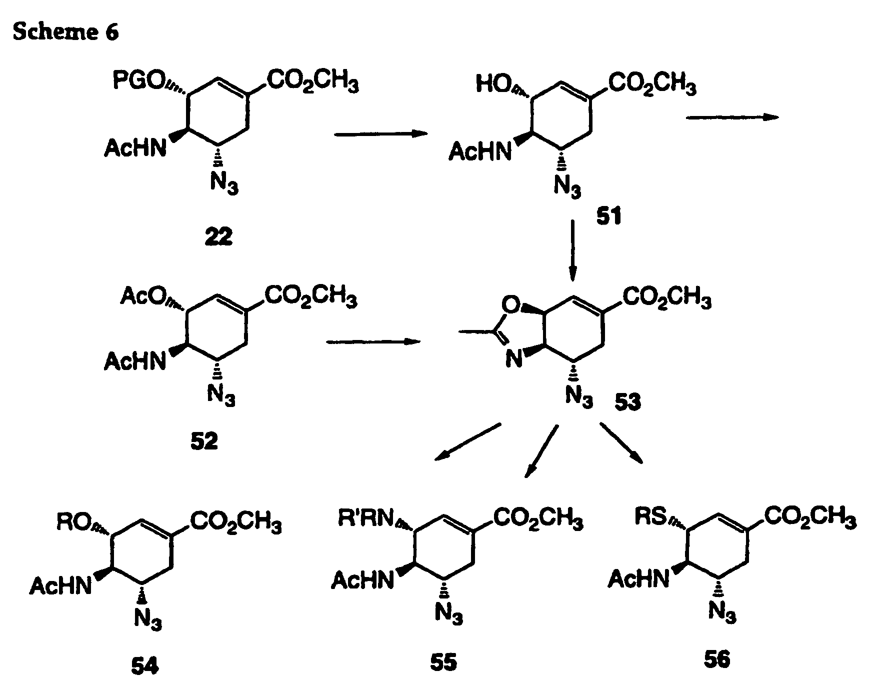

### Scheme 6

Another class of compounds of the invention are prepared by the method of **Scheme 6**. Protected alcohol **22** (PG=methoxymethyl ether) is deprotected under standard conditions described in "Protective Groups in Organic Synthesis" 2nd ed., T.W. Greene and P.G.M. Wuts, John Wiley & Sons, New York, NY, 1991. Alcohol **51** is converted to acetate **52** with acetic anhydride and pyridine under standard conditions. Acetate **52** is treated with TMSOTf or BF₃•OEt to afford oxazoline **53.** Such transformations are described in "Liebigs Ann. Chem.", 129 (1991) and "Carbohydrate Research", 181 (1993), respectively. Alternatively, alcohol **51** is transformed to oxazoline **53** by conversion to the corresponding mesylate or tosylate **23** and subsequently cyclized to the oxazoline under standard conditions, as described in "J. Org. Chem.", 50:1126 (1985) and "J. Chem. Soc.", 1385 (1970). Oxazoline **53** is reacted with ROH, RR'NH, or RSH (wherein R and R' are selected to be consistent with the definition of W₆ above) provide the corresponding ring opened derivatives **54, 55,** and **56** respectively. Such transformations are described in "J. Org. Chem.", 49:4889 (1984) and "Chem. Rev.", 71:483 (1971).

### Schemes 7-35

Other exemplary methods of preparing the compounds of the invention are shown in **Schemes 7-35** below. A detailed description of the methods is found in the Experimental section below.

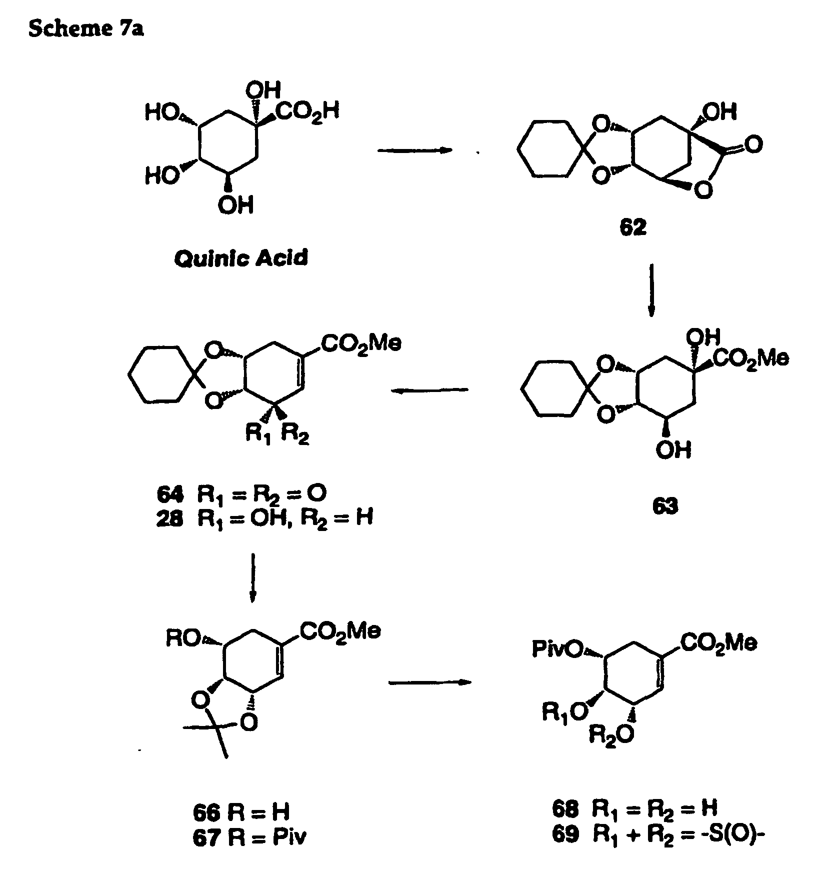

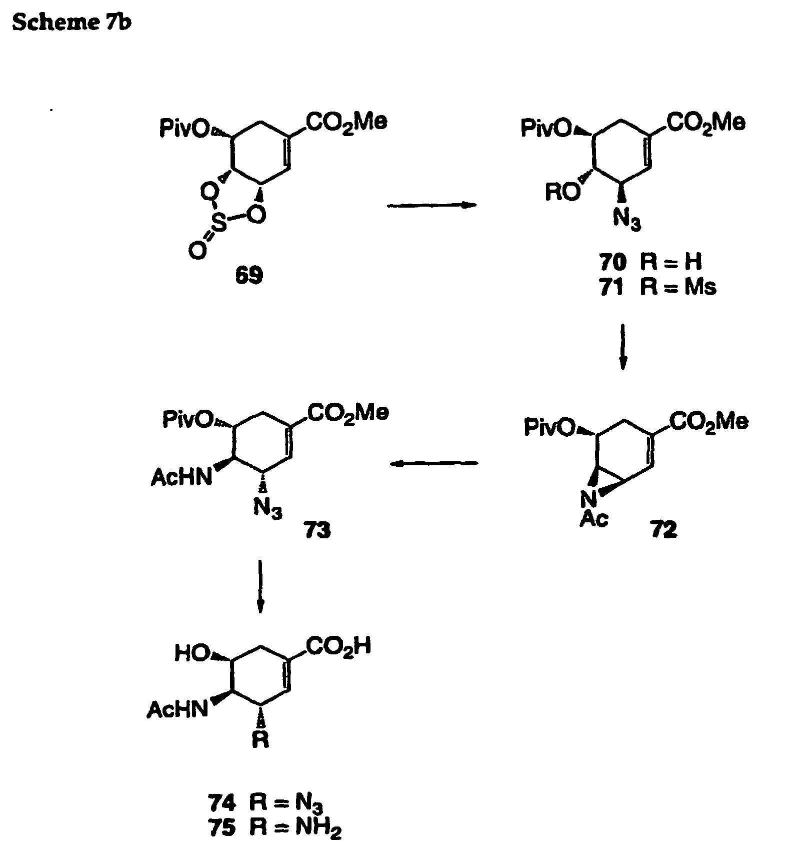

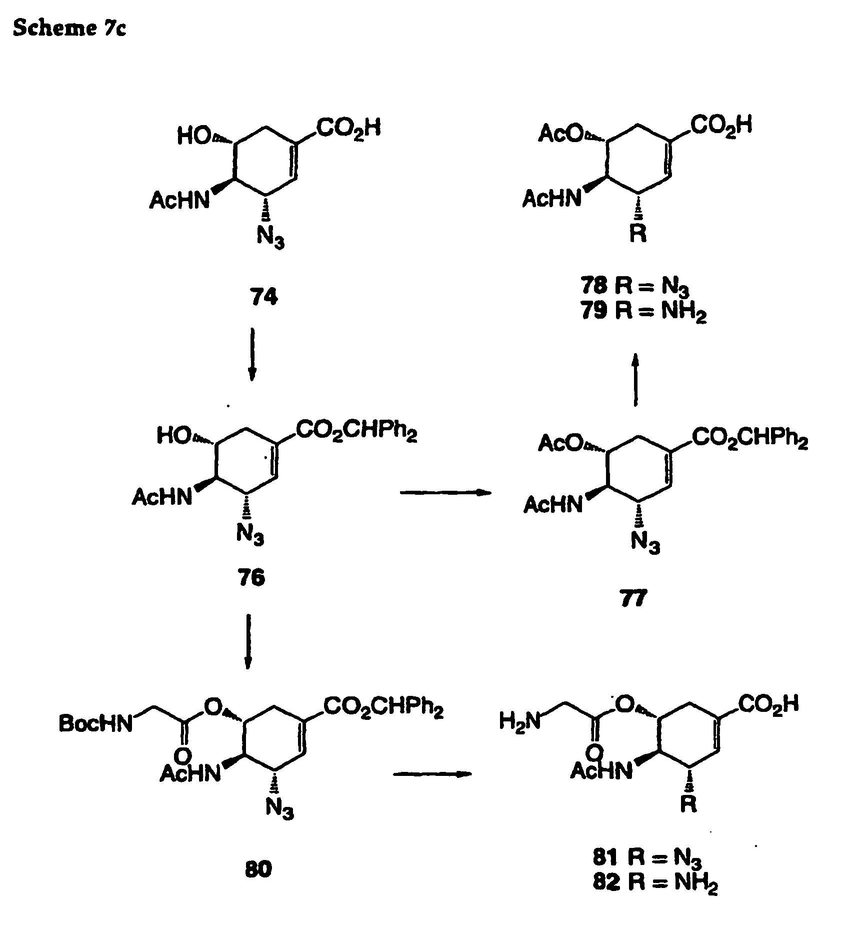

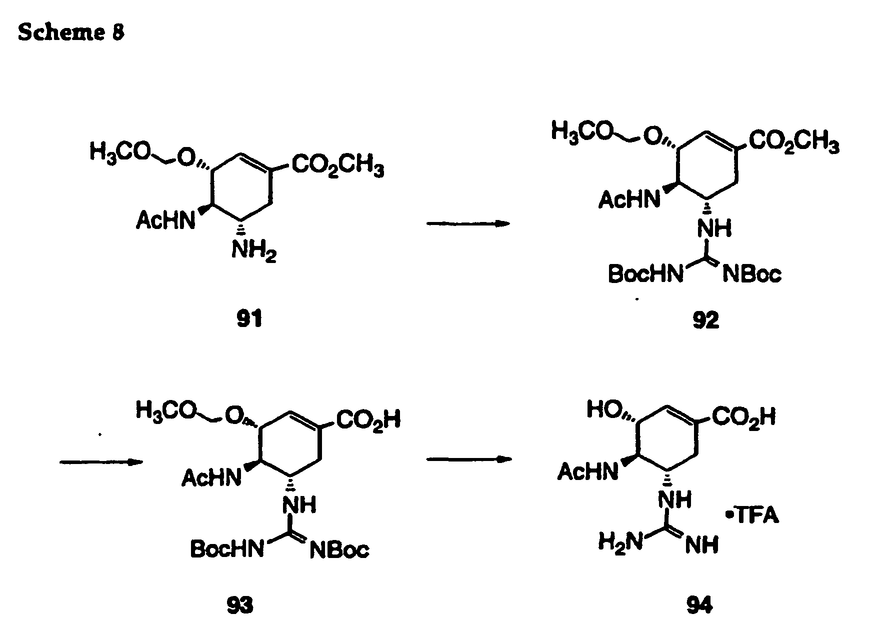

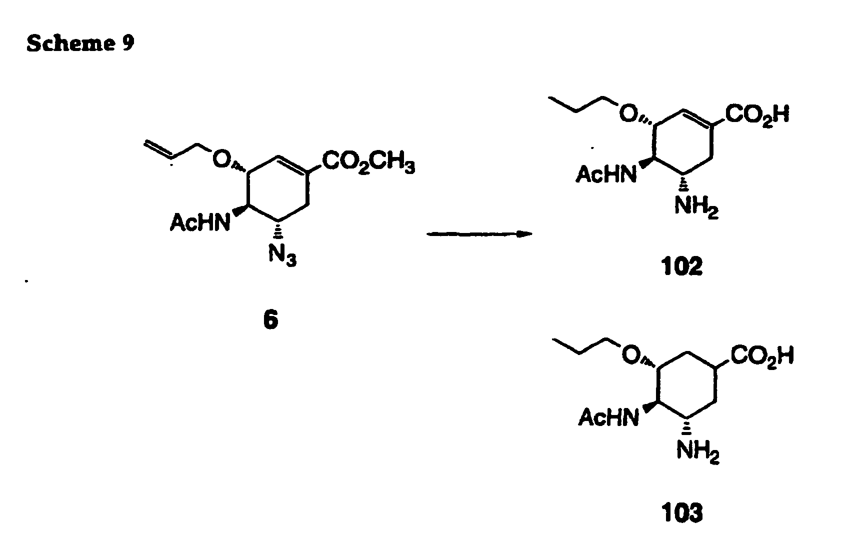

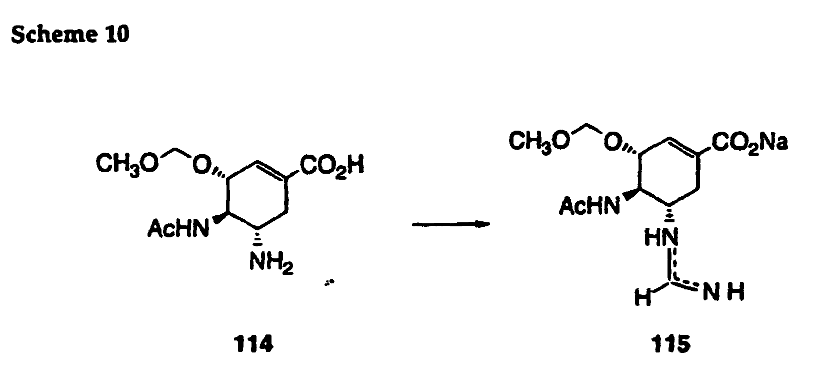

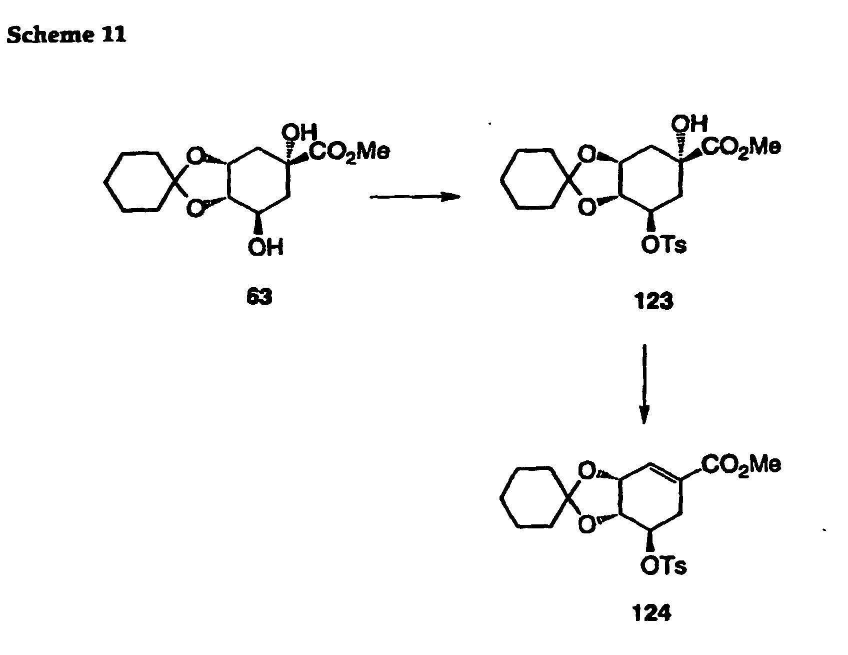

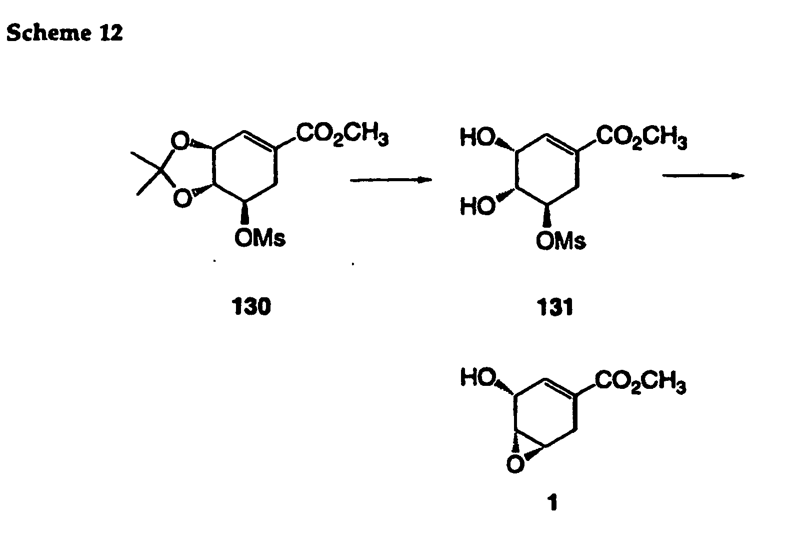

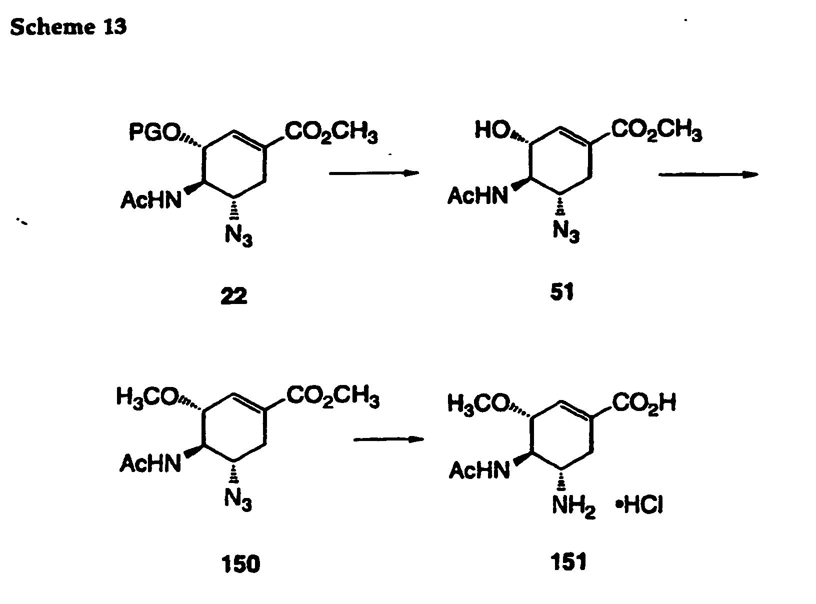

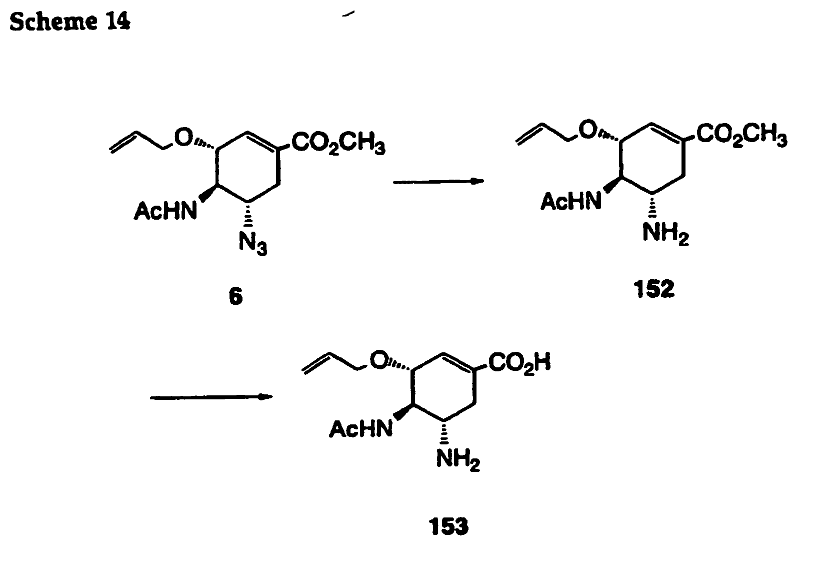

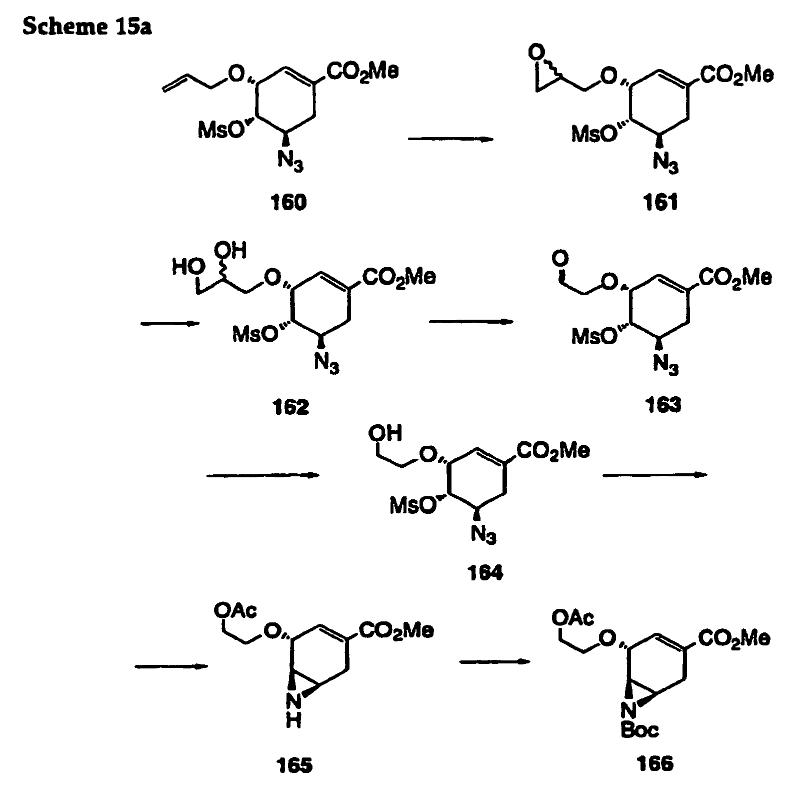

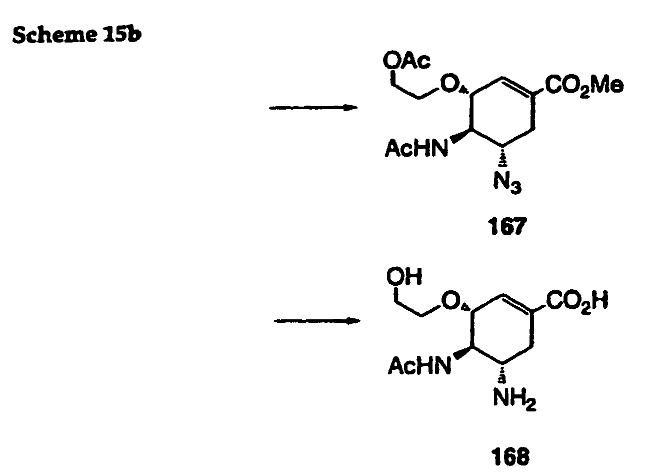

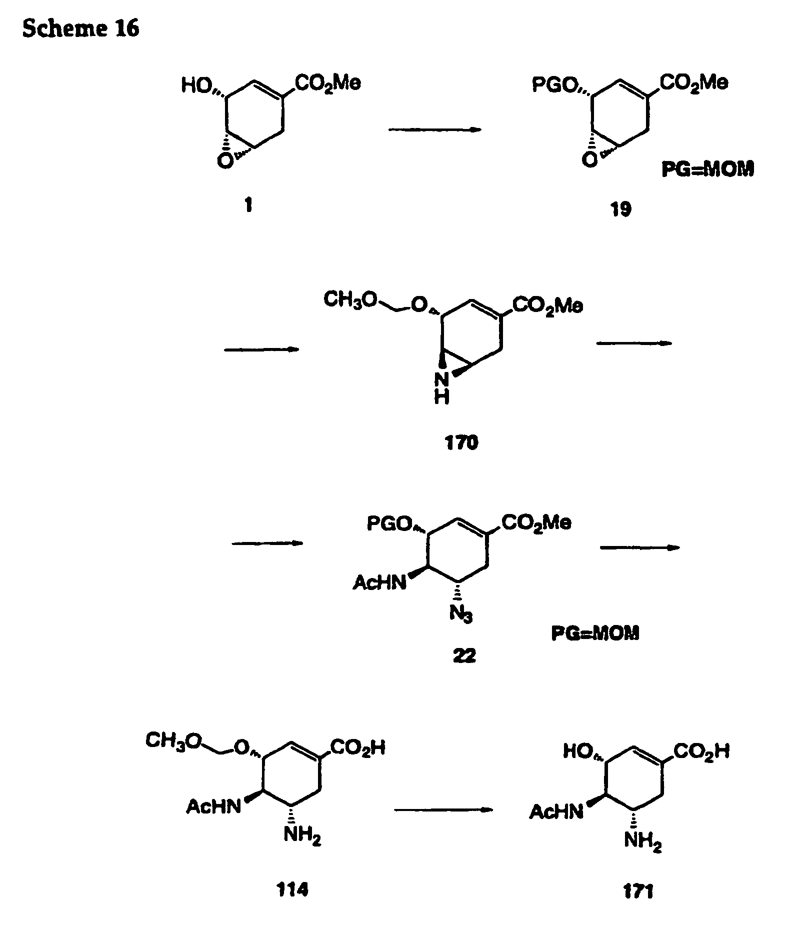

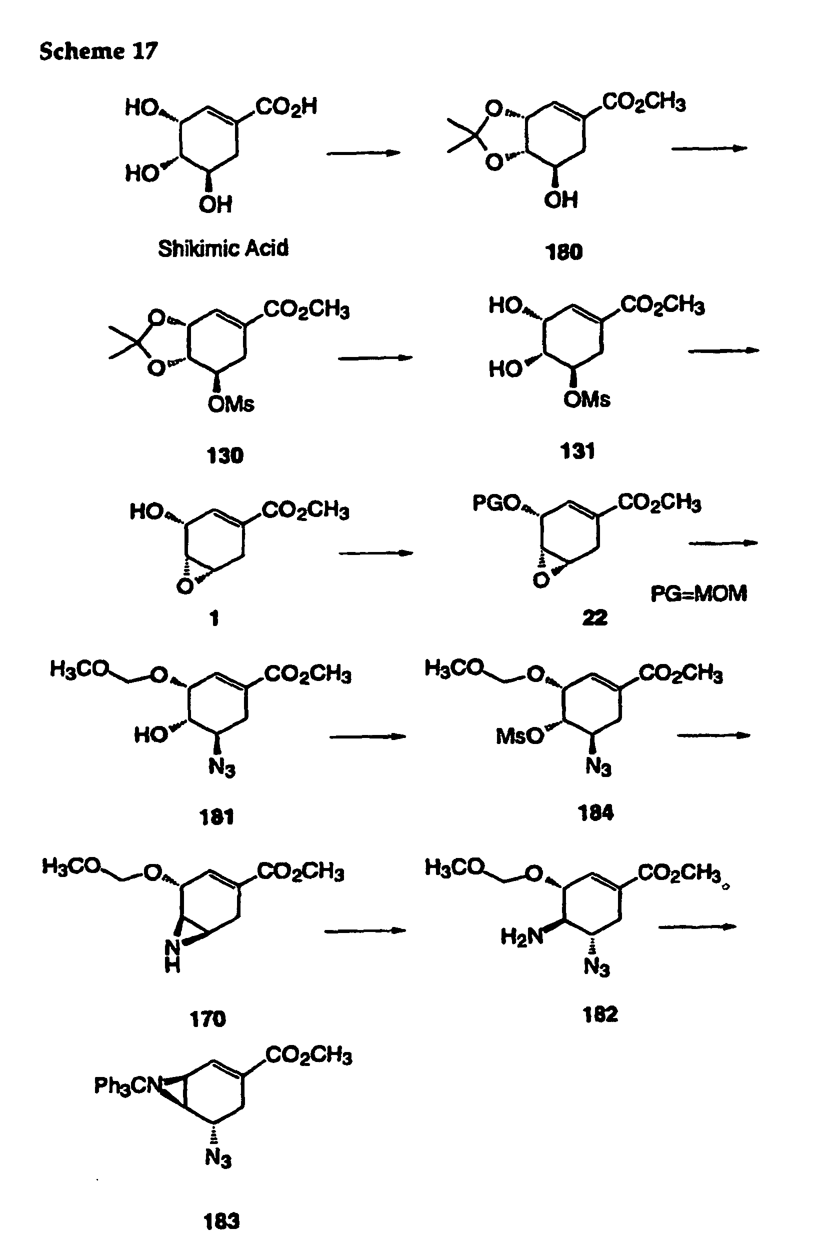

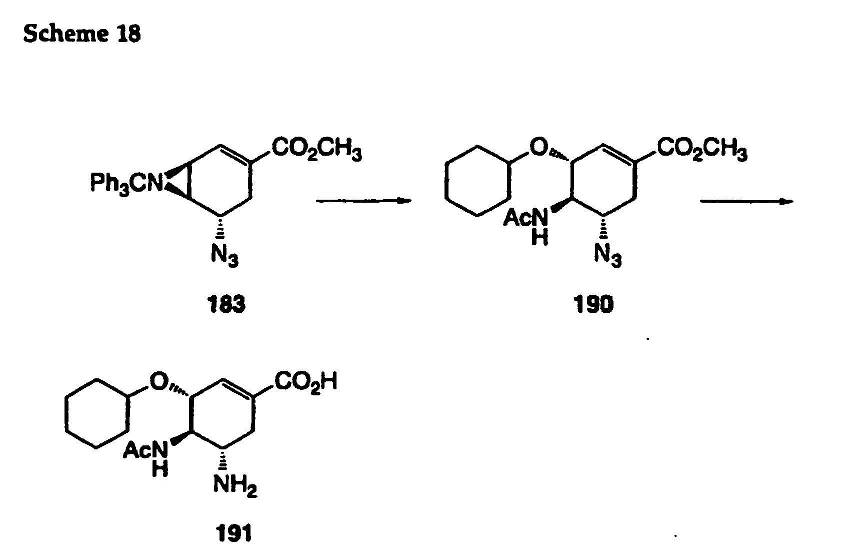

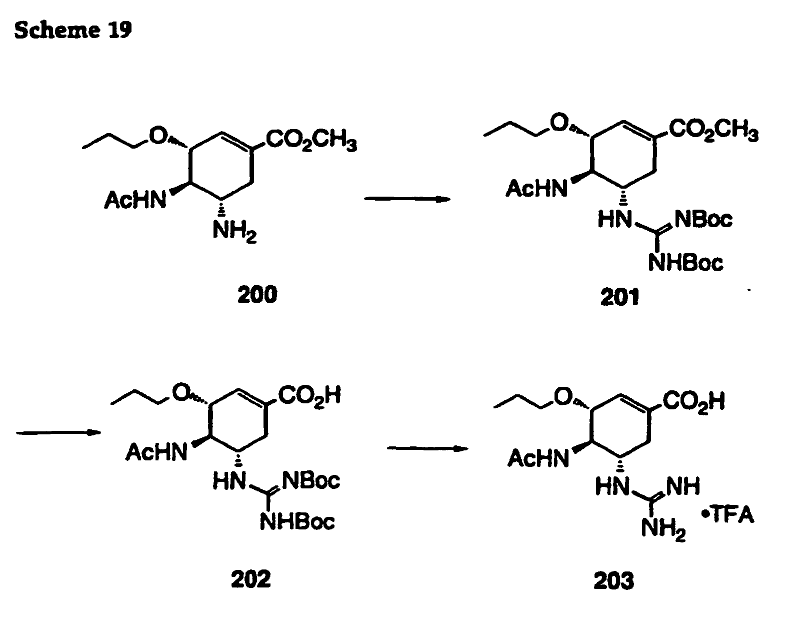

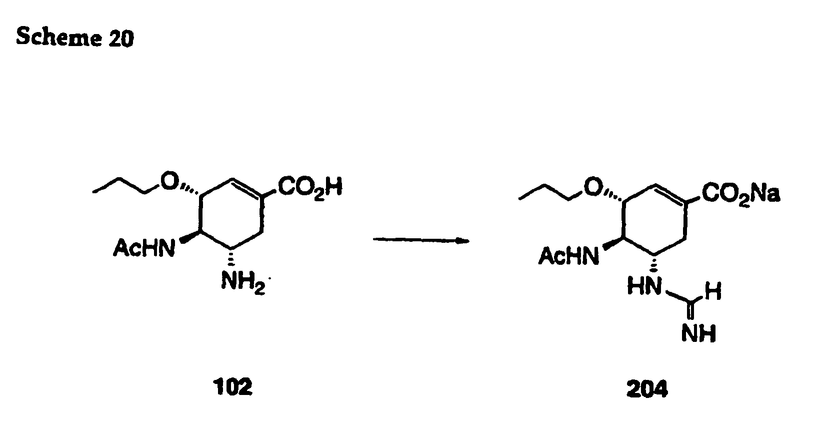

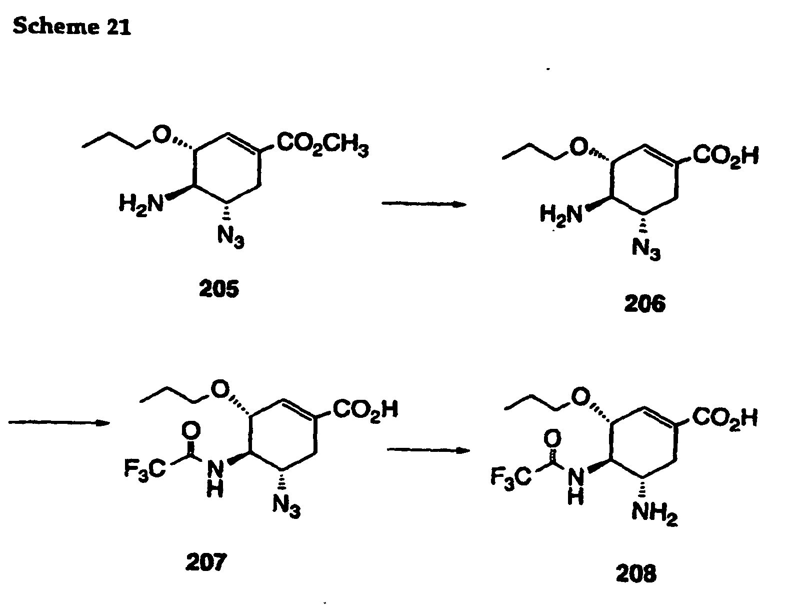

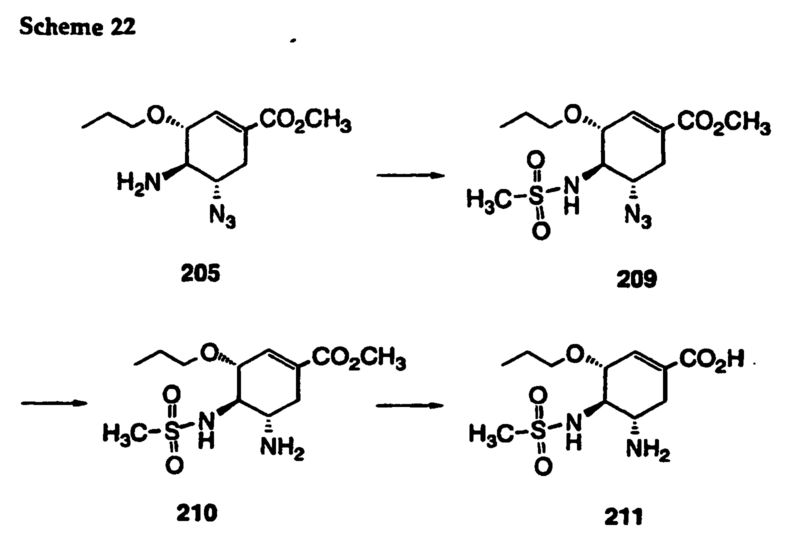

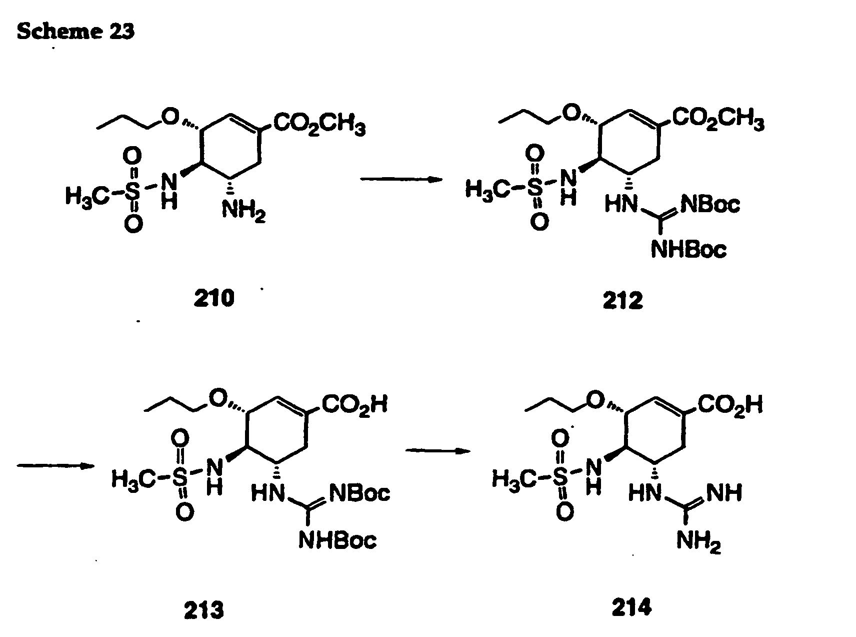

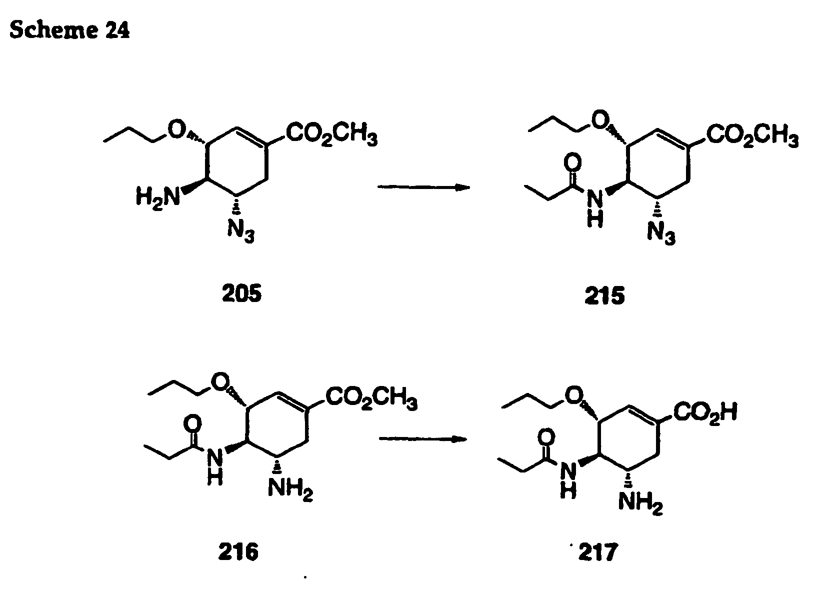

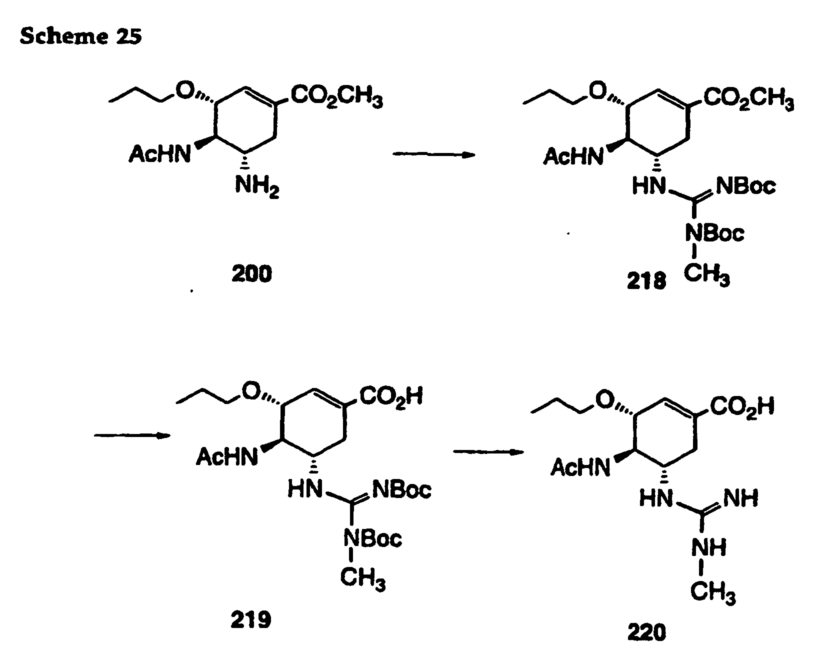

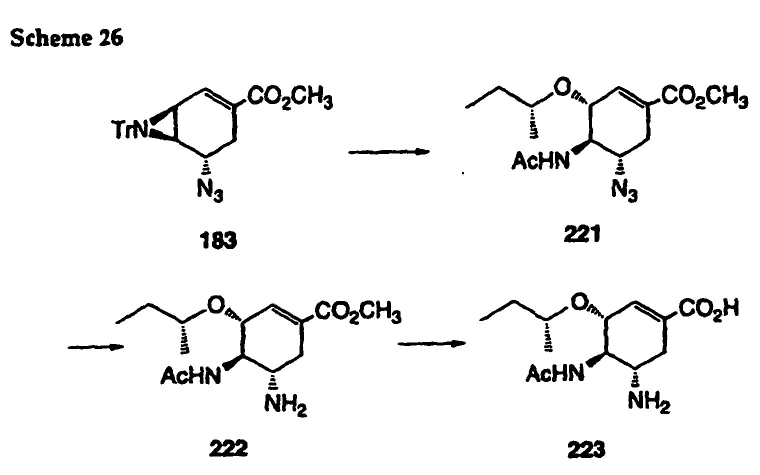

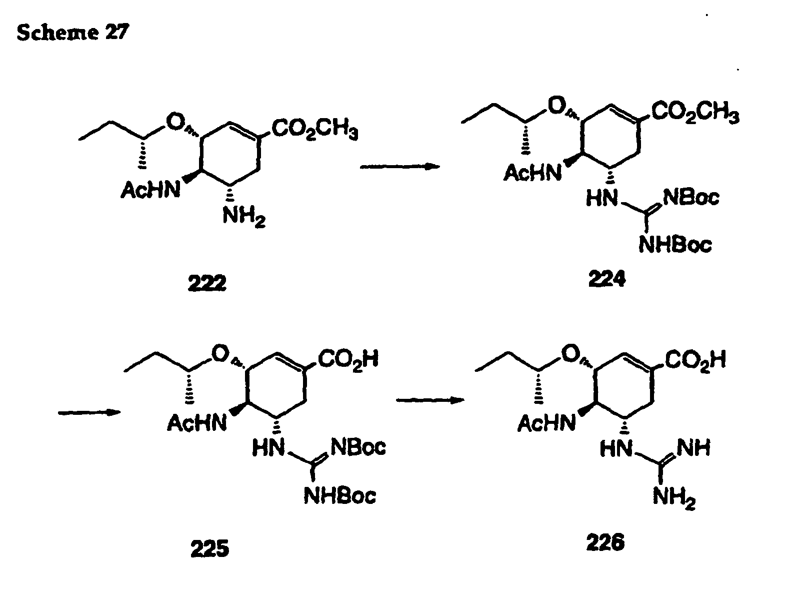

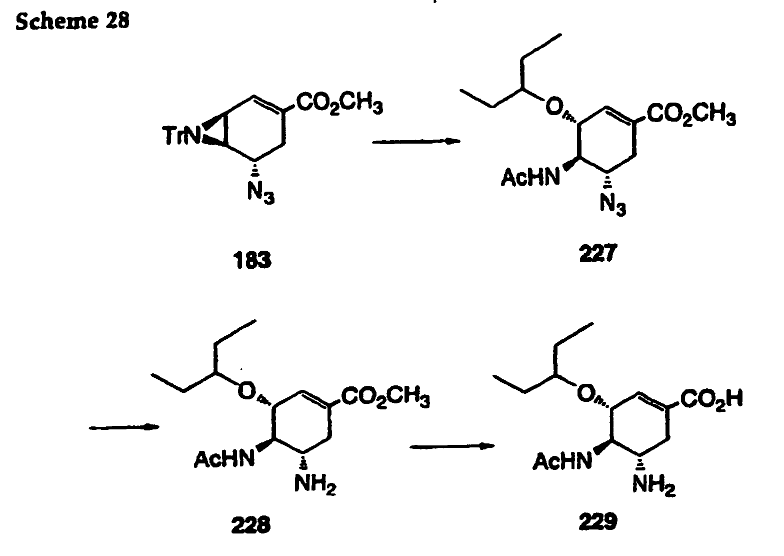

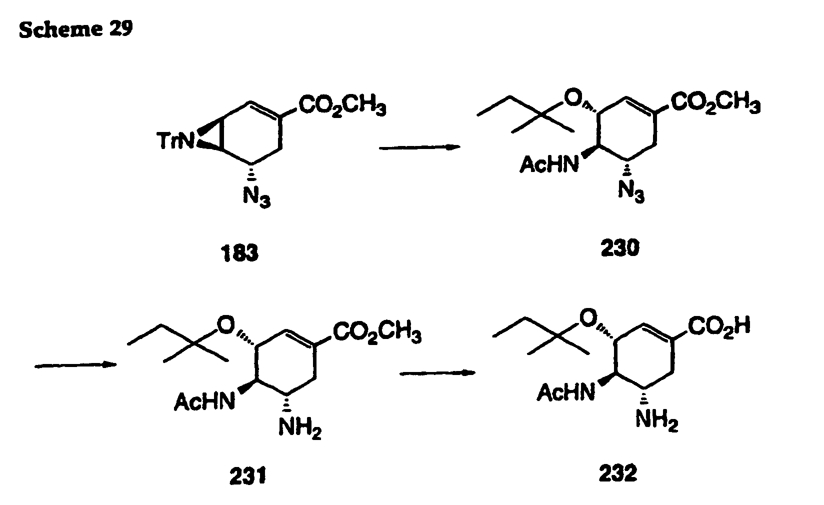

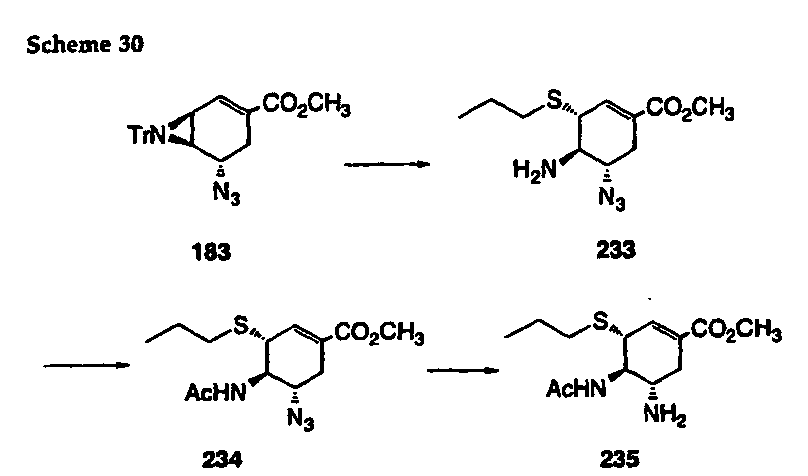

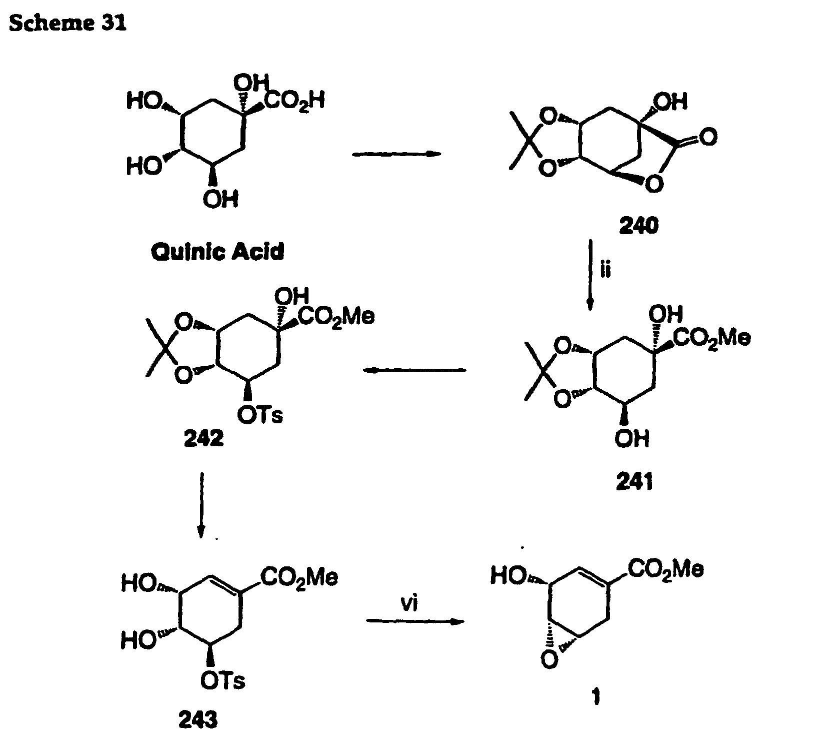

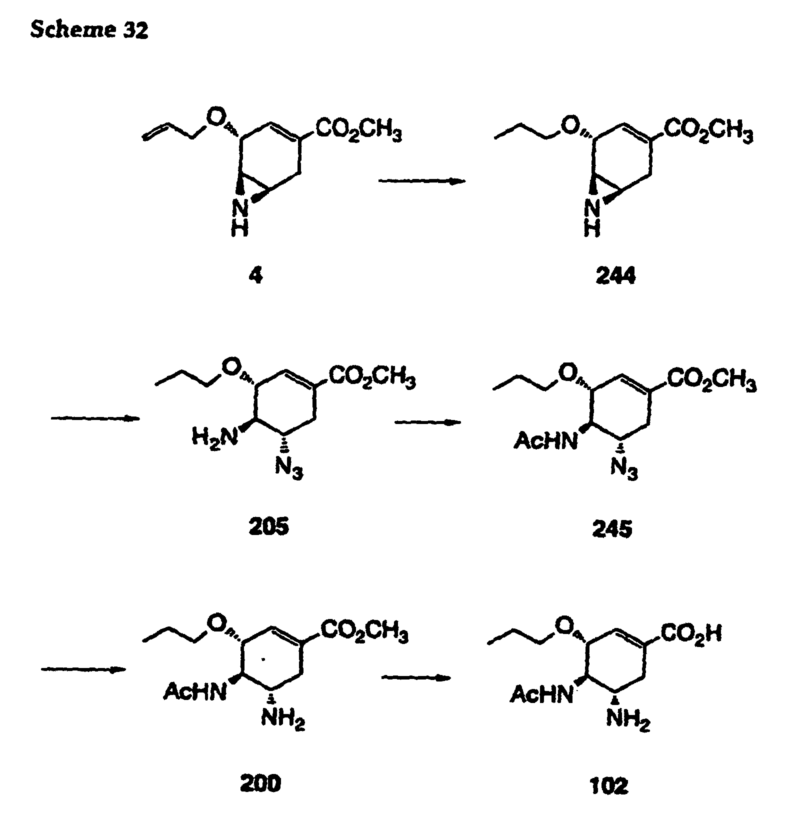

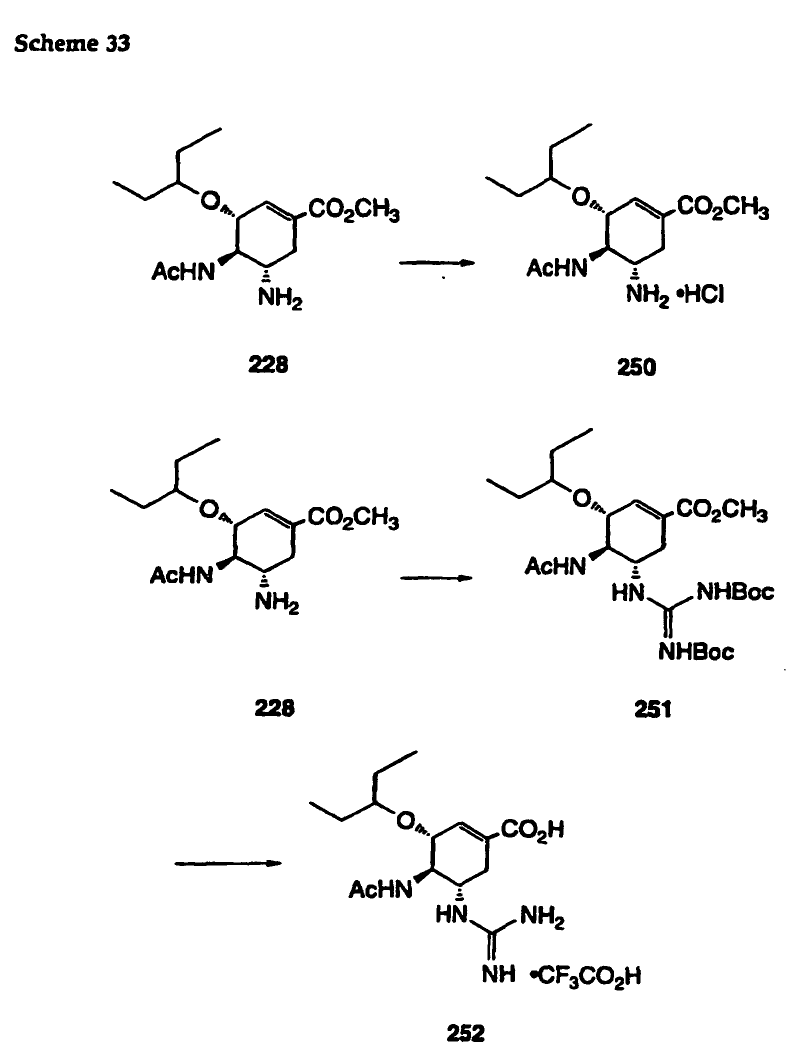

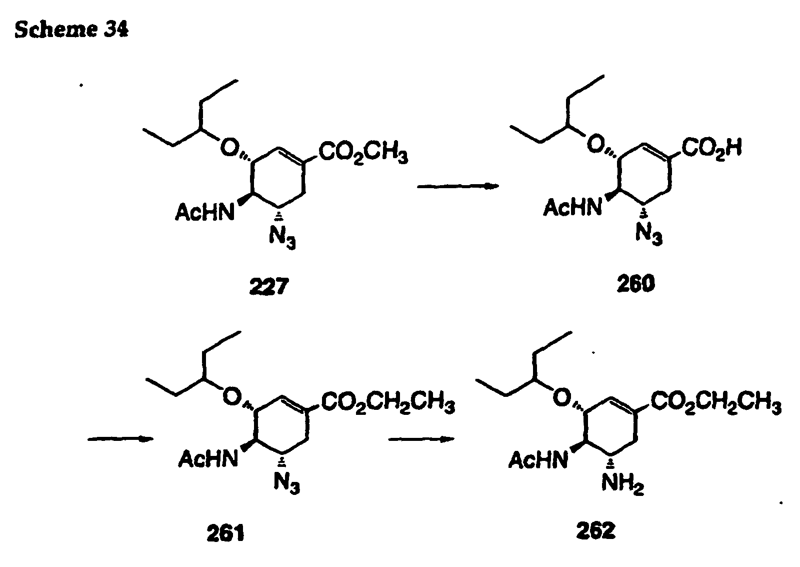

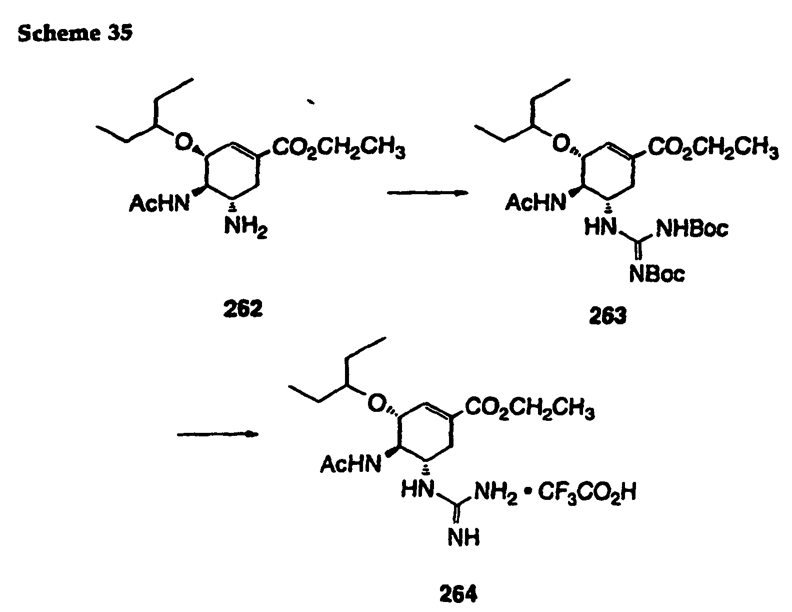

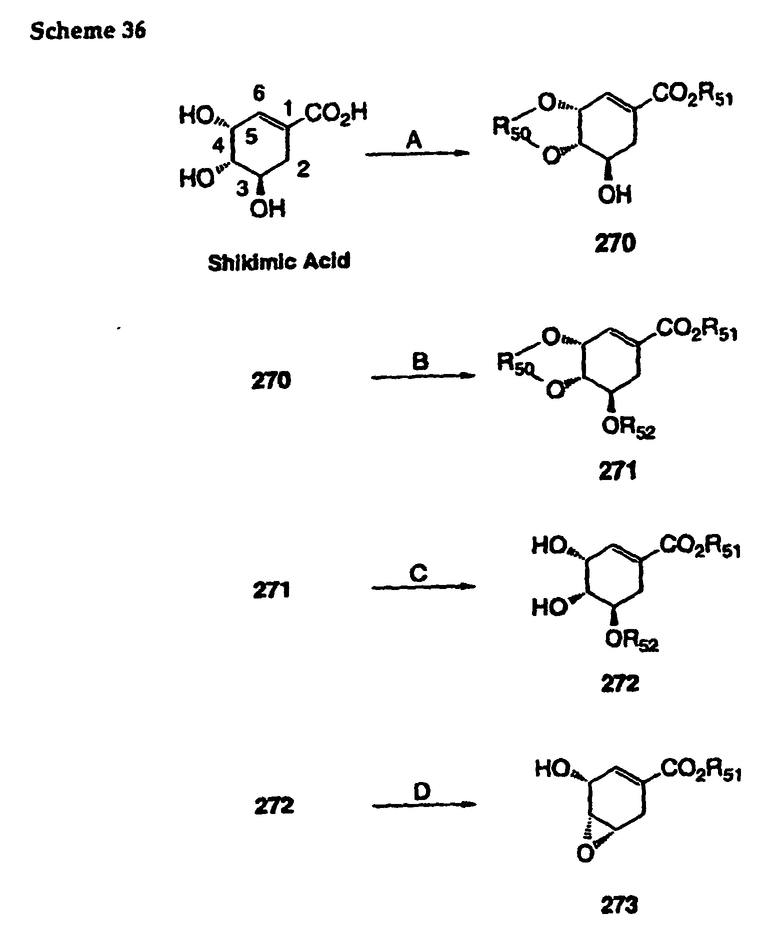

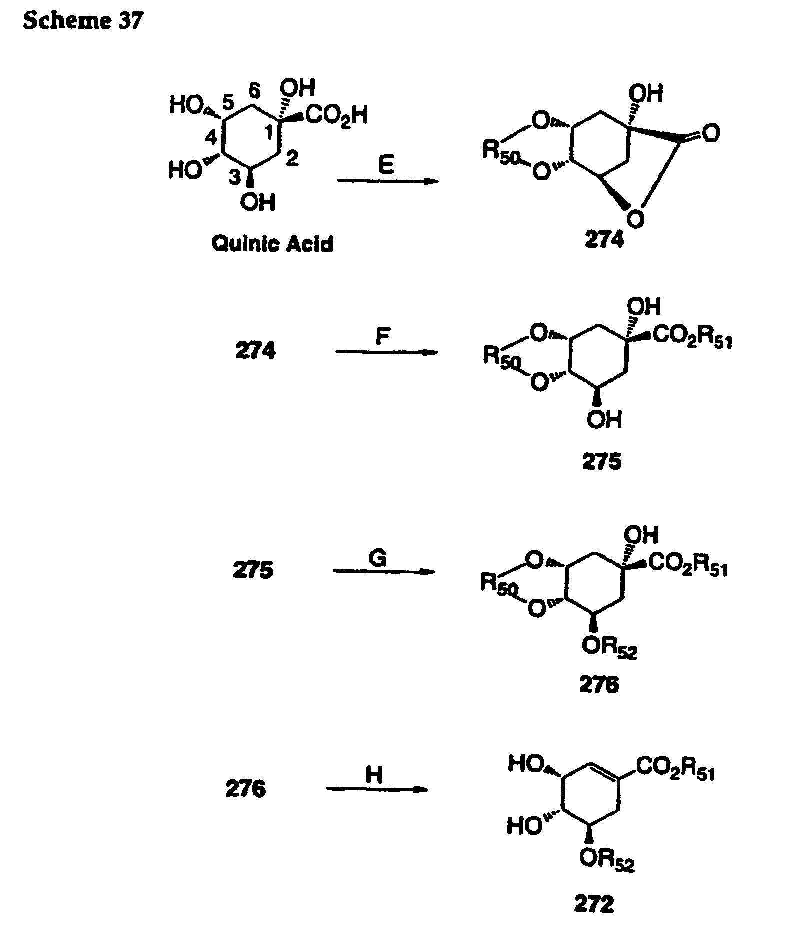

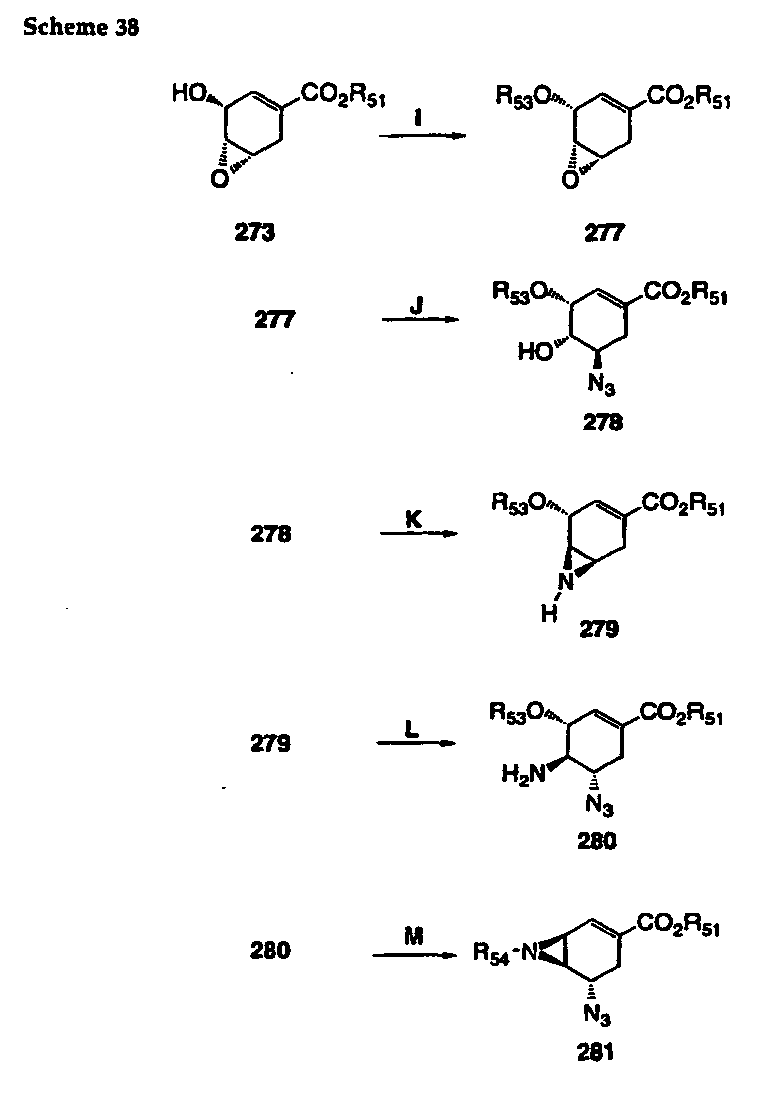

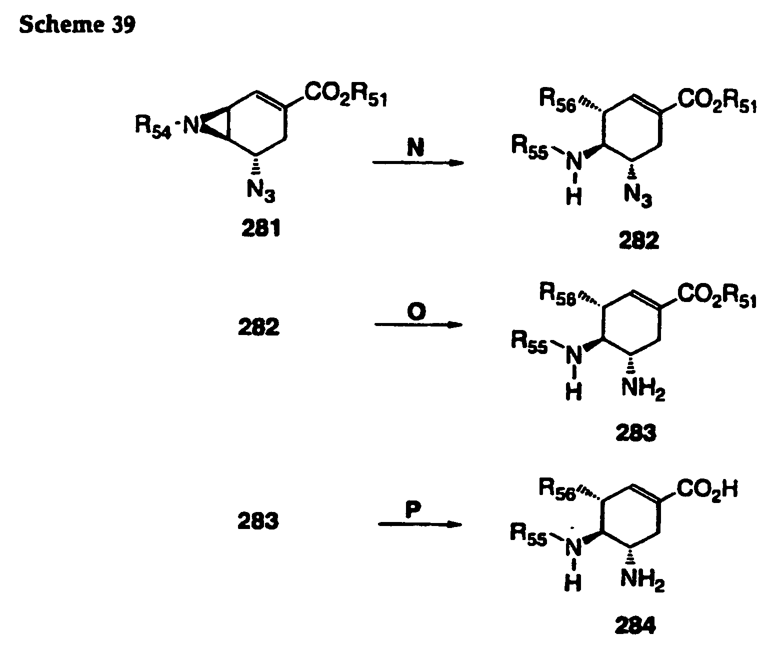

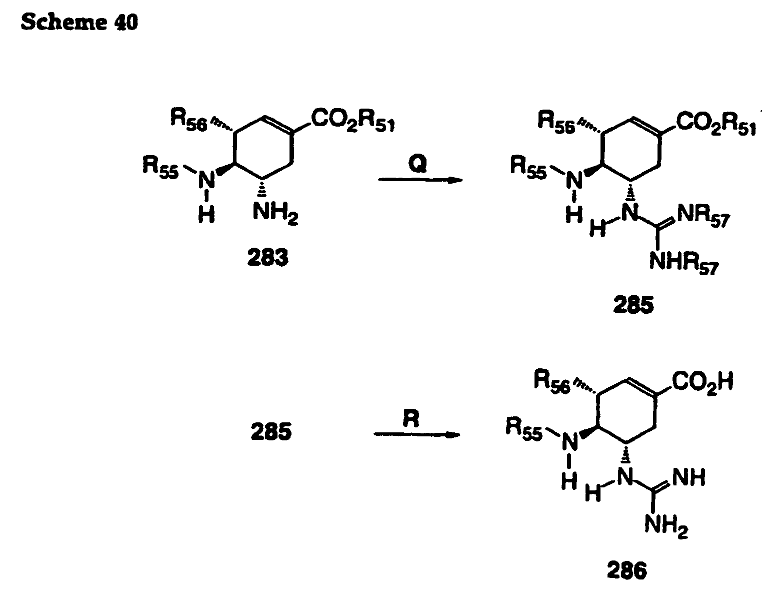

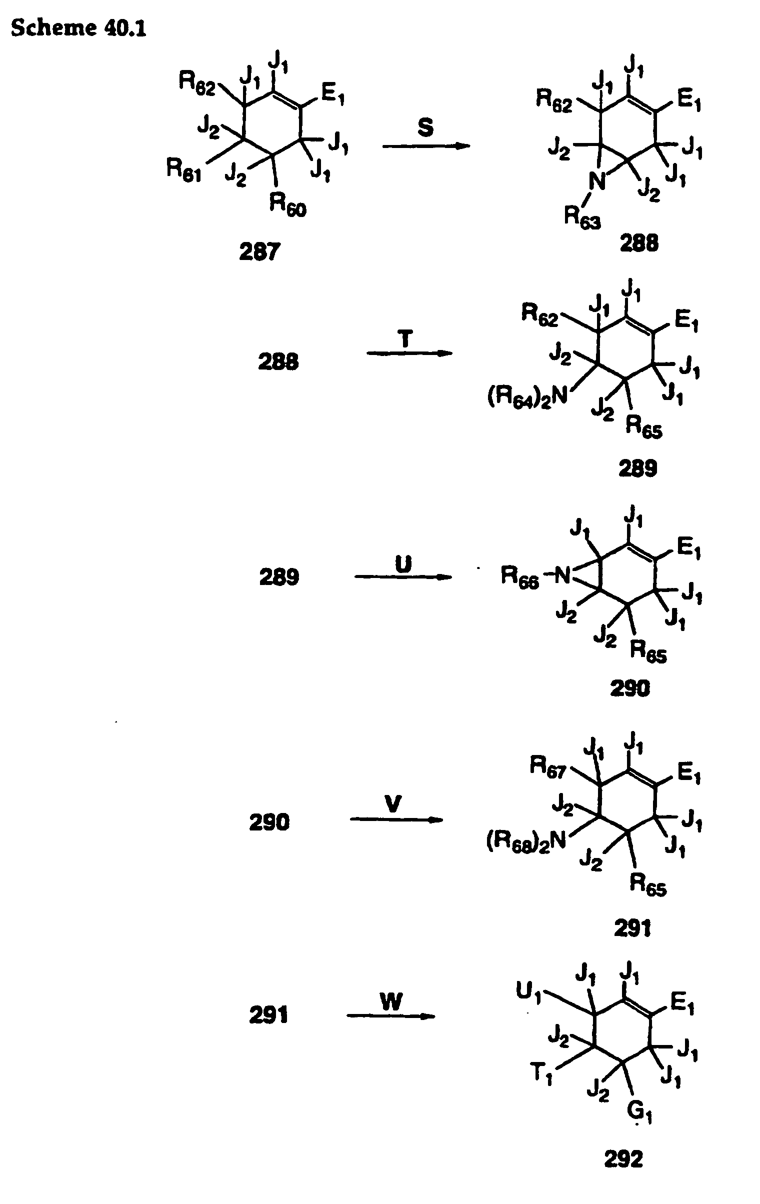

Additional embodiments of methods of making and using compositions of the invention are depicted in **Schemes 36-40.1**. One aspect of the invention is directed to methods of making compounds of the invention comprising processes A, B, C, D, E, F, G, H, I, J, K, L, M, N, O, P, Q, R, S, T, U, V or W of **Schemes 36-40.1,** alone or in combination with each other. **Table 27** describes exemplary method embodiments of processes A-W. Each embodiment is an individual method using the unit processes A-W alone or in combination. Each method embodiment of **Table 27** is separated by a ";". If the embodiment is a single letter than it corresponds to one of the processes A-W. If it is more than one letter than it corresponds to each of the processes performed sequentially in the order indicated.

Other aspects of the invention are directed to methods of using shikimic acid to prepare compound **270** shown as A in **Schemes 36,** methods of using compound **270** to prepare compound **271** shown as B in **Schemes 36,** methods of using compound **271** to prepare compound **272** shown as C in **Schemes 36,** methods of using compound **272** to prepare compound **273** shown as D in **Schemes 36,** methods of using quinic acid to prepare compound **274** shown as E in **Schemes 37,** methods of using compound **274** to prepare compound **275** shown as F in **Schemes 37,** methods of using compound **275** to prepare compound **276** shown as G in **Schemes 37,** methods of using compound **276** to prepare compound **272** shown as H in **Schemes 37**, methods of using compound **273** to prepare compound **277** shown as I in **Schemes 38**, methods of using compound **277** to prepare compound **278** shown as J in **Schemes 38**, methods of using compound **278** to prepare compound **279** shown as K in **Schemes 38**, methods of using compound **279** to prepare compound **280** shown as L in **Schemes 38**, methods of using compound **280** to prepare compound **281** shown as M in **Schemes 38**, methods of using compound **281** to prepare compound **282** shown as N in **Schemes 39,** methods of using compound **282** to prepare compound **283** shown as O in **Schemes 39,** methods of using compound **283** to prepare compound **284** shown as P in **Schemes 39,** methods of using compound **283** to prepare compound **285** shown as Q in **Schemes 40,** methods of using compound **285** to prepare compound **286** shown as **R** in **Schemes 40,** methods of using compound **287** to prepare compound **288** shown as S in **Schemes 40.1,** methods of using compound **288** to prepare compound **289** shown as T in **Schemes 40.1**, methods of using compound **289** to prepare compound **290** shown as U in **Schemes 40.1,** methods of using compound **290** to prepare compound **291** shown as V in **Schemes 40.1,** and methods of using compound **291** to prepare compound **292** shown as W in **Schemes 40.1.**

General aspects of these exemplary methods are described below and in the Example. Each of the products of the following processes is optionally separated, isolated, and/or purified prior to its use in subsecquent processes.

The terms "treated", "treating", "treatment", and the like, mean contacting, mixing, reacting, allowing to react, bringing into contact, and other terms common in the art for indicating that one or more chemical entities is treated in such a manner as to convert it to one or more other chemical entities. This means that "treating compound one with compound two" is synonymous with "allowing compound one to react with compound two", "contacting compound one with compound two", "reacting compound one with compound two", and other expressions common in the art of organic synthesis for reasonably indicating that compound one was "treated", "reacted", "allowed to react", etc., with compound two.

"Treating" indicates the reasonable and usual manner in which organic chemicals are allowed to react. Normal concentrations (0.01M to 10M, typically 0.1M to 1M), temperatures (-100°C to 250°C, typically -78°C to 150°C, more typically -78°C to 100°C, still more typically 0°C to 100°C), reaction vessels (typically glass, plastic, metal), solvents, pressures, atmospheres (typically air for oxygen and water insensitive reactions or nitrogen or argon for oxygen or water sensitive), etc., are intended unless otherwise indicated. The knowledge of similar reactions known in the art of organic synthesis are used in selecting the conditions and apparatus for "treating" in a given process. In particular, one of ordinary skill in the art of organic sysnthesis selects conditions and apparatus reasonably expected to successfully carry out the chemical reactions of the described processes based on the knowledge in the art.

### Process A, Scheme 36

Shikimic acid is used to prepare compound **270** by the following process.

The cis-4,5-diol function of shikimic acid is differentiated from the carboxylic acid at carbon **1** by selective protection of these two functionalities. Typically the cis-4,5-diol function is protected as a cyclic ketal and the carboxylic acid function is protected as an ester.

R₅₀ is an acid labile 1,2-diol protecting group such as those described Greene, John Wiley & Sons, Inc., New York, 1991, "Protective Groups in Organic Chemistry", typically a cyclic ketal or acetal, more typically, a ketal of cyclohexanone or acetone. R₅₁ is an acid stable carboxylic add protecting group such as those described in the above cited work of Greene, typically a linear, branched or cyclic alkyl, alkenyl, or alkynyl of 1 to 12 carbon atoms such as those shown as groups 2-7, 9-10, 15, or 100-660 of **Table 2**, more typically a linear or branched alkyl of 1 to 8 carbon atoms such as those shown as groups 2-5, 9, or 100-358 of **Table 2**, still more typically a linear or branched alkyl of 1 to 6 carbon atoms such as those shown as groups 2-5, 9, or 100-141 of **Table 2**, more typically yet, R51 is methyl, ethyl, n-propyl, i-propyl, n-butyl, sec-butyl, i-butyl, or t-butyl.

Shikimic acid is reacted to protect the carboxylic add with group R₅₁ and the cis-4,5-diol with group R₅₀. Typically shikimic acid is treated with an alcohol, such as methanol, ethanol, n-propanol, or i-propanol, and an add catalyst, such as a mineral acid or a sulfonic acid such as methane, benzene or toluene sulfonic acid, followed by a dialkyl ketal or acetal of a ketone or aldehyde, such as 2,2-dimethoxy-propane, or 1,1-dimethoxy-cyclohexane, in the presence of the corresponding ketone or aldehyde, such as acetone or cyclohexanone. Optionally, the product of the alcohol and acid catalyst treatment is separated, isolated and/or purified prior to treatment with dialkyl ketal or acetal. Alternatively shikimic acid is treated with CH₂N₂.

Typically, the process comprises treating shikimic acid with an alkanol and a sulfonic acid followed by treating with a geminal-dialkoxyalkane or geminal dialkoxycycloalkane and an alkanone or cycloalkanone to form compound **270.** More typically, the process comprises treating shikimic add with an alkanol and a sulfonic acid; evaporating excess alkanol to form a residue; treating the residue with a geminal-dialkoxyalkane or geminal-dialkoxycydoalkane and an alkanone or cycloalkanone to form compound **270.** Still more typically, the process comprises treating shikimic acid with methanol and para-toluenesulfonic acid; evaporating excess methanol to form a residue; treating the residue with 2,2-dimethoxypropane and acetone to form compound **270**.

An exemplary embodiment of this process is given as Example 55 below.

### Process B, Scheme 36

Compound **270** is used to prepare compound **271** by the following process.

The hydroxy group at position 3 is activated, typically, activated toward displacement reactions, more typically, activated toward epoxide ring forming displacement with an alcohol at position 4.

R₅₂ is an alcohol activating group, typically, an activating group toward displacement reactions, more typically, an activating group toward epoxide ring forming displacement with an alcohol at position 4. Such groups include those typical in the art such as sulfonic acid esters, more typically, methane, benzene or toluene sulfonic acid esters. In one embodiment, R₅₂, taken together with O (i.e. -OR₅₂), is a leaving group such as those common in the art.

Typically the process comprises treating compound **270** with an acid halide to form compound **271.** More typically, the process comprises treating compound **270** with a sulfonic acid halide in a suitable solvent to form compound **271.** Still more typically, the process comprises treating compound **270** with a sulfonic acid halide in a suitable solvent such as an amine, optionally, in the presence of a cosolvent, such as a haloalkane, to form compound **271.** More typically yet, the process comprises treating compound **270** with methane sulfonyl chloride in triethylamine/dichloromethane to form compound **271.**

An exemplary embodiment of this process is given as Example 56 below.

### Process C, Scheme 36

Compound **271** is used to prepare compound **272** by the following process.

The acid labile protecting group (R₅₀) for the hydroxy groups at positions 4 and 5 is removed. Typically, R₅₀ is removed without substaintially removing base labile carboxylic acid protecting groups (e.g. R₅₁) or hydroxy activating groups (e.g. R₅₂). Still more typically, R₅₀ is cleaved, under acidic conditions.

Typically the process comprises treating compound **271** with a protic solvent, more typically, in the presence of an acid catalyst as described above. Still more typically, the process comprises treating compound **271** with an alkanol as described above and an acid catalyst as described above. More typically yet, the process comprises treating compound **271** with methanol and para-toluene sulfonic acid to produce compound **272**.

An exemplary embodiment of this process is given as Example 57 below.

### Process D, Scheme 36

Compound **272** is used to prepare compound **273** by the following process.

The activated hydroxy group at position 3 of compound **272** is displaced by the hydroxy at position 4 of compound **272** to produce epoxide compound **273.** Typically the displacement is catalyzed by a suitable base, more typically, an amine base such as DBU or DBN.

Typically the process comprises treating compound **272** with a basic catalyst, optionally in the presecnce of a suitable solvent. Still more typically, the process comprises treating compound **272** with an amine base in a polar, non-protic solvent such as diethyl ether or THF. More typically yet, the process comprises treating compound **272** with DBU in THF to produce compound **273.**

An exemplary embodiment of this process is given as Example 58 below.

### Process E, Scheme 37

Quinic acid is used to prepare compound **274** by the following process.

The cis-4,5-diol function of quinic acid is differentiated from the carboxylic acid at carbon 1 by selective protection of these two functionalities. Typically the cis-4,5-diol function is protected as a cyclic ketal and the carboxylic acid function is protected as a lactone with the hydroxy group at position 3.

R₅₀ is as described above.

Typically, the process comprises treating quinic acid with a geminal-dialkoxyalkane or geminal dialkoxycycloalkane, as described above, and an alkanone or cycloalkanone, as described above, optionally, in the presence of an acid catalyst, as described above, to form compound **274**. More typically, the process comprises treating quinic acid with a geminal-dialkoxyalkane or geminal-dialkoxycycloalkane, an alkanone or cycloalkanone, and an acid catalyst to form compound **270**. Still more typically, the process comprises treating quinic acid with2,2-dimethoxypropane, acetone, and para-toluenesulfonic acid to form compound **274**.

An exemplary embodiment of this process is given as Example 101 below.

### Process F, Scheme 37

Compound **274** is used to prepare compound **275** by the following process.

The lactone is opened to form compound **275.** Typically, the lactone is opened to produce a protected carboxylic acid at position 1 and a free hydroxy at position 3. More typically, the lactone is opened under basic conditions to produce an R₅₁ protected carboxylic acid at position 1 and a free hydroxy group at position 3..

R₅₁ is as described above.

Typically compound **274** is treated with a suitable base in a suitable protic solvent. More typically compound **275** is treated with a metal alkoxide base, such as sodium, potassium or lithium alkoxide, in an alkanol, as described above. Still more typically, compound **274** is treated with NaOMe in MeOH to produce compound **275**.

An exemplary embodiment of this process is given as Example 102 below.

### Process G, Scheme 37

Compound **275** is used to prepare compound **276** by the following process.

The hydroxy group at position 3 is activated, typically, activated toward displacement reactions, more typically, activated toward epoxide ring forming displacement with an alcohol at position 4.

R₅₂ is an alcohol activating group, typically, an activating group toward displacement reactions, more typically, an activating group toward epoxide ring forming displacement with an alcohol at position 4. Such groups include those typical in the art such as sulfonic acid esters, more typically, methane, benzene or toluene sulfonic acid esters. In one embodiment, R₅₂, taken together with O (i.e. -OR₅₂), is a leaving group such as those common in the art.

Typically the process comprises treating compound **275** with an acid halide to form compound **276**. More typically, the process comprises treating compound **275** with a sulfonic acid halide in a suitable solvent to form compound **276**. Still more typically, the process comprises treating compound **275** with a sulfonic acid halide in a suitable solvent such as an amine, optionally, in the presence of a cosolvent, such as a haloalkane, to form compound **276**. More typically yet, the process comprises treating compound **275** with *p*-toluene sulfonyl chloride in pyridine dichloromethane to form compound **276**.

An exemplary embodiment of this process is given as Example 103 below.

### Process H, Scheme 37

Compound **276** is used to prepare compound **272** by the following process.

The hydroxy group at position 1 is eliminated and the cis-4,5-diol protecting group is removed. The hydroxy group at position 1 is eliminated to form an olefinic bond between positions 1 and 6 and the cis-4,5-diol protecting group is removed to regenerate the cis-4,5-diol.

Typically the process comprises treating compound **276** with a suitable dehydrating agent, such as a mineral acid (HCl, H₂SO₄) or SO₂Cl₂. More typically, compound **276** is treated with SO₂Cl₂, followed by an alkanol, optionally in the presence of an add catalyst. Still more typically, compound **276** is treated with SO₂Cl₂ in a suitable polar, aprotic solvent, such as an amine to form an olefin; the olefin is treated with an alkanol, as described above, and an acid catalyst, as described above, to form compound **272.** More typically yet, compound **276** is treated with SO₂Cl₂ in pyridine/CH₂Cl₂ at a temperature between -100°C and 0°C, typically -100°C and -10°C, more typically -78°C, to form an olefin; the olefin is treated with methanol and para-toluene sulfonic acid to form compound **272.**

An exemplary embodiment of this process is given as Example 104 below.

### Process I, Scheme 38

Compound **273** is used to prepare compound **277** by the following process.

The hydroxy group at position 5 is protected. Typically the protecting group is an acid labile hydroxy protecting. More typically, the protecting group resists transfer to adjacent hydroxy groups.

R₅₃ is an acid labile hydroxy protecting group such as those described in the above cited work of Greene. More typically, R₅₃ is an acid cleavable ether, still more typically, R₅₃ is methoxymethyl (MOM, CH₃-O-CH₂-).

Typically the process comprises treating compound **273** with a hydroxy protecting group reagent as described in Greene. More typically the process comprises treating compound **273** with a substituted or unsubstituted haloalkane or alkene, such as methoxymethyl chloride (MOM chloride, CH₃-O-CH₂-Cl), in a suitable solvent, such as a polar, aprotic solvent. Still more typically, the process comprises treating compound **273** with MOM chloride in an amine solvent. More typically yet, the process comprises treating compound **273** with MOM chloride in diisoproply ethyl amine.

An exemplary embodiment of this process is given as Example 59 below.

### Process J, Scheme 38

Compound **277** is used to prepare compound **278** by the following process.

The epoxide at positions 3 and 4 is opened to form an azide. More typically, the epoxide at positions 3 and 4 is opened to form a 3-azido-4-hydroxy compound **278.**

Typically the process comprises treating compound **277** with an azide salt in a suitable solvent. More typically, the process comprises treating compound **277** with sodium azide and a mild base, such as an ammonium halide, in a polar, protic solvent, such as an alkanol or water. Still more typically, the process comprises treating compound **277** with sodium azide and ammonium chloride in water/methanol solution to produce compound **278**.

An exemplary embodiment of this process is given as Example 60 below.

### Process K, Scheme 38

Compound **278** is used to prepare compound **279** by the following process.

The hydroxy group at position 4 of compound **278** is displaced by the 3-azido group to form the aziridine compound **279.**

Typically the process comprises treating compound **278** with a hydroxy activating group as described above, an organophosphine and a base. More typically the process comprises treating compound **278** with a sulfonic acid halide, such as those described above, to form an activated hydroxy compound, treating the activated hydroxy compound with trialkyl or tri arylphosphine, such as triphenylphosphine, to form a phosphonium salt, and treating the phosphonium salt with a base, such as an amine, to form compound **279.** Still more typically, the process comprises treating compound **278** with mesyl chloride, to form an activated hydroxy compound, treating the activated hydroxy compound with triphenylphosphine, to form a phosphonium salt, and treating the phosphonium salt with triethylamine and H₂O, to form compound **279.**

An exemplary embodiment of this process is given as Examples 61 and 62 below.

### Process L, Scheme 38

Compound **279** is used to prepare compound **280** by the following process.

The aziridine compound **279** is opened with azide to form azido amine **280.**

Typically the process comprises treating compound **279** with with an azide salt in a suitable solvent. More typically, the process comprises treating compound **279** with sodium azide and a mild base, such as an ammonium halide, in a polar, aprotic solvent, such as an ether, amine, or amide. Still more typically, the process comprises treating compound **279** with sodium azide and ammonium chloride in DMF solution to producee compound **280**.

An exemplary embodiment of this process is given as Example 63 below.

### Process M, Scheme 38

Compound **280** is used to prepare compound **281** by the following process.

The protected hydroxy group at position 5 is displaced by the amine at position 4 to form aziridine **281**. Typically the aziridine **281** is substituted with an acid labile group, more typically an aziridine activating group.

R₅₄ is an acid labile group, typically an acid labile amine protecting group such as those described in the above cited work of Greene. More typically, R₅₄ is an aziridine activating group, still more typically, a group capable of activating an aziridine toward acid catalyzed ring opening. Typical R₅₄ groups include by way of example and not limitation, a linear or branched 1-oxo-alk-1-yl group of 1 to 12 carbons wherein the alkyl portion is a 1 to 11 carbon linear or branched chain alkyl group (such as CH₃(CH₂)_{z}C(O)-, z is an integer from 0 to 10, i.e. acetyl CH₃C(O)-, etc.), substituted methyl (e.g. triphenylmethyl, Ph₃C-, trityl, Tr), or a carbamate such as BOC or Cbz or a sulfonate (e.g. alkyl sulphonates such as methyl sulphonate). More typical R₅₄ groups include triphenylmethyl and 1-oxo-alk-1-yl groups having 1 to 8, still more typically, 1,2,3,4,5, or 6, more typically yet, 2 or 3 carbon atoms.

Typically the process comprises treating compound **280** with a deprotecting agent to remove group R₅₃, an R₅₄ producing reagent such as those described in Greene (R₅₄-halide, such as acetylchloride, or Tr-Cl, or R₅₄-O-R₅₄, such as acetic anhydride), and a hydroxy activating group such as those described in process **B, Scheme 36**. More typically the process comprises treating compound **280** with a polar, protic solvent, optionally in the presence of an acid catalyst as described above, to form a first intermediate; treating the first intermediate with Tr-Cl in a polar, aprotic solvent, such as an amine, to form a second intermediate; and treating the second intermediate with a sulfonic acid halide, such as mesyl chloride or para toluene sulfonyl chloride, in a polar aprotic solvent, such as an amine, to produce compound **281.** Still more typically, the process comprises treating compound **280** with methanol and HCl, to form a first intermediate; treating the first intermediate with Tr-Cl and triethylamine, to form a second intermediate; and treating the second intermediate with mesyl chloride and triethylamine, to produce compound **281**.

An exemplary embodiment of this process is given as Example 64 below.

### Process N, Scheme 39

Compound **281** is used to prepare compound **282** by the following process.

Aziridine **281** is opened and the resulting amine is substituted with an R₅₅ group to form compound **282.** Typically, aziridine **281** is opened by acid catalyzed ring opening and the resulting amine is acylated.

R₅₅ is -C(O)CH₃, COCH₂F, COCHF₂ or COCF₃. R₅₆ is U₁ as described above.

Exemplary embodiments of this process are given as Examples 65, 86, 92, and 95 below.

### Process O, Scheme 39

Compound **282** is used to prepare compound **283** by the following process.

The azide of compound **282** is reduced to form amino compound **283.**

Typically the process comprises treating compound **282** with a reducing agent to form compound **283.** More typically the process comprises treating compound **282** with hydrogen gas and a catalyst (such as platinum on carbon or Lindlar's catalyst), or reducing reagents (such as a trialkyl or triaryl phosphine as described above). More typically still, the process comprises treating compound **282** with triphenylphosphine in water/THF to form compound **283.**

Exemplary embodiments of this process are given as Examples 87, 93, and 96 below.

### Process P, Scheme 39

Compound **283** is used to prepare compound **284** by the following process.

The carboxylic acid protecting group is removed.

Typically the process comprises treating compound **283** with a base. More typically, the process comprises treating compound **283** with a metal hydroxide in a suitable solvent such as an aprotic, polar solvent. More typically still, the process comprises treating compound **283** with aqueous potassium hydroxide in THF to produce compound **284.**

Exemplary embodiments of this process are given as Examples 88, 94, and 97 below.

### Process Q, Scheme 40

Compound **283** is used to prepare compound **285** by the following process.

The amine is converted to a protected guanidine.

R₅₇ is a guanidine protecting group common in the art, such as BOC or Me.

Typically the process comprises treating compound **283** with a guanidylating reagent such as those common in the art. Exemplary reagents include Bis-BOC Thio-Urea aminoiminomethanesulfonic acid (Kim; et al.; "Tet. Lett." 29(26):3183-3186 (1988) and 1-guanylpyrazoles (Bernatowicz; et al.; "Tet. Lett." 34(21):3389-3392 (1993). More typically, the process comprises treating compound **283** with Bis-BOC Thio-Urea acid. Still more typically, the process comprises treating compound **283** with Bis-BOC Thio-Urea acid and HgCl₂ to form compound **285.**

An exemplary embodiment of this process is given as Example 67 below.

### Process R, Scheme 40

Compound **285** is used to prepare compound **286** by the following process.

The carboxylic acid and guanidine protecting groups are removed.

Typically the process comprises treating compound **285** with a base; followed by treating with an acid, as described above. More typically the process comprises treating compound **285** with a metal hydroxide base, described above, to form an intermediate; and treating the intermediate with acid to form compound **286.** Still more typically the process comprises treating compound **285** with aqueous potassium hydroxide and THF, to form an intermediate; and treating the intermediate with TFA to form compound **286.**

### Process S, Scheme 40.1

Compound **287** is used to prepare compound **288** by the following process.

E₁, J₁ and J₂ of compounds **287** and **288** are as described above. Typically, E₁ is -CO₂R₅₁ as described above. J₁ and J₂ are H.

R₆₀ and R₆₁ are groups capable of reacting to form the R₆₃ (defined below) substituted aziridine ring of compound **288**. Typically, one of R₆₀ or R₆₁ is a primary or secondary amine, or a group capable of being converted to a primary or secondary amine. Such groups for R₆₀ and R₆₁ include by way of example and not limitation, -NH₂, and -N₃. The other of R₆₀ and R₆₁ is typically a group capable of being displaced by a primary or secondary amine to form an aziridine. Such groups include by way of example and not limitation, -OH, Br, Cl, and L Typically, R₆₀ and R₆₁ are in a trans configuration. More typically, R₆₀ is a primary or secondary amine, or a group capable of being converted to a primary or secondary amine and R₆₁ is a group capable of being displaced by a primary or secondary amine to form an aziridine. Still more typically, R₆₀ is β-azido or β-NH₂, and R₆₁ is α-OH, α-OMesyl, or α-OTosyl.

R₆₂ is described below in Process U, **Scheme 40.1.**

The process comprises treating compound **287** to form compound **288.** This is typically accomplished by treating compound **287** to displace R₆₁ by R₆₀. More typically, compound **287** is treated to activate R₆₁ toward displacement by R₆₀. Still more typically, compound **287** is treated to activate R₆₁ toward displacement by R₆₀, and R₆₀ is activated toward displacement of R₆₁. If both R₆₀ and R₆₁ are activated, the activations can be performed simultaneously or sequentially. If the activations are performed sequentially, they can be performed in any order, typically the activation of R₆₁ precedes the activation of R₆₀.

Activation of R₆₁ toward displacement by R₆₀ is typically accomplished by treating compound **287** with a hydroxy activating reagent such as mesyl or tosyl chloride. Activation of R₆₀ toward displacement of R₆₁ is typically accomplished by treating compound **287** to form a primary or secondary amine and treating the amine with a base. By way of example and not limitation, compound **287** is treated with a reducing agent capable of reducing an azide to an amine and a base.

In one embodiment of this process, compound **287** is treated with an R₆₁ activating reagent, and an R₆₀ activating reagent to produce compound **288.** In another embodiment, compound **287** is treated in a suitable solvent with an R₆₁ activating reagent, and an R₆₀ activating reagent to produce compound **288.** In another embodiment, compound **287** is treated with an R₆₁ activating reagent, an R₆₀ activating reagent, and a base to produce compound **288.** In another embodiment, compound **287** is treated in a suitable solvent with an R₆₁ activating reagent, an R₆₀ activating reagent, and a base to produce compound **288.** In another embodiment, compound **287** wherein R₆₀ is an azide is treated with an R₆₁ activating reagent, and an azide reducing reagent to produce compound **288.** In another embodiment, compound **287** wherein R₆₀ is an azide is treated in a suitable solvent with an R₆₁ activating reagent, and an azide reducing reagent to produce compound **288.** In another embodiment, compound **287** wherein R₆₀ is an azide is treated with an R₆₁ activating reagent, an azide reducing reagent, and a base to produce compound **288.** In another embodiment, compound **287** wherein R₆₀ is an azide is treated in a suitable solvent with an R₆₁ activating reagent, an azide reducing reagent, and a base to produce compound **288.** In another embodiment, compound **287** wherein R₆₀ is an azide and R₆₁ is a hydroxy, is treated with a hydroxy activating reagent, and an azide reducing reagent to produce compound **288.** In another embodiment, compound **287** wherein R₆₀ is an azide and R₆₁ is a hydroxy, is treated in a suitable solvent with an hydroxy activating reagent, and an azide reducing reagent to produce compound **288.** In another embodiment, compound **287** wherein R₆₀ is an azide and R₆₁ is a hydroxy, is treated with a hydroxy activating reagent, an azide reducing reagent, and a base to produce compound **288.** In another embodiment, compound **287** wherein R₆₀ is an azide and R₆₁ is a hydroxy, is treated in a suitable solvent with a hydroxy activating reagent, an azide reducing reagent, and a base to produce compound **288**.

An exemplary embodiments of this process are given as Process K, **Scheme 38,** above.

### Process T, Scheme 40.1

Compound **288** is used to prepare compound **289** by the following process.

R₆₄ is typically H. R₆₅ is typically G₁ or a group capable of being converted to G₁. More typically R₆₅ is -N₃, or -NH₂.

Typically, compound **288** is treated to form amine **289.** More typically, compound **288** is treated with a nudeopbile, typically a nitrogen nucleophile such as R₆₅, a cationic salt of R₆₅, or a protonated analog of R₆₅, such as by way of example and not limitation, NH₃, an azide salt (such as NaN₃, KN₃, or the like), HCN, a cyanide salt (such as NaCN, KCN, or the like), or a salt of a cyanoalkyl (e.g. (CH₂CN)⁻) (such as NaCH₂CN, KCH₂CN, or the like). Still more typically, compound **288** is treated with an azide salt. Optionally a base, typically a mild base such as an ammonium halide and a solvent, typically a polar, aprotic solvent, such as an ether, amine, or amide are used.

In one embodiment, compound **288** is treated with a nucleophile. In another embodiment, compound **288** is treated with a nucleophile in a suitable solvent to produce compound **289.** In another embodiment, compound **288** is treated with a nucleophile and a base to produce compound **289.** In another embodiment, compound **288** is treated with a nucleophile and a base in a suitable solvent to produce compound **289.** In another embodiment, compound **288** is treated with a nitrogen nucleophile to produce compound **289.** In another embodiment, compound **288** is treated with a nitrogen nucleophile in a suitable solvent to produce compound **289.** In another embodiment, compound **288** is treated with a nitrogen nucleophile and a base to produce compound **289.** In another embodiment, compound **288** is treated with a nitrogen nucleophile and a base in a suitable solvent to produce compound **289.** In another embodiment, compound **288** is treated with an azide salt to produce compound **289**. In another embodiment, compound **288** is treated with an azide salt in a suitable solvent to produce compound **289.** In another embodiment, compound **288** is treated with an azide salt and a base to produce compound **289.** In another embodiment, compound **288** is treated with an azide salt and a base in a suitable solvent to produce compound **289**.

An exemplary embodiment of this process is given as Process L, **Scheme 38**, above.

### Process U, Scheme 40.1

Compound **289** is used to prepare compound **290** by the following process.

R₆₂ is a group capable of reacting with an amine to form the R₆₆ (defined below) substituted aziridine ring of compound **290.** Typically, R₆₂ is a group capable of being displaced by a primary or secondary amine to form an aziridine. Such groups include by way of example and not limitation, -OR₅₃, -OH, Br, Cl, and L Typically, R₆₂ is in a trans configuration relative to the nitrogen in position 4. More typically, R₆₂ is -OR₅₃.

R₆₄ is H, typically an acid labile protecting group such as R₅₄.

R₆₆ is H, or R₅₄.

The process comprises treating compound **289** to form compound **290.** This is typically accomplished by treating compound **289** to displace R₆₂ by the amine at position 4. More typically, compound **289** is treated to activate the amine at position 4 toward displacement of R₆₂. Still more typically, compound **289** is treated to activate the amine at position 4 toward displacement of R₆₂, and R₆₂ is activated toward displacement by the amine at position 4. If both R₆₂ and the amine at position 4 are activated, the activations can be performed simultaneously or sequentially. If the activations are performed sequentially, they can be performed in any order, typically the activation of R₆₂ precedes the activation of the amine at position 4.

Activation of R₆₂ toward displacement by the amine at position 4 is typically accomplished by treating compound **289** with a hydroxy activating agent such as those described in process B, **Scheme 36**. Optionally, R₆₂ is deprotected prior to activation. Activation of the amine at position 4 toward R₆₂ displacement is typically accomplished by treating compound **289** to form a primary or secondary amine and treating the amine with an acid catalyst such as those described in Process N, **Scheme 39,** above..

Typically when R₆₂ is -OR₅₃ and R₆₆ is R₅₆, the process comprises treating compound **289** with a deprotecting agent to remove group R₅₃, an R₅₄ producing reagent such as those described in Greene (R₅₄-halide, such as acetylchloride, or Tr-Cl, or R₅₄-O-R₅₄, such as acetic anhydride), and a hydroxy activating group such as those described in Process B, **Scheme 36**. More typically the process comprises treating compound **289** with a polar, protic solvent, optionally in the presence of an acid catalyst as described above, to form a first intermediate; treating the first intermediate with Tr-Cl in a polar, aprotic solvent, such as an amine, to form a second intermediate; and treating the second intermediate with a sulfonic acid halide, such as mesyl chloride or para toluene sulfonyl chloride, in a polar aprotic solvent, such as an amine, to produce compound **290.** Still more typically, the process comprises treating compound **289** with methanol and HCl, to form a first intermediate; treating the first intermediate with Tr-Cl and triethylamine, to form a second intermediate; and treating the second intermediate with mesyl chloride and triethylamine, to produce compound **290**.

In one embodiment compound **289** is treated with an acid catalyst to produce compound **290.** In another embodiment compound **289** is treated with an acid catalyst in a suitable solvent to produce compound **290.** In another embodiment compound **289** is treated with a hydroxy activating reagent and an acid catalyst to produce compound **290.** In another embodiment compound **289** is treated with a hydroxy activating reagent and an add catalyst in a suitable solvent to produce compound **290**. In another embodiment compound **289** is treated with a hydroxy deprotecting reagent, a hydroxy activating reagent and an acid catalyst to produce compound **290.** In another embodiment compound **289** is treated with a hydroxy activating reagent and an acid catalyst in a suitable solvent to produce compound **290**.

An exemplary embodiment of this process is given as Process M, **Scheme 38**, above.

### Process V, Scheme 40.1

Compound **290** is used to prepare compound **291** by the following process.

Aziridine **290** is treated to form compound **291**. Typically, aziridine **290** is opened by acid catalyzed ring opening and the resulting amine is acylated.

R₆₈ is independently H, R₁ or R₅₅ as defined above. Typically R₅₅ is -C(O)R₅. Typically one R₆₈ is H and the other is W₃.

R₆₇ is U₁ as described above.

Typically the process comprises treating compound **290** with an acid catalyst and a compound of the formula W₆-X₁-H, wherein X₁ is as defined above to form an amine intermediate; and treating the amine intermediate with a compound of the formula W₃-X₁-W₃, or W₃-X₁₀, wherein X₁₀ is a leaving group, to form compound **291.** The treatment with a compound of the formula W6-X1-H and an acid catalyst may be prior to or simultaneous with the treatment with a compound of the formula W₃-X₁-W₃, or W₃-X₁₀. The acid catalyst is typically one of those described in Process N, **Scheme 39,** above. More typically, the process comprises treating compound **290** with a compound of the formula R₅-OH, R₅-SH, or R₅-NH₂ and an acid catalyst; and treating the intermediate with an alkanoic acid anhydride to form compound **291.**

One embodiment comprises treating compound **290** with a compound of the formula W₆-X₁-H and an acid catalyst to produce compound **291.** Another embodiment comprises treating compound **290** with a compound of the formula W₆-X₁-H and an acid catalyst in a suitable solvent to produce compound **291.** Another embodiment comprises treating compound **290** with a compound of the formula W₆-X₁-H, an acid catalyst and a compound of the formula W₃-X₁-W₃ or W₃-X₁₀ to produce compound **291.** Another embodiment comprises treating compound **290** with a compound of the formula W₆-X₁-H, an acid catalyst and a compound of the formula W₃-X₁-W₃ or W₃-X₁₀ in a suitable solvent to produce compound **291.**

Exemplary embodiments of this process are given as Process N, **Scheme 39**, above.

### Process W, Scheme 40.1

Compound **291** is used to prepare compound **292** by the following process.

Compound **291** is treated to form compound **292.** Typically R₆₅ is converted to form G₁. U₁ is an embodiment of R₆₇ and T₁ is an embodiment of -N(R₆₈)₂ prepared in Process V, **Scheme 40.1**, above.

In one embodiment, R₆₅ is deprotected, alkylated, guanidinylated, oxidized or reduced to form G₁. Any number of such treatments can be performed in any order or simultaneously. By way of example and not limitation, when R₆₅ is azido, embodiments of this process include Processes O, OQ, OQR, and OP. Typical alkylating agents are those common in the art including, by way of example and not limitation, an alkyl halide such as methyl iodide, methyl bromide, ethyl iodide, ethyl bromide, n-propyl iodide, n-propyl bromide, i-propyl iodide, i-propyl bromide; and an olefin oxide such as ethylene oxide or propylene oxide. A base catalyst as described herein maybe optionally employed in the alkylation step.

One embodiment comprises treating compound **291** wherein R₆₅ is azido with a reducing agent to produce compound **292.** Another embodiment comprises treating compound **291** wherein R₆₅ is azido with a reducing agent to produce compound **292** in a suitable solvent. Another embodiment comprises treating compound **291** wherein R₆₅ is amino with an alkylating agent to produce compound **292.** Another embodiment comprises treating compound **291** wherein R₆₅ is amino with an alkylating agent to produce compound **292** in a suitable solvent. Another embodiment comprises treating compound **291** wherein R₆₅ is azido with a reducing agent and an alkylating agent to produce compound **292.** Another embodiment comprises treating compound **291** wherein R₆₅ is azido with a reducing agent and an alkylating agent to produce compound **292** in a suitable solvent. Another embodiment comprises treating compound **291** wherein R₆₅ is amino with an alkylating agent and a base catalyst to produce compound **292.** Another embodiment comprises treating compound **291** wherein R₆₅ is amino with an alkylating agent and a base catalyst to produce compound **292** in a suitable solvent. Another embodiment comprises treating compound **291** wherein R₆₅ is azido with a reducing agent, an alkylating agent and a base catalyst to produce compound **292.** Another embodiment comprises treating compound **291** wherein R₆₅ is azido with a reducing agent, an alkylating agent and a base catalyst to produce compound **292** in a suitable solvent

Exemplary embodiments of this process are given as Process O, **Scheme 39**, above.

Exemplary embodiments of this process are given as Examples 68 and 69 below.

**Table 25 -**

| Exemplary Compounds of Formula R₅-OH (CAS No.) |
|---|
| C4 Fluoro Alcohols |
| (R*,R*)-(±)-3-fluoro-2-Butanol (139755-61-6) |
| 1-fluoro-2-Butanol (124536-12-5) |
| (R)-3-fluoro-1-Butanol (120406-57-7) |
| 3-fluoro-1-Butanol (19808-95-8) |
| 4-fluoro-2-Butanol (18804-31-4) |
| (R*,S*)-3-fluoro-2-Butanol (6228-94-0) |
| (R*,R*)-3-fluoro-2-Butanol (6133-82-0) |
| 2-fluoro-1-Butanol (4459-24-9) |
| 2-fluoro-2-methyl-1-Propanol (3109-99-7) |
| 3-fluoro-2-Butanol (1813-13-4) |
| 4-fluoro-1-Butanol (372-93-0) |
| 1-fluoro-2-methyl-2-Propanol (353-80-0) |

| C5 Fluoro Alcohols |
|---|
| 2-fluoro-1-Pentanol (123650-81-7) |
| (R)-2-fluoro-3-methyl-1-Butanol (113943-11-6) |
| (S)-2-fluoro-3-methyl-1-Butanol (113942-98-6) |
| 4-fluoro-3-methyl-1-Butanol (104715-25-5) |
| 1-fluoro-3-Pentanol (30390-84-2) |
| 4-fluoro-2-Pentanol (19808-94-7) |
| 5-fluoro-2-Pentanol (18804-35-8) |
| 3-fluoro-2-methyl-2-Butanol (7284-96-0) |
| 2-fluoro-2-methyl-1-Butanol (4456-02-4) |
| 3-fluoro-3-methyl-2-Butanol (1998-77-2) |
| 5-fluoro-1-Pentanol (592-80-3) |

| C6 Fluoro Alcohols |
|---|
| (R-(R*,S*))-2-fluoro-3-methyl-1-Pentanol (168749-88-0) |
| 1-fluoro-2,3-dimethyl-2-Butanol (161082-90-2) |
| 2-fluoro-2,3-dimethyl-1-Butanol (161082-89-9) |
| (R)-2-fluoro-4-methyl-1-Pentanol (157988-30-2) |
| (S-(R*,R*))-2-fluoro-3-methyl-1-Pentanol (151717-18-9) |
| (R*,S*)-2-fluoro-3-methyl-1-Pentanol (151657-14-6) |
| (S)-2-fluoro-3,3-dimethyl-1-Butanol (141022-94-8) |
| (M)-2-fluoro-2-methyl-1-Pentanol (137505-57-8) |
| (S)-2-fluoro-1-Hexanol (127608-47-3) |
| 3-fluoro-3-methyl-1-Pentanol (112754-22-0) |
| 3-fluoro-2-methyl-2-Pentanol (69429-54-5) |
| 2-fluoro-2-methyl-3-Pentanol (69429-53-4) |
| 1-fluoro-3-Hexanol (30390-85-3) |
| 5-fluoro-2-methyl-2-Pentanol (21871-78-3) |
| 5-fluoro-3-Hexanol (19808-92-5) |
| 4-fluoro-3-methyl-2-Pentanol (19808-90-3) |
| 4-fluoro-4-methyl-2-Pentanol (19031-69-7) |
| 1-fluoro-3,3-dimethyl-2-Butanol (4604-66-4) |
| 2-fluoro-2-methyl-1-Pentanol (4456-03-5) |
| 2-fluoro-4-methyl-1-Pentanol (4455-95-2) |
| 2-fluoro-1-Hexanol (1786-48-7) |
| 3-fluoro-2,3-dimethyl-2-Butanol (661-63-2) |
| 6-fluoro-1-Hexanol (373-32-0) |

| C7 Fluoro Alcohols |
|---|
| 5-fluoro-5-methyl-1-Hexanol (168268-63-1) |
| (R)-1-fluoro-2-methyl-2-Hexanol (153683-63-7) |
| (S)-3-fluoro-1-Heptanol (141716-56-5) |
| (S)-2-fluoro-2-methyl-1-Hexanol (132354-09-7) |
| (R)-3-fluoro-1-Heptanol (120406-54-4) |
| (S)-2-fluoro-1-Heptanol (110500-31-7) |
| 1-fluoro-3-Heptanol (30390-86-4) |
| 7-fluoro-2-Heptanol (18804-38-1) |
| 2-ethyl-2-(fluoromethyl)-1-Butanol (14800-35-2) |
| 2-(fluoromethyl)-2-methyl-1-Pentanol (13674-80-1) |
| 2-fluoro-5-methyl-1-Hexanol (4455-97-4) |
| 2-fluoro-1-Heptanol (1786-49-8) |
| 7-fluoro-1-Heptanol (408-16-2) |

| C8 Fluoro Alcohols |
|---|
| (M)-2-fluoro-2-methyl-1-Heptanol (137505-55-6) |
| 6-fluoro-6-methyl-1-Heptanol (135124-57-1) |
| 1-fluoro-2-Octanol (127296-11-1) |
| (R)-2-fluoro-1-Octanol (118205-91-7) |
| (±)-2-fluoro-2-methyl-1-Heptanol (117169-40-1) |
| (S)-2-fluoro-1-Octanol (110500-32-8) |
| (S)-1-fluoro-2-Octanol (110270-44-5) |
| (R)-1-fluoro-2-Octanol (110270-42-3) |
| (±)-1-fluoro-2-Octanol (110229-70-4) |
| 2-fluoro-4-methyl-3-Heptanol (87777-41-1) |
| 2-fluoro-6-methyl-1-Heptanol (4455-99-6) |
| 2-fluoro-1-Octanol (4455-93-0) |
| 8-fluoro-1-Octanol (408-27-5) |

| C9 Fluoro Alcohols |
|---|
| 6-fluoro-2,6-dimethyl-2-Heptanol (160981-64-6) |
| (S)-3-fluoro-1-Nonanol (160706-24-1) |
| (R-(R*,R*))-3-fluoro-2-Nonanol (137909-46-7) |
| (R-(R*,S*))-3-fluoro-2-Nonanol (137909-45-6) |
| 3-fluoro-2-Nonanol (137639-20-4) |
| (S-(R*,R*))-3-fluoro-2-Nonanol (137639-19-1) |
| (S-(R*,S*))-3-fluoro-2-Nonanol (137639-18-0) |
| (±)-3-fluoro-1-Nonanol (134056-76-1) |
| 2-fluoro-1-Nonanol (123650-79-3) |
| 2-fluoro-2-methyl-1-Octanol (120400-89-7) |
| (R)-2-fluoro-1-Nonanol (118243-18-8) |
| (S)-1-fluoro-2-Nonanol (111423-41-7) |
| (S)-2-fluoro-1-Nonanol (110500-33-9) |
| 1-fluoro-3-Nonanol (30390-87-5) |
| 2-fluoro-2,6-dimethyl-3-Heptanol (684-74-2) |
| 9-fluoro-1-Nonanol (463-24-1) |

| C10 Fluoro Alcohols |
|---|
| 4-fluoro-1-Decanol (167686-45-5) |
| (P)-10-Huoro-3-Decanol (145438-91-1) |
| (R-(R*,R*))-3-fluoro-5-methyl-1-Nonanol (144088-79-9) |
| (P)-10-fluoro-2-Decanol (139750-57-5) |
| 1-fluoro-2-Decanol (130876-22-1) |
| (S)-2-fluoro-1-Decanol (127608-48-4) |
| (R)-1-fluoro-2-Decanol (119105-16-7) |
| (S)-1-fluoro-2-Decanol (119105-15-6) |
| 2-fluoro-1-Decanol (110500-35-1) |
| 1-fluoro-5-Decanol (106533-31-7) |
| 4-fluoro-2,2,5,5-tetramethyl-3-Hexanol (24212-87-1) |
| 10-fluoro-1-Decanol (334-64-5) |

| C11 Fluoro Alcohols |
|---|
| 10-fluoro-2-methyl-1-Decanol (139750-53-1) |
| 2-fluoro-1-Undecanol (110500-34-0) |
| 8-fluoro-5,8-dimethyl-5-Nonanol (110318-90-6) |
| 11-fluoro-2-Undecanol (101803-63-8) |
| 11-fluoro-1-Undecanol (463-36-5) |

| C12 Fluoro Alcohols |
|---|
| 11-fluoro-2-methyl-1-Undecanol (139750-52-0) |
| 1-fluoro-2-Dodecanol (132547-33-2) |
| (R*,S*)-7-fluoro-6-Dodecanol (130888-52-7) |
| (R*,R*)-7-fluoro-6-Dodecanol (130876-18-5) |
| (S)-2-fluoro-1-Dodecanol (127608-49-5) |
| 12-fluoro-2-pentyl-Heptanol (120400-91-1) |
| (R*,S*)-(±)-7-fluoro-6-Dodecanol (119174-39-9) |
| (R*,R*)-(±)-7-fluoro-6-Dodecanol (119174-38-8) |
| 2-fluoro-1-Dodecanol (110500-36-2) |
| 11-fluoro-2-methyl-2-Undecanol (101803-67-2) |
| 1-fluoro-1-Dodecanol (100278-87-3) |
| 12-fluoro-1-Dodecanol (353-31-1) |

| C4 Nitro Alcohols |
|---|
| (R)-4-nitro-2-Butanol (129520-34-9) |
| (S)-4-nitro-2-Butanol (120293-74-5) |
| 4-nitro-1-Butanol radical ion(1-) (83051-13-2) |
| (R*,S*)-3-nitro-2-Butanol (82978-02-7) |
| (R*,R*)-3-nitro-2-Butanol (82978-01-6) |
| 4-nitro-1-Butanol (75694-90-5) |
| (±)-4-nitro-2-Butanol (72959-86-5) |
| 4-nitro-2-Butanol (55265-82-2), |
| 1-aci-nitro-2-Butanol (22916-75-2) |
| 3-aci-nitro2-Butanol (22916-74-1) |
| 2-methyl-3-nitro-1-Propanol (21527-52-6) |
| 3-nitro-2-Butanol (6270-16-2) |
| 2-methyl-1-nitro-2-Propanol (5447-98-3) |
| 2-aci-nitro-1-Butanol (4167-97-9) |
| 1-nitro-2-Butanol (3156-74-9) |
| 2-nitro-1-Butanol (609-31-4) |
| 2-methyl-2-nitro-1-Propanol (76-39-1) |

| C5 Nitro Alcohols |
|---|
| (R)-3-methyl-3-nitro-2-Butanol (154278-27-0) |
| 3-methyl-1-nitro-1-Butanol (153977-20-9) |
| (±)-1-nitro-3-Pentanol (144179-64-6) |
| (S)-1-nitro-3-Pentanol (144139-35-5) |
| (R)-1-nitro-3-Pentanol (144139-34-4) |
| (R)-3-methyl-1-nitro-2-Butanol (141434-98-2) |
| (±)-3-methyl-1-nitro-2-Butanol (141377-55-1) |
| (R*,R*)-3-nitro-2-Pentanol (138751-72-1) |
| (R*,S*)-3-nitro-2-Pentanol (138751-71-0) |
| (R*,R*)-2-nitro-3-Pentanol (138668-26-5) |
| (R*,S*)-2-nitro-3-Pentanol (138668-19-6) |
| 3-nitro-1-Pentanol (135462-98-5) |
| (R)-5-nitro-2-Pentanol (129520-35-0) |
| (S)-5-nitro-2-Pentanol (120293-75-6) |
| 4-nitro-1-Pentanol (116435-64-4) |
| (±)-3-methyl-3-nitro-2-Butanol (114613-30-8) |
| (S)-3-methyl-3-nitro-2-Butanol (109849-50-5) |
| 3-methyl-4-nitro-2-Butanol (96597-30-7) |
| (±)-5-nitro-2-Pentanol (78174-81-9) |
| 2-methyl-2-nitro-1-Butanol (77392-55-3) |
| 3-methyl-2-nitro-1-Butanol (77392-54-2) |
| 3-methyl-4-nitro-1-Butanol (75694-89-2) |
| 2-methyl-4-nitro-2-Butanol (72183-50-7) |
| 3-methyl-3-nitro-1-Butanol (65102-50-3) |
| 5-nitro-2-Pentanol (54045-33-9) |
| 2-methyl-3-aci-nitro-2-Butanol (22916-79-6) |
| 2-methyl-1-aci-nitro-2-Butanol (22916-78-5) |
| 2-methyl-3-nitro-2-Butanol (22916-77-4) |
| 2-methyl-1-nitro-2-Butanol (22916-76-3) |
| 5-nitro-1-Pentanol (21823-27-8) |
| 2-methyl-3-nitro-1-Butanol (21527-53-7) |
| 2-nitro-3-Pentanol (20575-40-0) |
| 3-methyl-3-nitro-2-Butanol (20575-38-6) |
| 3-nitro-2-Pentanol (5447-99-4) |
| 2-nitro-1-Pentanol (2899-90-3) |
| 3-methyl-1-nitro-2-Butanol (2224-38-6) |
| 1-nitro-2-Pentanol (2224-37-5) |

| C6 Nitro Alcohols |
|---|
| (-)-4-methyl-1-nitro-2-Pentanol (158072-33-4) |
| 3-(nitromethyl)-3-Pentanol (156544-56-8) |
| (R*,R*)-3-methyl-2-nitro-3-Pentanol (148319-17-9) |
| (R*,S*)-3-methyl-2-nitro-3-Pentanol (148319-16-8) |
| 6-nitro-2-Hexanol (146353-95-9) |
| (±)-6-nitro-3-Hexanol (144179-63-5) |
| (S)-6-nitro-3-Hexanol (144139-33-3) |
| (R)-6-nitro-3-Hexanol (144139-32-2) |
| 3-nitro-2-Hexanol (127143-52-6) |
| 5-nitro-2-Hexanol (110364-37-9) |
| 4-methyl-1-nitro-2-Pentanol (102014-44-8) |
| (R*,S*)-2-methyl-4-nitro-3-Pentanol (82945-29-7) |
| (R*,R*)-2-methyl-4-nitro-3-Pentanol (82945-20-8) |
| 2-methyl-5-nitro-2-Pentanol (79928-61-3) |
| 2,3-dimethyl-1-nitro-2-Butanol (68454-59-1) |
| 2-methyl-3-nitro-2-Pentanol (59906-62-6) |
| 3,3-dimethyl-1-nitro-2-Butanol (58054-88-9) |
| 2,3-dimethyl-3-nitro-2-Butanol (51483-61-5) |
| 2-methyl-1-nitro-2-Pentanol (49746-26-1) |
| 3,3-dimethyl-2-nitro-1-Butanol (37477-66-0) |
| 6-nitro-1-Hexanol (31968-54-4) |
| 2-methyl-3-nitro-1-Pentanol (21527-55-9) |
| 2,3-dimethyl-3-nitro-1-Butanol (21527-54-8) |
| 2-methyl-4-nitro-3-Pentanol (20570-70-1) |
| 2-methyl-2-nitro-3-Pentanol (20570-67-6) |
| 2-nitro-3-Hexanol (5448-00-0) |
| 4-nitro-3-Hexanol (5342-71-2) |
| 4-methyl-4-nitro-1-Pentanol (5215-92-9) |
| 1-nitro-2-Hexanol (2224-40-0) |

| C7 Nitro Alcohols |
|---|
| 1-nitro-4-Heptanol (167696-66-4) |
| (R)-1-nitro-2-Heptanol (146608-19-7) |
| 7-nitro-1-Heptanol (133088-94-5) |
| (R*,S*)-3-nitro-2-Heptanol (127143-73-1) |
| (R*,R*)-3-nitro-2-Heptanol (127143-72-0) |
| (R*,S*)-2-nitro-3-Heptanol (127143-71-9) |
| (R*,R*)-2-nitro-3-Heptanol (127143-70-8) |
| (R*,S*)-2-methyl-5-nitro-3-Hexanol (103077-95-8) |
| (R*,R*)2-methyl-5-nitro-3-Hexanol (103077-87-8) |
| 3-ethyl-4-nitro-1-Pentanol (92454-38-1) |
| 3-ethyl-2-nitro-3-Pentanol (77922-54-4) |
| 2-nitro-3-Heptanol (61097-77-6) |
| 2-methyl-1-nitro-3-Hexanol (35469-17-1) |
| 2-methyl-4-nitro-3-Hexanol (20570-71-2) |
| 2-methyl-2-nitro-3-Hexanol (20570-69-8) |
| 5-methyl-5-nitro-2-Hexanol (7251-87-8) |
| 1-nitro-2-Heptanol (6302-74-5) |
| 3-nitro-4-Heptanol (5462-04-4) |
| 4-nitro-3-Heptanol (5342-70-1) |

| C8 Nitro Alcohols |
|---|
| (±)-1-nitro-3-Octanol (141956-93-6) |
| 1-nitro-4-Octanol (167642-45-7) |
| (S)-1-nitro-4-Octanol (167642-18-4) |
| 6-methyl-6-nitro-2-Heptanol (142991-77-3) |
| (R*,S*)-2-nitro-3-Octanol (135764-74-8) |
| (R*,R*)-2-nitro-3-Octanol (135764-73-7) |
| 5-nitro-4-Octanol (132272-46-9) |
| (R*,R*)-3-nitro-4-Octanol (130711-79-4) |
| (R*,S*)-3-nitro-4-Octanol (130711-78-3) |
| 4-ethyl-2-nitro-3-Hexanol (126939-74-0) |
| 2-nitro-3-Octanol (126939-73-9) |
| 1-nitro-3-Octanol (126495-48-5) |
| (R*,R*)-(±)-3-nitro-4-Octanol (118869-22-0) |
| (R*,S*)-(±)-3-nitro-4-Octanol (118869-21-9) |
| 3-nitro-2-Octanol (127143-53-7) |
| (R*,S*)-2-methyl-5-nitro-3-Heptanol (103078-03-1) |
| (R*,R*)-2-methyl-5-nitro-3-Heptanol (103077-90-3) |
| 8-nitro-1-Octanol (101972-90-1) |
| (±)-2-nitro-1-Octanol (96039-95-1) |
| 3,4-dimethyl-1-nitro-2-Hexanol (64592-02-5) |
| 3-(nitromethyl)-4-Heptanol (35469-20-6) |
| 2,5-dimethyl-1-nitro-3-Hexanol (35469-19-3) |
| 2-methyl-1-nitro-3-Heptanol (35469-18-2) |
| 2,4,4-trimethyl-1-nitro-2-Pentanol (35223-67-7) |
| 2,5-dimethyl-4-nitro-3-Hexanol (22482-65-1) |
| 2-nitro-1-Octanol (2882-67-9) |
| 1-nitro-2-Octanol (2224-39-7) |

| C9 Nitro Alcohols |
|---|
| 4-nitro-3-Nonanol (160487-89-8) |
| (R*,R*)-3-ethyl-2-nitro-3-Heptanol (148319-18-0) |
| 2,6-dimethyl-6-nitro-2-Heptanol (117030-50-9) |
| (R*,S*)-2-nitro-4-Nonanol (103077-93-6) |
| (R*,R*)-2-nitro-4-Nonanol (103077-85-6) |
| 2-nitro-3-Nonanol (99706-65-7) |
| 9-nitro-1-Nonanol (81541-84-6) |
| 2-methyl-1-nitro-3-Octanol (53711-06-1) |
| 4-nitro-5-Nonanol (34566-13-7) |
| 2-methyl-3-(nitromethyl)-3-Heptenol (5582-88-7) |
| 1-nitro-2-Nonanol (4013-87-0) |

| C10 Nitro Alcohols |
|---|
| 2-nitro-4-Decanol (141956-94-7) |
| (R*,S*)-3-nitro-4-Decanol (135764-76-0) |
| (R*,R*)-3-nitro-4-Decanol (135764-75-9) |
| 5,5-dimethyl-4-(2-nitroethyl)-1-Hexanol (133088-96-7) |
| (R*,R*)-(±)-3-nitro-4-Decanol (118869-20-8) |
| (R*,S*)-(±)-3-nitro-4-Decanol (118869-19-5) |
| 5-nitro-2-Decanol (112882-29-8) |
| 3-nitro-4-Decanol (93297-82-6) |
| 4,6,6-trimethyl-1-nitro-2-Heptanol (85996-72-1) |
| 2-methyl-2-nitro-3-Nonanol (80379-17-5) |
| 1-nitro-2-Decanol (65299-35-6) |
| 2,2,4,4-tetramethyl-3-(nitromethyl)-3-Pentanol (58293-26-8) |

| C11 Nitro Alcohols |
|---|
| 11-nitro-5-Undecanol (167696-69-7) |
| (R*,R*)-2-nitro-3-Undecanol (144434-56-0) |
| (R*,S*)-2-nitro-3-Undecanol (144434-55-9) |
| 2-nitro-3-Undecanol (143464-92-0) |
| 2,2-dimethyl-4-nitro-3-Nonanol (126939-76-2) |
| 4,8-dimethyl-2-nitro-1-Nonanol (118304-30-6) |
| 11-nitro-1-Undecanol (81541-83-5) |

| C12 Nitro Alcohols |
|---|
| 2-methyl-2-nitro-3-Undecanol (126939-75-1) |
| 2-nitro-1-Dodecanol (62322-32-1) |
| 1-nitro-2-Dodecanol (62322-31-0) |
| 2-nitro-3-Dodecanol (82981-40-6) |
| 12-nitro-1-Dodecanol (81541-78-8) |

**Table 26 -**

| Exemplary Compounds of Formula R₅-OH (CAS No./Aldrich No.) | | |
|---|---|---|
| 3-BROMO-1-PROPANOL | 627189 | 167169 |
| 1,3-DICHLORO-2-PROPANOL | 96231 | 184489 |
| 3-CHLORO-2,2-DIMETHYL-1-PROPANOL | 13401564 | 189316 |
| 2,2-BIS(CHLOROMETHYL)-1-PROPANOL | 5355544 | 207691 |
| 1,3-DIFLUORO-2-PROPANOL | 453134 | 176923 |
| 2-(METHYLTHIO)ETHANOL | 5271385 | 226424 |
| 2-(DIBUTYLAMINO)ETHANOL | 102818 | 168491 |
| 2-(DIISOPROPYLAMINO)ETHANOL | 96800 | 168726 |
| 3-METHYL-3-BUTEN-1-OL | 763326 | 129402 |
| 2-METHYL-3-BUTEN-2-OL | 115184 | 136816 |
| 3-METHYL-2-BUTEN-1-OL | 556821 | 162353 |
| 4-HEXEN-1-OL | 928927 | 237604 |
| 5-HEXEN-1-OL | 821410 | 230324 |
| CIS-2-HEXEN-1-OL | 928949 | 224707 |
| TRANS-3-HEXEN-1-OL | 928972 | 224715 |
| TRANS-2-HEXEN-1-OL | 928950 | 132667 |
| (+/-)-6-METHYL-5-HEPTEN-2-OL | 4630062 | 195871 |
| DIHYDROMYRCENOL | 18479588 | 196428 |
| TRANS,TRANS-2,4-HEXADIEN-1-OL | 17102646 | 183059 |
| 2,4-DIMETHYL-2,6-HEPTADIEN-1-OL | 80192569 | 238767 |
| GERANIOL | 106241 | 163333 |
| 3-BUTYN-1-OL | 927742 | 130850 |
| 3-PENTYN-1-OL | 10229104 | 208698 |
| ISETHIONIC ACID, SODIUM SALT | 1562001 | 220078 |
| (4-(2-HYDROXYETHYL)-1-PIPERAZINE-PROPANESULFONIC ACID) | 16052065 | 163740 |
| HEPES, SODIUM SALT | 75277393 | 233889 |
| 1-METHYLCYCLOPROPANEMETHANOL | 2746147 | 236594 |
| 2-METHYLCYCLOPROPANEMETHANOL | 6077721 | 233811 |
| (+/-)-CHRYSANTHEMYL ALCOHOL | 18383590 | 194654 |
| CYCLOBUTANEMETHANOL | 4415821 | 187917 |
| 3-CYCLOPENTYL-1-PROPANOL | 767055 | 187275 |
| 1-ETHYNYLCYCLOPENTANOL | 17356193 | 130869 |
| 3-METHYLCYCLOHEXANOL | 591231 | 139734 |
| 3,3,5,5-TETRAMETHYLCYCLOHEXANOL | 2650400 | 190624 |
| 4-CYCLOHEXYL-1-BUTANOL | 4441570 | 197408 |
| DIHYDROCARVEOL | 619012 | 218421 |
| (1S,2R,5S)-(+)-MENTHOL | 15356704 | 224464 |
| (1S,2S,5R)-(+)-NEOMENTHOL | 2216526 | 235180 |
| (1S,2R,5R)-(+)-ISOMENTHOL | 23283978 | 242195 |
| (+/-)-3-CYCLOHEXENE-1-METHANOL | 72581329 | 162167 |
| (+)-P-MENTH-1-EN-9-OL | 13835308 | 183741 |
| (S)-(-)-PERILLYL ALCOHOL | 536594 | 218391 |
| TERPINEN-4-OL | 562743 | 218383 |
| ALPHA-TERPINEOL | 98555 | 218375 |
| (+/-)-TRANS-P-MENTH-6-ENE-2,8-DIOL | 32226543 | 247774 |
| CYCLOHEPTANEMETHANOL | 4448753 | 138657 |
| TETRAHYDROFURFURYL ALCOHOL | 97994 | 185396 |
| (S)-(+)-2-PYRROLIDINEMETHANOL | 23356969 | 186511 |
| 1-METHYL-2-PYRROLIDINEETHANOL | 67004642 | 139513 |
| 1-ETHYL-4-HYDROXYPIPERIDINE | 3518830 | 224634 |
| 3-HYDROXYPIPERIDINE HYDROCHLORIDE | 64051792 | 174416 |
| (+/-)-2-PIPERIDINEMETHANOL | 3433372 | 155225 |
| 3-PIPERIDINEMETHANOL | 4606659 | 155233 |
| 1-METHYL-2-PIPERIDINEMETHANOL | 20845345 | 155241 |
| 1-METHYL-3-PIPERIDINEMETHANOL | 7583531 | 146145 |
| 2-PIPERIDINEETHANOL | 1484840 | 131520 |
| 4-HYDROXYPIPERIDINE | 5382161 | 128775 |
| 4-METHYL-1-PIPERAZINEPROPANOL | 5317339 | 238716 |
| EXO-NORBORNEOL | 497370 | 179590 |
| ENDO-NORBORNEOL | 497369 | 186457 |
| 5-NORBORNENE-2-METHANOL | 95125 | 248533 |
| (+/-)-3-METHYL-2-NORBORNANEMETHANOL | 6968758 | 130575 |
| ((1S)-ENDO)-(-)-BORNEOL | 464459 | 139114 |
| (1R)-ENDO-(+)-FENCHYL ALCOHOL | 2217029 | 196444 |
| 9-ETHYLBICYCLO(3.3.1)NONAN-9-OL | 21951333 | 193895 |
| (+/-)-ISOPINOCAMPHEOL | 51152115 | 183229 |
| (S)-CIS-VERBENOL | 18881044 | 247065 |
| (1R,2R,3R,5S)-(-)-ISOPINOCAMPHEOL | 25465650 | 221902 |
| (1R)-(-)-MYRTENOL | 515004 | 188417 |
| 1-ADAMANTANOL | 768956 | 130346 |
| 3,5-DIMETHYL-1-ADAMANTANOL | 707379 | 231290 |
| 2-ADAMANTANOL | 700572 | 153826 |
| 1-ADAMANTANEMETHANOL | 770718 | 184209 |
| 1-ADAMANTANEETHANOL | 6240115 | 188115 |
| 3-FURANMETHANOL | 4412913 | 196398 |
| FURFURYL ALCOHOL | 98000 | 185930 |
| 2-(3-THIENYL)ETHANOL | 13781674 | 228796 |
| 4-METHYL-5-IMIDAZOLEMETHANOL HYDROCHLORIDE | 38585625 | 227420 |
| METRONIDAZOLE | 443481 | 226742 |
| 4-(HYDROXYMETHYL)IMIDAZOLE HYDROCHLORIDE | 32673419 | 219908 |
| 4-METHYL-5-THIAZOLEETHANOL | 137008 | 190675 |
| 2-(2-HYDROXYETHYL)PYRIDINE | 103742 | 128643 |
| 2-HYDROXY-6-METHYLPYRIDINE | 3279763 | 128740 |
| 4-PYRIDYLCARBINOL | 586958 | 151629 |
| 3-PYRIDYLCARBINOL N-OXIDE | 6968725 | 184446 |
| 1-BENZYL-4-HYDROXYPIPERIDINE | 4727724 | 152986 |
| 1-(4-CHLOROPHENYL)-1-CYCLOPENTANEMETHANOL | 80866791 | 188697 |
| (4S,5S)-(-)-2-METHYL-5-PHENYL-2-OXAZOLINE-4-METHANOL | 53732415 | 187666 |
| 6-(4-CHLOROPHENYL)-4,5-DIHYDRO-2-(2-HYDROXYBUTYL)-3(2H)-PYRIDAZINONE | 38958826 | 243728 |
| N-(2-HYDROXYETHYL)PHTHALIMIDE | 3891074 | 138339 |
| 2-NAPHTHALENEETHANOL | 1485070 | 188107 |
| 1-NAPHTHALENEETHANOL | 773999 | 183458 |
| 2-ISOPROPYLPHENOL | 88697 | 129526 |
| 4-CHLORO-ALPHA,ALPHADIMETHYLPHENETHYL ALCOHOL | 5468973 | 130559 |
| 4-FLUORO-ALPHA-METHYLBENZYL ALCOHOL | 403418 | 132705 |
| 3-PHENYL-1-PROPANOL | 122974 | 140856 |
| 3-(4-METHOXYPHENYL)-1-PROPANOL | 5406188 | 142328 |
| 4-FLUOROPHENETHYL ALCOHOL | 7589277 | 154172 |
| 4-METHOXYPHENETHYL ALCOHOL | 702238 | 154180 |
| TRANS-2-METHYL-3-PHENYL-2-PROPEN-1-OL | 1504558 | 155888 |
| 2-ANILINOETHANOL | 122985 | 156876 |
| 3-FLUOROBENZYL ALCOHOL | 456473 | 162507 |
| 2-FLUOROBENZYL ALCOHOL | 446515 | 162515 |
| 2-METHYL-1-PHENYL-2-PROPANOL | 100867 | 170275 |
| ALPHA-(CHLOROMETHYL)-2,4-DICHLOROBENZYL ALCOHOL | 13692143 | 178403 |
| 2-PHENYL-1-PROPANOL | 1123859 | 179817 |
| 4-CHLOROPHENETHYL ALCOHOL | 1875883 | 183423 |
| 4-BROMOPHENETHYL ALCOHOL | 4654391 | 183431 |
| 4-NTTROPHENETHYL ALCOHOL | 100276 | 183466 |
| 2-NITROPHENETHYL ALCOHOL | 15121843 | 183474 |
| BETA-ETHYLPHENETHYL ALCOHOL | 2035941 | 183482 |
| 4-PHENYL-1-BUTANOL | 3360416 | 184756 |
| 2-METHOXYPHENETHYL ALCOHOL | 7417187 | 187925 |
| 3-METHOXYPHENETHYL ALCOHOL | 5020417 | 187933 |
| 3-PHENYL-1-BUTANOL | 2722363 | 187976 |
| 2-METHYLPHENETHYL ALCOHOL | 19819988 | 188123 |
| 3-METHYLPHENETHYL ALCOHOL | 1875894 | 188131 |
| 4-METHYLPHENETHYL ALCOHOL | 699025 | 188158 |
| 5-PHENYL-1-PENTANOL | 10521912 | 188220 |
| 4-(4-METHOXYPHENYL)-1-BUTANOL | 22135508 | 188239 |
| 4-(4-NITROPHENYL)-1-BUTANOL | 79524202 | 188751 |
| 3,3-DIPHENYL-1-PROPANOL | 20017678 | 188972 |
| 1-PHENYL-2-PROPANOL | 14898874 | 189235 |
| (+/-)-ALPHA-ETHYLPHENETHYL ALCOHOL | 701702 | 190136 |
| 1,1-DIPHENYL-2-PROPANOL | 29338496 | 190756 |
| 3-CHLOROPHENETHYL ALCOHOL | 5182445 | 193518 |
| 2-CHLOROPHENETHYL ALCOHOL | 19819955 | 193844 |
| (+/-)-1-PHENYL-2-PENTANOL | 705737 | 195286 |
| 2,2-DIPHENYLETHANOL | 1883325 | 196568 |
| 4-ETHOXY-3-METHOXYPHENETHYL ALCOHOL | 77891293 | 197599 |
| 3,4-DIMETHOXYPHENETHYL ALCOHOL | 7417212 | 197653 |
| 3-(3,4-DIMETHOXYPHENYL)-1-PROPANOL | 3929473 | 197688 |
| 2-(4-BROMOPHENOXY)ETHANOL | 34743889 | 198765 |
| 2-FLUOROPHENETHYL ALCOHOL | 50919067 | 228788 |
| 3-(TRIFLUOROMETHYL)PHENETHYL ALCOHOL | 455016 | 230359 |
| 2-(PHENYLTHIO)ETHANOL | 699127 | 232777 |
| 1-(2-METHOXYPHENYL)-2-PROPANOL | 15541261 | 233773 |

**Table 27 -**

| Exemplary Method Embodiments of Processes A-R |
|---|
| A; B; C; D; I; J; K; L; M; N; O; P; Q; R; E; F; G; H; AB; BC; CD; DI; IJ; JK; KL; LM; MN; NO; OP; OQ; QR; EF; FG; GH; HI; ABC; BCD; CDI; DIJ; IJK; JKL; KLM; LMN; MNO; NOP; NOQ; OQR; EFG; FGH; GHI; HIJ; ABDC; BCDI; CDIJ; DIJK; IJKL; JKLM; KLMN; LMNO; MNOP; MNOQ; NOQR; EFHG; FGHI; GHIJ; HIJK; ABCDI; BCDIJ; CDIJK; DIJKL; IJKLM; JKLMN; KLMNO; LMNOP; LMNOQ; MNOQR; EFGHI; FGHIJ; GHIJK; HIJKL; ABCDIJ; BCDIJK; CDIJKL; DIJKLM; IJKLMN; JKLMNO; KLMNOP; KLMNOQ; LMNOQR; EFGHIJ; FGHIJK; GHIJKL; HIJKLM; ABCDIJK; BCDIJKL; CDIJKLM; DIJKLMN; IJKLMNO; JKLMNOP; JKLMNOQ; KLMNOQR; EFGHIJK; FGHIJKL; GHIJKLM; HIJKLMN; ABCDIJKL; BCDIJKLM; CDIJKLMN; DIJKLMNO; IJKLMNOP; IJKLMNOQ; JKLMNOQR; EFGHIJKL; FGHIJKLM; GHIJKLMN; HIJKLMNO; ABCDIJKLM; BCDIJKLMN; CDIJKLMNO; DIJKLMNOP; DIJKLMNOQ; IJKLMNOQR; EFGHIJKLM; FGHIJKLMN; GHIJKLMNO; HIJKLMNOP; HIJKLMNOQ; ABCDIJKLMN; BCDIJKLMNO; CDIJKLMNOP; CDIJKLMNOQ; DIJKLMNOQR; EFGHIJKLMN; FGHIJKLMNO; GHIJKLMNOP; GHIJKLMNOQ; HIJKLMNOQR; ABCDIJKLMNO; BCDIJKLMNOP; BCDIJKLMNOQ; CDIJKLMNOQR; EFGHIJKLMNO; FGHIJKLMNOP; FGHIJKLMNOQ; GHIJKLMNOQR; ABCDIJKLMNOP; ABCDIJKLMNOQ; BCDIJKLMNOQR; EFGHIJKLMNOP; EFGHIJKLMNOQ; FGHIJKLMNOQR; ABCDIJKLMNOQR; EFGHIJKLMNOQR; S; T; U; V; W; ST; TU; UV; VW; STU; TUV; UVW; STUV; TUVW; STUVW. |

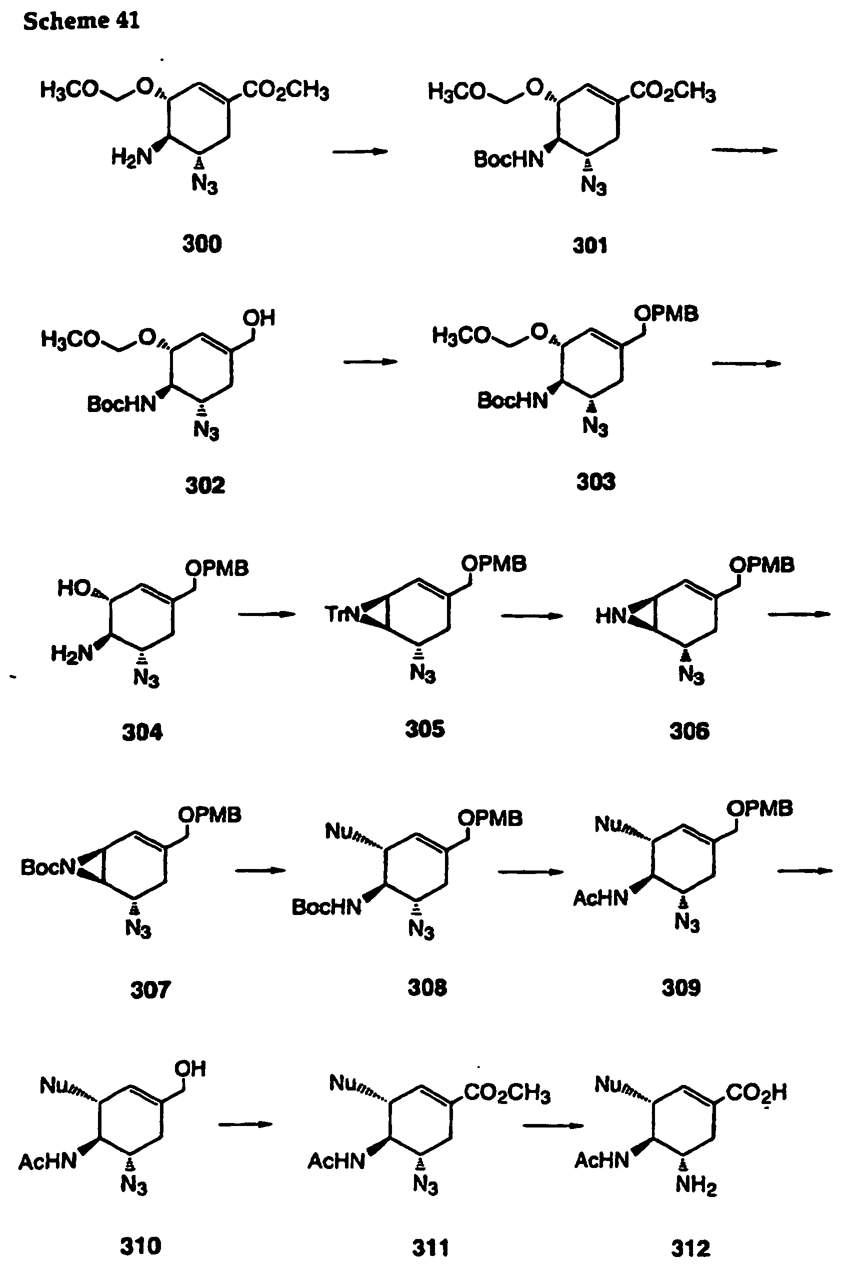

### Scheme 41

The amine **300** (an intermediate in Example 52, optionally purified prior to use) is treated with Boc anhydride to give the mono Boc protected amine **301**. Such a transformation is found in Greene, T.W. "Protective Groups in Organic Synthesis" 2nd Ed. (John Wiley & Sons, New York, 1991) pages 327-328.

Methyl ester **301** is reduced to the corresponding primary allylic alcohol **302** with DIBAL at low temperature. Such a conversion is described by Garner, P. and Park, J. M., "J. Org. Chem.", 52:2361 (1987).

The primary alcohol **302** is protected as its *p*-methoxy benzyl ether derivative **303** by treatment with 4-methoxybenzyl chloride under basic conditions. Such a conversion is described in Horita, K. et. al., "Tetrahedron", 42:3021 (1986).

The MOM and Boc protecting groups of **303** are removed by treatment with TFA/CH₂Cl₂ to give the amino alcohol **304**. Such transformations are found in Greene, T.W "Protective Groups in Organic Synthesis", 2nd. Ed. (John Wiley & Sons, New York, 1991).

Conversion of **304** into the corresponding trityl protected aziridine **305** is accomplished in a one pot reaction two step sequence: 1) TrCl/TEA, 2) MsCl/TEA. Such a transformation has been previously described.

Aziridine **305** is then converted the corresponding Boc protected derivative **307** by first removal of the trityl group with HCl/acetone to give **306**. Such a transformation is described in Hanson, R. W. and Law, H. D. "J. Chem. Soc.", 7285 (1965). Aziridine **306** is then converted into the corresponding Boc derivative **307** by treatment with Boc anhydride. Such a conversion is described in Fitremann, J., et. al. "Tetrahedron Lett.", 35:1201 (1994).

The allylic aziridine **307** is opened selectively at the allylic position with a carbon nucleophile delivered via a higher order organocuprate in the presence of BF₃·Et₂O at low temperature to give the opened adduct **308.** Such an opening is described in Hudlicky, T., et. al. "Synlett." 1125 (1995).

The Boc protected amine **308** is converted into the N-acetyl derivative **309** in a two step sequence: 1) TFA/CH₂Cl₂; 2) Ac₂O/pyridine. Such transformations can be found in Greene, T.W., "Protective Groups in Organic Synthesis", 2nd. Ed. John Wiley & Sons, New York, 1991) pages 327-328 and pages 351-352.

Benzyl ether **309** is deprotected with DDQ at room temperature to give the primary allylic alcohol **310.** Such a transformation is found in Horita, K., et. al. "Tetrahedron" 42:3021 (1986).

Alcohol **310** is oxidized and converted in a one pot reaction into the methyl ester **311** via a Corey oxidation using MnO2/AcOH/MeOH/NaCN. Such a transformation can be found in Corey, E. J., et. al. "J. Am. Chem. Soc.", 90:5616 (1968).

Azido ester **311** is converted into amino acid **312** in a two step sequence 1) Ph₃P/H₂O/THF; 2) KOH/THF. Such a conversion has been described previously.

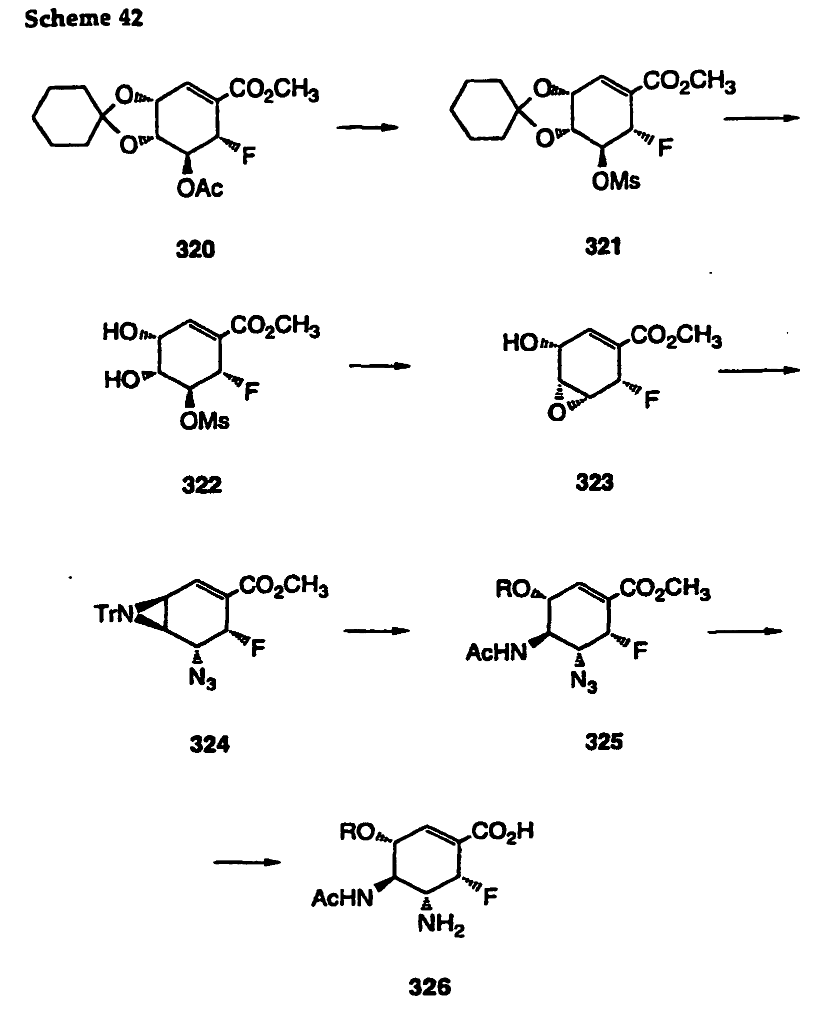

### Scheme 42

The known fluoro acetate **320** (Sutherland, J. K., et.al. "J. Chem. Soc. Chem. Commun." 464 (1993) is deprotected to the free alcohol and then converted into the corresponding mesylate **321** in two steps: 1) NaOMe; 2) MsCl/TEA. Such transformations are described in Greene, T.W., "Protective Groups in Organic Synthesis", 2nd. Ed. (John Wiley & Sons, New York, 1991).

Deprotection of **321** under acidic conditions gives diol **322** which is cyclized to the epoxy alcohol **323** under basic conditions. Such a conversion has been previously described.

Conversion of **323** to the N-trityl protected aziridine **324** is accomplished with the following sequence: 1) MOMCl/TEA; 2) NaN₃/NH₄Cl; 3) MsCl/TEA; 4) PPh₃/TEA/H₂O; 5) NaN₃/NH₄Cl; 6) HCl/MeOH; 7) i)TrCl, ii) MsCl/TEA. Such a sequence has been previously described.

The aziridine **324** is then opened with the appropriate alcohol under Lewis acid conditions and then treated with Ac₂O/pyridine to give the acetylated product **325.** Such a transformation has been previously described.

The ester 325 is converted to the corresponding amino acid **326** in a two step sequence: 1) PPh₃/H₂O/THF; 2) KOH/THF. Such a transformation has been previously described.

United States Patent No. 5,214,165, and in particular, the "Descriptions and Examples" at column 9, line 61 to column 18, line 26, describes the preparation of 6α and 6β fluoro Shikimic acid. These fluoro compounds are suitable starting materials for methods of making compounds of the invention that use Shikimic acid.

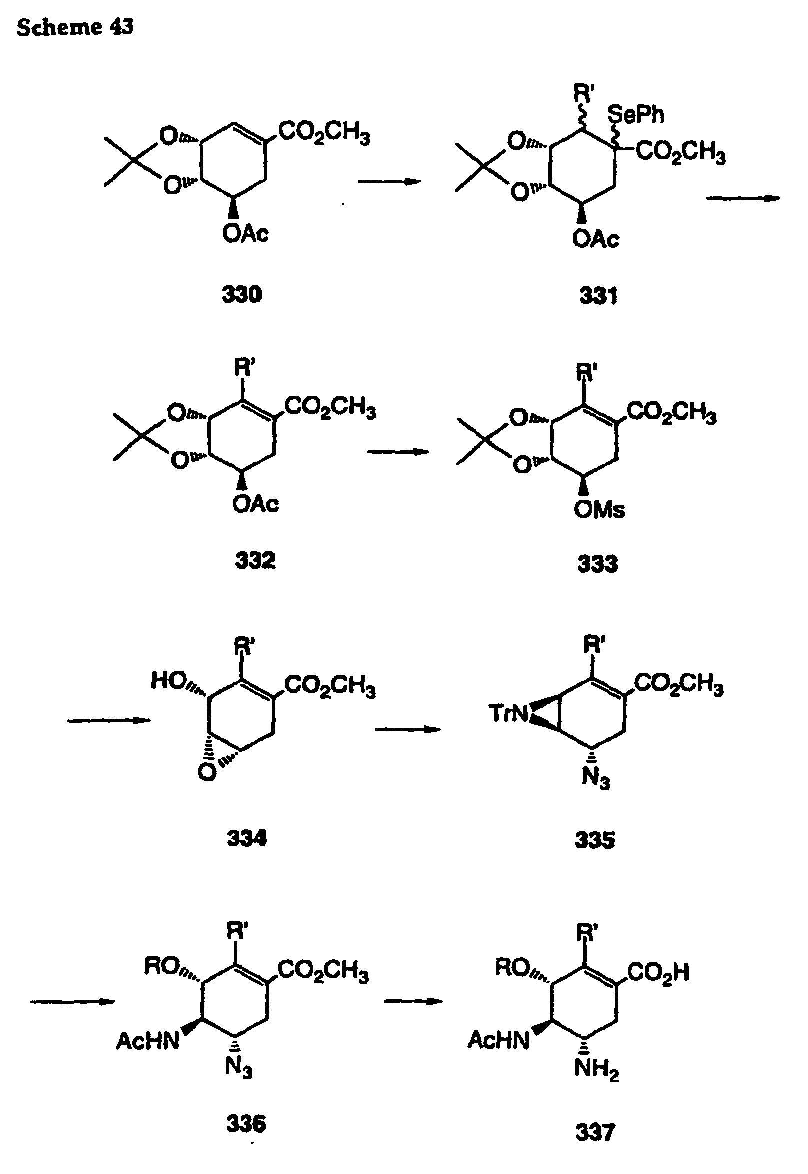

### Scheme 43

Unsaturated ester **330** (obtainable by standard actetylation methods from the acetonide alcohol described in Campbell, M. M., et. al., "Synthesis", 179 (1993)) is reacted with the appropriate organocuprate where R' is the ligand to be transferred from the organocuprate. The resultant intermediate is then trapped with PhSeCl to give **331** which is then treated with 30% H₂O₂ to give the α,β-unsaturated ester **332**. Such a transformation can be found in Hayashi, Y., et. al, "J. Org. Chem." 47:3428 (1982).

Acetate **332** is then converted into the corresponding mesylate **333** in a two step sequence: 1) NaOMe/MeOH; 2) MsCl/TEA. Such a transformation has been previously described and can also be found in Greene, T.W., "Protective Groups in Organic Synthesis", 2nd. Ed. (John Wiley & Sons, New York,1991).

The acetonide **333** is then converted into the epoxy alcohol **334** in a two step sequence: 1) *p*-TsOH/MeOH/Δ; 2) DBU/THF. Such a transformation has been previously described.

Conversion of epoxide **334** into N-trityl aziridine **335** is accomplished by the following sequence: 1) MOMCl/TEA; 2) NaN₃/NH₄Cl; 3) MsCl/TEA; 4) PPh₃/TEA/H₂O; 5) MaN₃/NH₄Cl; 6) HCl/MeOH; 7) i)TrCl, ii) MsCl/TEA. Such a sequence has been previously described.

The aziridine **335** is then opened with the appropriate alcohol under Lewis acid conditions and then treated with Ac₂O/pyridine to give the acetylated product **336.** Such a transformation has been previously described.

The azido ester **336** is converted to the corresponding amino acid **337** in a two step sequence: 1) PPh₃/H₂O/THF; 2) KOH/THF. Such a transformation has been previously described.

Schemes 44 and 45 are referred to in the examples.

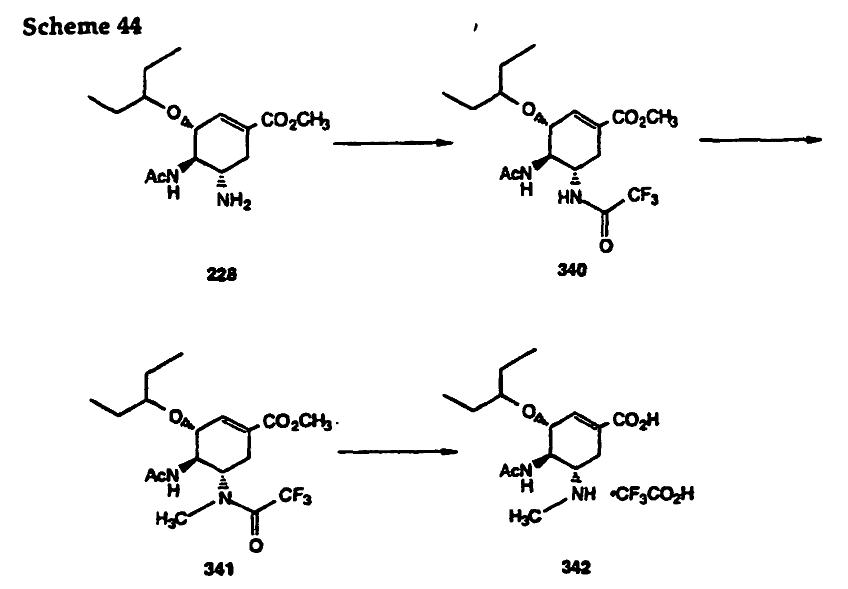

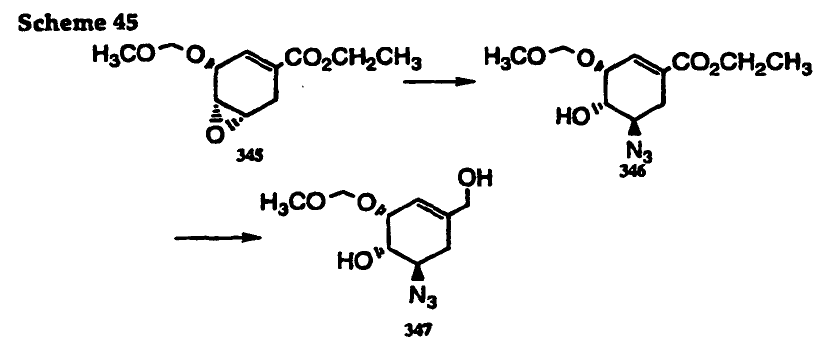

Modification of the exemplary starting materials to form different E₁ groups has been described in detail and will not be elaborated here. See Fleet, G.W.J. et al.; "J. Chem. Soc. Perkin Trans. I", 905-908 (1984), Fleet, G.W.J. et al.; "J. Chem. Soc., Chem. Commun.", 849-850 (1983), Yee, Ying K. et al.; "J. Med. Chem.", 33:2437-2451 (1990); Olson, R.E. et al.; "Bioorganic & Medicinal Chemistry Letters", 4(18):2229-2234 (1994); Santella, J.B. III et al.; "Bioorganic & Medicinal Chemistry Letters", 4(18):2235-2240 (1994); Judd, D.B. et al.; "J. Med. Chem.", 37:3108-3120 (1994) and Lombaert, S. De et al.; "Bioorganic & Medicinal Chemistry Letters", 5(2):151-154 (1994).

The E₁ sulfur analogs of the carboxylic acid compounds of the invention are prepared by any of the standard techniques. By way of example and not limitation, the carboxylic acids are reduced to the alcohols by standard methods. The alcohols are converted to halides or sulfonic acid esters by standard methods and the resulting compounds are reacted with NaSH to produce the sulfide product. Such reactions are described in Patai, "The Chemistry of the Thiol Group" (John Wiley, New York, 1974), pt. 2, and in particular pages 721-735.

Modifications of each of the above schemes leads to various analogs of the specific exemplary materials produced above. The above cited citations describing suitable methods of organic synthesis are applicable to such modifications.

In each of the above exemplary schemes it may be advantageous to separate reaction products from one another and/or from starting materials. The desired products of each step or series of steps is separated and/or purified (hereinafter separated) to the desired degree of homogeneity by the techniques common in the art. Typically such separations involve multiphase extraction, crystallization from a solvent or solvent mixture, distillation, sublimation, or chromatography. Chromatography can involve any number of methods including, for example, size exclusion or ion exchange chromatography, high, medium, or low pressure liquid chromatography, small scale and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like.

Selection of appropriate methods of separation depends on the nature of the materials involved. For example, boiling point, and molecular weight in distillation and sublimation, presence or absence of polar functional groups in chromatography, stability of materials in acidic and basic media in multiphase extraction, and the like. One skilled in the art will apply techniques most likely to achieve the desired separation.

All literature and patent citations above are hereby expressly incorporated by reference at the locations of their citation. Specifically cited sections or pages of the above cited works are incorporated by reference with specificity. The invention has been described in detail sufficient to allow one of ordinary skill in the art to make and use the subject matter of the following claims. It is apparent that certain modifications of the methods and compositions of the following claims can be made within the scope and spirit of the invention.

### Examples

### General

The following Examples refer to the Schemes.

Some Examples have been performed multiple times. In repeated Examples, reaction conditions such as time, temperature, concentration and the like, and yields were within normal experimental ranges. In repeated Examples where significant modifications were made, these have been noted where the results varied significantly from those described. In Examples where different starting materials were used, these are noted. When the repeated Examples refer to a "corresponding" analog of a compound, such as a "corresponding ethyl ester", this intends that an otherwise present group, in this case typically a methyl ester, is taken to be the same group modified as indicated. For example, the "corresponding ethyl ester of compound 1" is

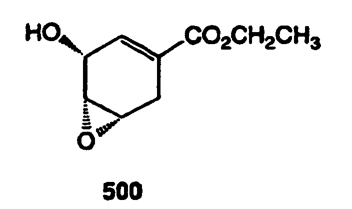

### Example 1

Epoxy alcohol 1: Prepared from shikimic acid by the procedure of McGowan and Berchtold, "J. Org. Chem.", 46:2381 (1981).

### Example 2

**Epoxy allyl ether 2:** To a solution of epoxy alcohol 1 (2.37g, 14.08 mmol) in dry benzene (50 mL) was added thallium(I)ethoxide (1.01 mL) in one portion. After 2 hr the reaction was concentrated *in vacuo* and the residue dissolved in acetonitrile. Allyl iodide (3.0 mL) was added and the mixture was stirred in the dark for 16 h. The solids were filtered thru a celite pad and washed with chloroform. Concentration *in vacuo* followed by flash chromatography (40% EtOAc in hexane) gave 1.24 g (42%) of 2 as a pale viscous oil. ¹H NMR (300 MHz, CDCl₃): δ 6.75 (1H, m); 6.10-5.90 (1H, m, -CH=, allyl); 5.40-5.15 (2H, m, =CH₂, allyl); 4.47-4.43 (1H, m); 4.30-4.15 (2H, m, -CH₂-, allyl); 3.73 (3H, s); 3.55-3.50 (1H, m); 3.45-3.40 (1H, m); 3.15-3.00 (1H, dm, *J* = 19.5 Hz), 2.50-2.35 (1H, dm, *J* = 2.7, 19.5 Hz).

### Example 3

**Azido alcohol 3:** Epoxide 2 (1.17 g, 5.57 mmol), sodium azide (1.82 g) and ammonium chloride (658 mg) were refluxed in MeOH/H₂O (8:1) (35 mL) for 18 h. The reaction was then concentrated *in vacuo* and the residue partitioned between ethyl ether and water. The organic layer was washed with brine and dried. Concentration *in vacuo* gave 3 as a pale oil 1.3 g (92%) which was used without further purification. ¹H NMR (300 MHz, CDCl₃): δ 6.95-6.85 (1H, m); 6.00-5.85 (1H, m, -CH=, allyl); 5.35-5.25 (2H, m, =CH₂, allyl); 4.25-4.10 (2H, m, -CH₂-, allyl); 4.12 (1H, bt, *J* =4.2 Hz); 3.95-3.75 (2H, m); 3.77 (3H, s); 2.85 (1H, dd, *J* =5.3, 18.3 Hz); 2.71 (1H, bs); 2.26 (1H, dd, *J* =7.2, 18.3 Hz).

### Example 4

**Aziridine 4:** To a solution of alcohol **3** (637 mg, 2.52 mmol) in CH₂Cl₂ (20 mL) cooled to 0°C was added DMAP (few crystals) and triethyl amine (442 µL). MsCl (287 µL) was then added and the reaction stirred for 2 h at 0°C. Volatiles were removed and the residue partitioned between ethyl ether and water. The organic layer was washed with saturated bicarbonate, brine and then dried. Concentration *in vacuo* gave 881 mg of crude mesylate. ¹H NMR (300 MHz, CDCl₃): δ 6.87-6.84 (1H, s); 6.00-5.85 (1H, m, -CH=, allyl); 5.40-5.25 (2H, m, =CH₂, allyl); 4.72 (1H, dd, *J* = 3.9, 85 Hz); 4.32 (1H, bt, *J* = 3.9 Hz); 4.30-4.15 (2H, m, -CH₂-, allyl); 3.77 (3H, s); 3.14 (3H, s); 2.95 (1H, dd, *J* = 5.7,18.6 Hz); 2.38 (1H, dd, *J* = 6.7,18.6 Hz).

The crude mesylate was dissolved in dry THF (20 mL) and treated with Ph₃P (727 mg). After stirring for 3 h at room temperature, water (15 mL) and solid NaHCO₃ (1.35 g) was added and the mixture stirred overnight at room temperature. The reaction was then concentrated *in vacuo* and the residue partitioned between EtOAc, saturated bicarbonate and brine. The organic layer was separated and dried over MgSO₄. Concentration *in vacuo* and flash chromatography of the residue gave the aziridine 4170 mg (33%) as a pale yellow oil. ¹H NMR (300 MHz, CDCl₃): δ 6.82-6.80 (1H, m); 6.04-5.85 (1H, m, -CH=, allyl); 5.35-5.20 (2H, m, =CH₂, allyl); 4.39 (1H, bd, *J* =2.4 Hz); 4.20-4.05 (2H, m, -CH₂-allyl); 3.73 (3H, s); 2.90-2.80 (1H, bd, *J* =18.9 Hz); 2.65-2.40 (2H, m).

### Example 5

**N-acetyl aziridine 5:** Aziridine **4** (170 mg, 0.814 mmol) was dissolved in CH₂Cl₂ (2 mL) and pyridine (4 mL) and cooled to 0°C. Acetyl chloride (87 µL) was then added and the reaction stirred at 0°C for 1 h. Volatiles were removed *in vacuo* and the residue partitioned between ethyl ether, saturated bicarbonate and brine. The organic layer was separated and dried over MgSO₄. Concentration gave crude 5 196 mg (96%) which was used without further purification. ¹H NMR (300 MHz, CDCl₃): δ 6.88-6.86 (1H, m); 6.00-5.85 (1H, m, -CH=, allyl); 5.40-5.20 (2H, m, =CH₂, allyl); 4.45-4.40 (1H, m); 4.16 (2H, d, *J* =6.0 Hz, -CH₂-, allyl); 3.76 (3H, s); 3.00-2.95 (2H, m); 2.65 (1H, bd, *J* =18.5 Hz); 2.14 (3H, s).

### Example 6

**Azido allyl ether 6:** Aziridine **5** (219 mg, 0.873 mmol), sodium azide (426 mg) and ammonium chloride (444 mg) in dry DMF (7 mL) was heated at 65°C under argon overnight. The reaction was poured into saturated bicarbonate/brine and extracted with ethyl ether several times. The combined ether layers were washed with brine and dried. Concentration followed by flash chromatography (EtOAc only) gave the azido amine 77 mg (35%) which was dissolved in CH₂Cl₂ (1 mL) and pyridine (1 mL) and cooled to 0°C. Acetyl chloride (38 µL) was added and after 45 min solid NaHCO₃ was added and the volatiles removed under vacuum. The residue was partitioned between EtOAc and brine. The organic layer was dried over MgSO₄ and concentrated *in vacuo.* Flash chromatography (EtOAc only) gave 6 90 mg (99%). ¹H NMR (500 MHz, CDCl₃): δ 6.86 (1H, bt, *J* =2.2 Hz); 5.95-5.82 (1H, m, CH=, allyl); 5.68 (1H, bd, *J* =7.3 Hz); 5.35-5.20 (2H, m, =CH₂, allyl); 4.58-4.52 (1H, m); 4.22-4.10 (2H, m); 4.04 (1H, dd, *J* =5.9, 12.5 Hz); 3.77 (3H, s); 3.54-3.52 (1H, m); 2.89 (1H, dd, *J* = 5.9,17.6 Hz); 2.32-2.22 (1H, m); 2.06 (3H, s).

### Example 7

**Azido diol 7:** To a solution of olefin **6** (90 mg, 0.306 mmol) in acetone (3 ml) and water (258 µL) was added N-methyl morpholine-N-oxide (39 mg) and OsO₄ (73 µL of a 2.5 % w/w in t-butanol). The reaction was then stirred at room temperature for 3 days. Solid sodium hydrosulfite was added and after stirring for 20 min the reaction was filtered thru a celite pad and washed with copious amounts of acetone. Concentration *in vacuo* followed by flash chromatography (10% MeOH in CH₂Cl₂) gave the diol **7** 50 mg (50%). ¹H NMR (300 MHz, CD₃CN): δ 6.80-6.70 (1H, m); 420-4.15 (1H, bm); 3.95-3.80 (1H, m); 3.80-3.25 (6H, m); 3.70 (3H, s); 3.10 (1H, bs); 2.85 (1H, bs); 2.85-2.75 (1H, m); 2.30-2.15 (1H, m); 2.16 (1H, bs); 1.92 (3H, s).

### Example 8

**Amino acid diol 8:** A solution of the diol **7** (23 mg, 0.07 mmol) in THF (1 mL) was treated with aq. KOH (223 µL, of 0.40 M solution) at room temperature. After stirring for 1.5 h the reaction was acidified to pH=4 with Amberlite IR-120 (plus) ion exchange resin. The resin was filtered and washed with MeOH. Concentration *in vacuo* gave the crude carboxylic acid which was dissolved in ethanol (1.5 mL). To this solution was added Lindlar's catalyst (20 mg) and the reaction stirred over a hydrogen atmosphere (1 atm via a balloon) for 20 h. The reaction mixture was filtered thru a celite pad and washed with hot ethanol and water. The ethanol was removed under vacuum and the resulting aqueous layer lyophilized to give a mixture of the desired amino acid 8 and the starting azide **7** as a white powder. Compound 8: ¹H NMR (500 MHz, D₂O): δ 6.5 (1H, s); 4.24-4.30 (2H, m); 4.25-4.18 (1H, m); 3.90-3.55 (5H, complex m); 2.96-2.90 (1H, m); 2.58-2.50 (1H, complex m); 2.12 (3H, s).

### Example 9

**Compound 62:** A suspension of Quinic acid (60 g), cyclohexanone (160 mL) and toluenesulfonic acid (600 mg) in benzene (450 mL) was refluxed with Dean-Stark for 14 hrs. The reaction mixture was cooled to room temperature and poured into saturated NaHCO₃ solution (150 mL). The aqueous layer was extracted with CH₂Cl₂ (3x). The combined organic layers were washed with water (2x), brine (1x), and dried over Na₂SO₄. Concentration gave a whited solid, which was recrystallized from ether (75 g, 95%): ¹H NMR (CDCl₃) δ 4.73 (dd, J = 6.1, 2.5 Hz, 1 H), 4.47 (ddd, J = 7.0, 7.0, 3.0 Hz, 1H), 4.30 (ddd, J = 5.4, 2.6, 1.4 Hz, 1H), 2.96 (s, 1H), 2.66 (d, J = 11.7 Hz, 1H), 2.40-2.15 (m, 3 H), 1.72-1.40 (m, 10 H).

### Example 10

**Compound 63:** To a solution of lactone **62** (12.7 g, 50 mmol) in methanol (300 mL) was added sodium methoxide (2.7 g, 50 mmol) in one portion. The mixture was stirred at room temperature for 3 hrs, and quenched with acetic acid (3 mL) and stirred for 10 min. The mixture was poured into saturated NH₄Cl solution (300 mL), and extracted with CH₂Cl₂ (3x). The combined organic phase was washed with brine (1x), and dried over MgSO₄. Purification by flash column chromatography (Hexane/EtOAc = 1/1 to 1/2) gave diol (11.5 g, 80%) and starting material (1.2 g, 10%): ¹H NMR (CDCl₃) δ 4.47 (ddd, J = 7.4, 5.8, 3.5 Hz, 1 H), 4.11 (m, 1 H), 3.98 (m, 1 H), 3.81 (s, 3 H), 3.45 (s, 1 H), 2.47 (d, J = 3.3 Hz, 1 H), 2.27 (m, 2 H), 2.10 (dd, J = 11.8, 4.3 Hz, 1 H), 1.92-1.26 (m, 10 H).

### Example 11

**Compound 64:** To a mixture of diol **63** (1.100 g, 3.9 mmol), molecule sieves (3 A, 2.2 g) and pyridine (1.1 g) in CH₂Cl₂ (15 mL) was added PCC (3.3 g, 15.6 mmol) in one portion. The mixture was stirred at room temperature for 26 hrs, and diluted with ether (30 mL). The suspension was filtered through a pad of celite, and washed with ether (2x20 mL). The combined ether was washed with brine (2x), and dried over MgSO₄. Concentration and purification was by flash column chromatography (Hexane/EtOAc = 3/1) gave the ketone (0.690 g, 67%): ¹H NMR (CDCl₃) δ 6.84 (d, J = 2.8 Hz, 1 H), 4.69 (ddd, J = 6.4, 4.9, 1.6 Hz, 1 H), 4.30 (d, J = 5.0 Hz, 1 H), 3.86 (s, 3 H), 3.45 (d, J = 22.3 Hz, 1 H), 2.86 (m, 1 H), 1.69-1.34 (m, 10 H).

### Example 12

**Compound 28:** To a solution of ketone **64** (0.630 g, 2.4 mmol) in MeOH (12 mL) at 0°C was added NaBH₄ in 30 min. The mixture was stirred for additional 1.5 hrs at 0°C, and quenched with 15 mL of saturated NH₄Cl solution. The solution was extracted with CH₂Cl₂ (3x), and the combined organic extract was dried over MgSO₄. Purification by flash column chromatography (Hexane/EtOAc = 2/1) gave the alcohol (0.614 g, 97%): ¹H NMR (CDCl₃) δ 6.94 (d, J = 0.5 Hz, 1 H), 4.64 (ddd, J = 9.8, 6.7, 3.2 Hz, 1 H), 4.55 (dd, J = 7.1,42 Hz, 1 H), 4.06 (m, 1 H), 3.77 (s, 3 H), 3.04 (dd, J = 16.5, 21 Hz, 1 H), 2.73 (d, J = 10.2 Hz, 1 H), 1.94 (m, 1 H), 1.65-1-29 (m, 10 H).

### Example 13

**Compound 66:** Alcohol **28** (2.93 g, 10.9 mmol) and toluenesulfonic acid (1.5 g) were dissolved in acetone (75 mL), and the mixture was stirred at room temperature for 15 hrs. The reaction was quenched with water (30 mL), and basified with concentrated NH₃-H₂O until PH = 9. Acetone was removed under reduced pressure, and the water phase was extracted with CH₂Cl₂ (3x). The combined organic extracts were washed with brine (1x), and dried over Na₂SO₄. Concentration gave the desired product: ¹H NMR (CDCl₃) δ 7.01 (m, 1 H), 4.73 (m, 1 H), 4.42 (m, 1 H), 3.97 (m, 1 H), 3.76 (s, 3 H), 2.71-2.27 (m, 2 H), 2.02 (s, 3 H), 1.98 (s, 3 H).

### Example 14

**Compound 67:** To a solution of alcohol **66** (10.9 mmol) in CH₂Cl₂ (60 mL) at 0°C was added pyridine (4.4 mL, 54.5 mmol), followed by addition of trimethylacetyl chloride (2.7 mL, 21.8 mmol). The mixture was warmed to room temperature and stirred for 14 hrs. The mixture was diluted with CH₂Cl₂, and washed with water (2x), brine (1x), and dried over MgSO₄. Purification by flash column chromatography (Hexane/EtOAc = 9/1) gave the diester (2320 g, 68%): ¹H NMR (CDCl₃) δ 6.72 (m, 1 H), 5.04 (m, 1 H), 4.76 (m, 1 H), 4.40 (m, 1 H), 3.77 (s, 3 H), 2.72-2.49 (m, 2 H), 137 (s, 3 H), 135 (s, 3 H), 1.23 (s, 9 H).

### Example 15

**Compound 68:** Diester **67** (2.32 g, 2.3 mmol) was dissolved in acetone/H₂O (1/1, 100 mL) and heated at 55°C for 16 hrs. Solvents were removed, water (2 x 50 mL) was added and evaporated. Concentration with toluene (2 x 50 mL) gave diol, which was used without further purification: ¹H NMR (CDCl₃) δ 6.83 (m, 1 H), 5.06 (m, 1 H), 4.42 (m, 1 H), 4.09 (m, 1 H), 3.77 (s, 3 H), 2.68-2.41 (m, 2 H), 1.22 (s, 9 H).

### Example 16

**Compound 69:** To a solution of diol **68** (0.410 g, 1.5 mmol) in THF (8 mL) at 0°C was added triethylamine (0.83 mL, 6.0 mmol), followed by slow addition of thionyl chloride (0.33 mL, 4.5 mmol). The mixture was warmed to room temperature and stirred for 3 hrs. The mixture was diluted with CHCl₃, and washed with water (3x), brine (1x), and dried over MgSO₄. Purification by flash column chromatography (Hexanes/EtOAc = 5/1) gave a exo/endo mixture (0.430 g, 90%): ¹H NMR (CDCl₃) δ 6.89-6.85 (m, 1 H), 5.48-4.84 (m, 3 H), 3.80, 3.78 (s, 3 H), 2.90-2.60 (m, 2 H), 1.25, 1.19 (s, 9 H).

### Example 17

**Compound 70:** The mixture of sulfone **69** (0.400 g, 1.3 mmol) and sodium azide (0.410 g, 6.29 mmol) in DMF (10 mL) was stirred for 20 hrs. The reaction mixture was then diluted with ethyl acetate, washed with saturated NH₄Cl solution, water, brine, and dried over MgSO₄. Concentration gave the azide (0.338 g, 90%): ¹H NMR (CDCl₃) δ 6.78 (m, 1 H), 5.32 (m, 1 H), 4.20 (m, 1 H), 3.89 (m, 1 H), 3.78 (s, 3 H), 3.00-2.60 (m, 2 H), 1.21 (s, 9 H).

### Example 18

**Compound 71:** To a solution of alcohol **70** (0.338 g, 1.1 mmol) in CH₂Cl₂ (11 mL) at 0°C was added triethylamine (0.4 mL, 2.9 mmol), followed by slow addition of methylsulfonic chloride (0.18 mL, 2.3 mmol). The mixture was stirred at 0°C for 30 min., and diluted with CH₂Cl₂. The organic layer was washed with water (2x), brine, and dried over MgSO₄. Purification by flash column chromatography (Hexane/EtOAc = 3/1) gave the desired compound (0.380 g, 82%): ¹H NMR (CDCl₃) δ 6.82 (m, 1 H), 5.44 (m, 1 H), 4.76 (dd, J = 7.3, 1.4 Hz, 1 H), 4.48 (m, 1 H), 3.80 (s, 3 H), 3.11 (s, 3 H), 2.82-2.61 (m, 2 H), 1.21 (s, 9 H).

### Example 19

**Compound 72:** The mixture of azide **71** (0.380 g, 0.94 mmol) and triphenylphosphine (0.271 g, 1.04mmol) in THF (19 mL) was stirred for 2 hrs. The reaction was quenched with water (1.9 mL) and triethylamine (0.39 mL, 2.82 mmol), and the mixture was stirred for 14 hrs. Solvents were removed under reduced pressure, and the mixture was used for next step. To a solution of above mixture in CH₂Cl₂ (20 mL) at 0°C was added pyridine (0.68 mL, 8.4 mmol), followed by slow addition of acetyl chloride (0.30 mL, 4.2 mmol). The mixture was stirred at 0°C for 5 min., and diluted with ethyl acetate. The mixture was washed with water (2x), brine (1x), dried over MgSO₄. Purification by flash column chromatography (Hexanes/EtOAc = 3/1) gave the aziridine (0.205 g, 83%): ¹H NMR (CDCl₃) δ 7.19 (m, 1 H), 5.58 (m, 1 H), 3.77 (s, 3 H), 3.14 (m, 2 H), 2.85 (dd, J = 7.0, 1.6 Hz, 1 H), 2.34 (m, 1 H), 2.16 (s, 3 H), 1.14 (s, 9 H).

### Example 20

**Compound 73:** The mixture of aziridine **72** (0.200 g, 0.68 mmol), sodium azide (0.221 g, 3.4 mmol), and ammonium chloride (0.146 g, 2.7 mmol) in DMF (10 mL) was stirred at room temperature for 14 hrs. Then the mixture was diluted with ethyl acetate, and washed with water (5x), brine (1x), and dried over MgSO₄. Purification by flash column chromatography (hexanes/EtOAc = 2/1) gave desired product and deacetyl amine (0.139 g). The mixture was dissolved in acetic anhydride (2 mL), and stirred for 2 hrs. Excess anhydride was removed under reduced pressure, and give the desired product (149 mg): ¹H NMR (CDCl₃) δ 6.76 (m, 1 H), 5.53 (d, J = 8.5 Hz, 1 H), 5.05 (m, 1 H), 4.31 (m, 1 H), 4.08 (m, 1 H), 3.79 (s, 3 H), 2.91 (m, 1 H), 2.51 (m, 1 H), 1.99 (s, 3 H), 1.20 (s, 9 H).

### Example 21

**Compound 74:** A solution of potassium hydroxide in MeOH/H₂O (0.5 M, 4.4 mL, 2.2 mmol) was added to ester **73** (149 mg, 0.44 mmol) and the mixture was stirred at room temperature for 3 hrs. The mixture was cooled to 0°C, and acidified with Amberlite (acidic) to PH = 3-4. The mixture was filtered, and washed with MeOH. Concentration gave the carboxylic acid as a white solid (73 mg, 69%): ¹H NMR (CD₃OD) δ 6.62 (m, 1 H), 4.15 (m, 1 H), 3.95-3.72 (m, 2 H), 2.84 (dd, J = 6.7, 1.4 Hz, 1 H), 2.23 (m, 1 H), 1.99 (s, 3 H).

### Example 22

**Compound 75:** The mixture of azide **74** (8 mg) and Pd-C (Lindlar) (15 mg) in ethanol (2 mL) was stirred under hydrogen for 16 hrs. The mixture was filtered through celite, washed with hot MeOH-H₂O (1/1). Concentration gave a solid. The solid was dissolved in water, and passed through a short C-8 column, and washed with water. Concentration gave a white solid (6 mg): ¹H NMR (D₂O) δ 6.28 (m, 1 H), 4.06-3.85 (m, 3 H), 2.83 (dd, J =17.7, 5.4 Hz, 1 H), 2.35 (m, 1 H), 2.06 (s, 3 H).

### Example 23

**Compound 76:** Carboxylic acid **74** (68 mg, 0.28 mmol) and diphenyldiazomethane (61 mg, 0.31 mmol) were dissolved in ethanol (12 mL), and stirred for 16 hrs. The reaction was quenched with acetic acid (0.5 mL), and the mixture was stirred for 10 min. Solvents were removed under reduced pressure. Purification by flash column chromatography (EtOAc) gave the ester (56 mg, 50%): ¹H NMR (CD₃OD) δ 7.36-7.23 (m, 10 H), 6.88 (s, 1 H), 6.76 (s, 1 H), 4.21 (m, 1 H), 3.93-3.79 (m, 2 H), 2.89 (dd, J = 17.7, 5.0 Hz, 1 H), 2.34 (m, 1 H), 2.00 (s, 3 H).

### Example 24

**Compound 77:** To a solution of alcohol **76** (20 mg, 0.05 mmol) in CH₂Cl₂ (1 mL) was added pyridine (40 µL, 0.5 mmol), followed by addition of acetic anhydride (24 µL, 0.25 mmol). The mixture was stirred for 24 hrs, and solvents and reagents were removed under reduced pressure. Purification by flash column chromatography (Hexane/EtOAc = 1/2) gave the diester (20 mg, 91%): ¹H NMR (CDCl₃) δ 7.40-7.27 (m, 10 H), 6.95 (s, 1 H), 6.87 (m, 1 H), 5.60 (m, 1 H), 5.12 (ddd, J = 16.4, 10.2, 5.9 Hz, 1 H), 4.28 (dd, J = 20.0, 9.4 Hz, 1 H), 4.15 (m, 1 H), 2.93 (dd, J = 17.8, 5.2 Hz, 1 H), 2.57 (m, 1 H), 2.09 (s, 3 H), 2.01 (s, 3 H).

### Example 25

**Compound 78:** The mixture of diester **77** (20 mg, 0.045 mmol), anisole (50 µL, 0.45 mmol), and TFA (1 mL) in CH₂Cl₂ (1 mL) was stirred for 20 min. Solvents and reagents were removed under reduced pressure. Purification by flash column chromatography (EtOAc to EtOAc/AcOH = 100/1) gave the carboxylic acid (6 mg): ¹H NMR (CDCl₃) δ 6.85 (m, 1 H), 5.54 (m, 1 H), 5.12 (m, 1 H), 4.31-4.03 (m, 2 H), 2.89 (m, 1 H), 2.60-2.41 (m, 1 H), 2.11 (s, 3 H), 2.03 (s, 3 H).

### Example 26

**Compound 79:** The mixture of azide **78** (6 mg, 0.02 mmol) and Pd-C (Lindlar) (15 mg) in EtOH/H₂O (2.2 mL, 10/1) was stirred under hydrogen for 3 hrs. The mixture was filtered through a pad of celite, washed with hot MeOH/H₂O (1/1). Evaporation gave a white solid. The solid was dissolved in water, and passed through a C-8 column. Evaporation of water gave a white powder (3 mg): ¹H NMR (D₂O) δ 6.32 (m, 1 H), 5.06 (m, 1 H), 4.06 (t, J = 10.4 Hz, 1 H), 3.84 (m, 1 H), 2.83 (m, 1 H), 2.42 (m, 1 H), 2.06 (s, 3 H), 2.00 (s, 3 H).

### Example 27

**Compound 80:** To a solution of alcohol **76** (35 mg, 0.086 mmol), Boc-glycine (30 mg, 0.172 mmol), and catalytic amount DMAP in CH₂Cl₂ (1 mL) was added DCC (35 mg, 0.172 mmol). The mixture was stirred for 30 min, and filtered and washed with CHCl₃. The CHCl₃ solution was washed with water (2x). Concentration gave a white solid. Purification by flash column chromatography (Hexane/EtOAc = 1/2) gave product (30 mg): ¹H NMR (CDCl₃) δ 7.39-7.26 (m, 10 H), 6.95 (s, 1 H), 6.86 (m, 1 H), 5.77 (m, 1 H), 5.27 (m, 1 H), 4.99 (m, 1 H), 4.18-4.01 (m, 2 H), 3.94-3.84 (m, 2 H), 2.96 (dd, J = 7.8, 5.9 Hz, 1 H), 2.57 (m, 1 H), 2.02 (s, 3 H), 1.45 (s, 9 H).

### Example 28

**Compound 81:** The mixture of diester **80** (30 mg, 0.05 mmol), anisole (150 µL), and TFA (1 mL) in CH₂Cl₂ (1 mL) was stirred for 3 hrs. Solvents and reagents were evaporated . The mixture was dissolved in water, and washed with CHCl₃ (3x). Water phase was evaporated to gave a white solid (15 mg): ¹H NMR (CD₃OD) δ 6.73 (m, 1 H), 5.25-5.15 (m, 1 H), 4.35 (m, 1 H), 4.17 (m, 1 H), 3.82 (m, 2 H), 2.93 (dd, J = 17.7, 5.6 Hz, 1 H), 2.42 (m, 1 H), 1.97 (s, 3 H).

### Example 29

**Compound 82:** The mixture of azide **81** (15 mg, 0.05 mmol) and Pd-C (Lindlar) (30 mg) in EtOH/H₂O (4 mL, 1/1) was stirred under hydrogen for 3 hrs. The mixture was filtered through a pad of celite, and washed with hot MeOH/H₂O (1/1). Concentration gave a glass-like solid. The solid was dissolved in water, and passed through C-8 column. Evaporation of water gave the amino acid: ¹H NMR (D₂O) δ 6.68 (m, 1 H), 5.28 (m, 1 H), 4.29 (m, 1 H), 4.08-3.79 (m, 3 H), 2.85 (m, 1 H), 2.41 (m, 1 H), 2.04 (s, 3 H).

### Example 30

**bis-Boc guanidinyl methyl ester 92:** Treated according to the procedure of Kim and Qian, "Tetrahedron Lett.", 34:7677 (1993). To a solution of amine 91 (42 mg, 0.154 mmol), bis-Boc thiourea (43 mg, 0.155 mmol) and triethylamine (72 µL) in dry DMF (310 µL) cooled to 0°C was added mercury chloride (46 mg, 0.170 mmol) in one portion. After 30 min the reaction was warmed to room temperature and stirred for an additional 2.5 h. The reaction mixture was then filtered through a celite pad, concentrated and purified by flash column chromatography (100% ethyl acetate) to give 70 mg (89%) of 92 as a colorless foam. ¹H NMR (CDCl₃, 300 MHz): δ 11.37 (s, 1H); 8.60 (d, 1H, *J* = 7.8 Hz); 6.83 (t, 1H, *J* = 2.1 Hz); 6.63 (d, 1H, *J* = 8.4 Hz); 4.76 (d, 1H, *J* = 7.0 Hz); 4.71 (d, 1H, *J* = 7.0 Hz); 4.45-4.10 (complex m, 2H); 3.76 (s, 3H); 339 (s, 3H); 2.84 (dd, 1H, *J* = 5.4,17.4 Hz); 2.45-2.30 (m, 1H); 1.92 (s, 3H); 1.49 (s, 18H).

### Example 31

**bis-Boc guanidinyl carboxylic acid 93:** To a solution of ester **92** (70 mg, 0.136 mmol) in THE (3 mL) cooled to 0°C was added aq. KOH (350 µL of a 0.476 M solution). The reaction was then warmed to room temperature and stirred for 2 h. The reaction was then acidified to pH = 4.5 with Amberlite IR-120 (plus) acidic resin. The resin was then filtered and washed with ethanol and H₂O. Concentration *in vacuo* gave 66 mg (97%) of carboxylic acid **93** as a white solid. ¹H NMR (CDCl₃, 300 MHz): δ 11.40 (br s,1H); 8.67 (d, 1H, *J* = 7.8 Hz); 6.89 (s, 1H); 6.69 (br d, 1H, *J* = 8.4 Hz); 4.77 (d, 1H, *J* = 7.2 Hz); 4.70 (d, 1H, *J* = 7.2 Hz); 4.40-4.15 (m, 2H); 3.39 (s, 3H); 2.84 (dd, 1H, *J* = 4.8, 17.1 Hz); 2.45-2.30 (m, 1H); 1.95 (s, 3H); 1.49 (s, 9H); 1.48 (s, 9H).

### Example 32

**Guanidine carboxylic acid TFA salt 94:** To a solution of bis-Boc guanidinyl carboxylic acid **93** (23 mg, 0.046 mmol) in CH₂Cl₂ (1 mL) cooled to 0°C was added neat trifluoroacetic acid (500 µL). After 30 min the reaction was warmed to room temperature and stirred for an additional 1.25 h. Volatiles were removed under vacuum and the residue co-evaporated with several portions of H₂O to give a pale orange solid. The residue was purified by reverse phase C₁₈ chromatography using H₂O as an eluent. Fractions containing the desired product were pooled and lyophilized to give 15 mg of 93 as a white powder. ¹H NMR (D₂O, 500 MHz): δ 6.82 (t, 1H, *J* = 2.0 Hz); 451-4.47 (m, 1H); 3.93 (dd, 1H, *J* = 9.0, 11.2 Hz); 3.87-3.80 (apparent ddd, 1H); 2.88 (m, 1H); 2.48-2.45 (complex m); 2.07 (s, 3H). ¹³C NMR (D₂O): δ 176.1; 170.0; 157.1; 139.2; 129.5; 69.4; 56.2; 50.9; 30.3; 22.2.

### Example 33

**Synthesis of 102:** A solution of azido allyl ether **6** (24 mg, 0.082 mmol) in ethanol (1 mL) was treated with hydrogen gas (1 atm) over Lindlar's catalyst (30 mg) for 1.5 h. The reaction mixture was filtered through a celite pad and washed with hot ethanol. Concentration *in vacuo* gave a pale solid which was dissolved in THF (1.5 mL) and treated with aqueous KOH (246 µL of a 0.50 M solution). After stirring at ambient temperature for 2 h the reaction was acidified to pH = 4.0 with Amberlite IR-120 (plus) acidic resin, filtered and washed with ethanol and H₂O. Concentration *in vacuo* gave an orange solid which was purified by a C₁₈ column chromatography eluting with H₂O. Fractions containing the product were pooled and lyophilized to give a 2 to 1 mixture of **102** and the fully saturated compound **103** as a white powder. ¹H NMR data for compound **102**: ¹H NMR (D₂O, 500 MHz): δ: 7.85 (s, 1H); 4.29 (br d, 1H, *J* = 9.2 Hz); 4.16 (dd, 1H, *J* = 11.6, 11.6 Hz); 3.78 - 3.72 (m, 2H); 3.62 (apparent ddd, 1H); 2.95 (apparent dd, 1H); 2.58 - 2.52 (m, 1H); 2.11 (s, 3H); 1.58 (q, 2H, *J* = 7.3 Hz); 0.91 (t, 3H, *J* = 7.3 Hz).

### Example 34

**Synthesis of 115:** A solution of amino acid **114** (10.7 mg, 0.038 mmol) in water (1.3 mL) cooled to 0°C was adjusted to pH = 9.0 with 1.0 M NaOH. Benzyl formimidate hydrochloride (26 mg, 0.153 mmol) was then added in one portion and the reaction stirred between 0 - 5°C for 3 h while maintaining the pH between 8.5 - 9.0 with 1.0 M NaOH. The reaction was then concentrated *in vacuo* and the residue applied to a C₁₈ column and eluted with water. Fractions containing the product were pooled and lyophilized to give the formamidine carboxylic acid **115** (10 mg) as a white powder. ¹H NMR (D₂O, 300 MHz, mixture isomers): δ 7.83 (s, 1H); [6.46(s) & 6.43 (s); 1 H total]; 4.83 (d, 1H, *J* = 7.3 Hz); 4.73 (d, 1H, *J* = 7.3 Hz); 4.50 - 4.35 (m, 1H); 4.10-4.05 (m, 1H); [4.03 - 3.95 (m) & 3.80 - 3.65 (m), 1 H total]; 3.39 (s, 3H); 2.90 - 2.75 (m, 1H); 2.55 - 2.30 (m, 1H); [2.03 (s) & 2.01 (s), 3H total].

### Example 35

**Compound 123:** To a solution of alcohol **63** (5.842 g, 205 mmol) and DMAP (200 mg) in pyridine (40 mL) was added tosyl chloride (43 g, 22.6 mmol). The mixture was stirred at room temperature for 40 hrs, and pyridine was removed under reduced pressure. The reaction was quenched with water, and extracted with EtOAc (3x). The combined organic extracts were washed with water, brine, and dried over MgSO₄. Purification by flash column chromatography (Hexanes/EtOAc = 2/1) gave the tosylate (8.04 g, 89%): ¹H NMR (CDCl₃) δ 7.84 (d, J = 8.3 Hz, 2H), 7.33 (d, J = 8.1 Hz, 2 H), 4.78 (m, 1 H), 4.43 (m, 1 H), 4.06 (m, 1 H), 3.79 (s, 3 H), 2.44 (s, 3 H), 2.43-1.92 (m, 4 H), 1.61-1.22 (m, 10 H).

### Example 36

**Compound 124:** To a solution of alcohol **123** (440 mg, 1.0 mmol) in pyridine (3 mL) was added POCl₃ (100 µL, 1.1 mmol). The mixture was stirred at room temperature for 12 hrs, and quenched with saturated NH₄Cl solution. The water phase was extracted with ether (3x). The combined ether layers were washed with water (2x), 2 N HCl solution (2x), brine, and dried over MgSO₄. Purification by flash column chromatography (Hexane/EtOAc = 2/1) gave a mixture of the desired product **124** and some inpurity (350 mg, 83%, 2/1).

### Example 37

**Compound 1:** To a solution of the known acetonide of methyl shikimate (877 mg, 3.85 mmol, "Tetrahedron Lett.", 26:21 (1985)) in dichloromethane (15 mL) at -10°C was added methanesulfonyl chloride (330 µL, 4.23 mmol) followed by the dropwise addition of triethylamine (640 µL, 4.62 mmol). The solution was stirred at -10°C for 1 h then at 0°C for 2 h, at which time methanesulfonyl chloride (30 µL), triethylamine (64 µL) was added. After 1 h cold water was added, the organic phase was separated, washed with water, dried (MgSO₄), and evaporated. The crude product was chromatographed on silica gel (1/1-hexane/ethyl acetate) to provide mesylate **130** (1.1 g, 93%) as an oil. Mesylate **130** (990 mg, 3.2 mmol) was dissolved in tetrahydrofuran (5 mL) and was treated with 1M HCl (5 mL). The solution was stirred at room temperature for 19 h, diluted with water (5 mL) and stirred an additional 7 h. Evaporation of the organic solvent precipitated an oily residue which was extracted into ethyl acetate. The combined organic extracts were washed with brine, dried (MgSO₄), and evaporated. Addition of CH₂Cl₂ to the crude residue precipitated a white solid which was filtered and washed with CH₂Cl₂ to afford diol **131** (323 mg, 38%). To a partial suspension of diol **131** (260 mg, 0.98 mmol) in THF (5 mL) at 0°C was added DBU (154 µL, 1.03 mmol). The solution was stirred at 0°C for 3 h and then was warmed to room temperature stirring for 5 h. The solvent was evaporated and the crude residue was partitioned between ethyl acetate (40 mL) and 5% citric acid (20 mL). The organic phase was washed with brine. Aqueous phases were back extracted with ethyl acetate (15 mL) and the combined organic extracts were dried (MgSO₄) and evaporated to afford the epoxide (117 mg, 70%) as a white solid which gave an ¹H NMR spectrum consistent with structure **1** prepared by literature method.

### Example 38

**Alcohol 51:** To a solution of protected alcohol (PG=methoxymethyl) (342 mg, 1.15 mmol) in CH₂Cl₂ (10 mL) at 0°C was added trifluoroacetic acid (8 mL). After 5 min at 0°C, the solution was stirred 1 h at room temperature and was evaporated. The crude product was purified on silica gel (ethyl acetate) to afford alcohol **51** (237 mg, 82%) as an oil: ¹H NMR (300 MHz, CDCl₃) δ 2.11 (s, 3H), 2.45 (m, 1H), 2.97 (dd, 1H, *J* = 3.8,18.8), 3.66 (m, 2H), 3.78 (s, 3H), 4.40 (br s, 1H), 5.22 (br s, 1H), 6.19 (br s, 1H), 6.82 (m, 1H).

### Example 39

**Methyl ether 150:** To a solution of alcohol **51** (46 mg, 0.18 mmol) and methyl iodide (56 µL, 0.90 mmol) in THF (0.7 mL) at 0°C was added NaH as a 60% mineral oil dispersion (8 mg, 020 mmol). The solution was stirred at 0°C for 2.5 h, and a second portion of NaH (2 mg) was added. After an additional 1 h at 0°C and 4 h at room temperature the solution was cooled to 0°C and 5% citric acid (0.5 mL) was added. The mixture was extracted with ethyl acetate (4X2mL) and the combined organic extracts were dried (MgSO₄), and evaporated. Purification of the crude residue on silica gel (ethyl acetate) gave methyl ether **150** (12 mg, 25%) as a solid: ¹H NMR (300 MHz, CDCl₃) δ 2.07 (s, 3H), 2.23-2.34 (m, 1H), 2.89 (app ddd, 1H), 3.43 (s, 3H), 358 (m, 1H), 3.78 (s, 3H), 4.13 (m, 1H), 4.40 (m, 1H), 5.73 (d, 1H, *J* = 7.6), 6.89 (m,1H).

### Example 40

**Amino acid 151:** To a solution of methyl ether **150** (12 mg, 0.45 mmol) in THF(1 mL)/water (100 µL) was added polymer support Ph₃P (75 mg, 3 mmol P/g resin). The mixture was stirred at room temperature for 19 h. The resin was filtered, washed several times with THF and the combined filtrate and washings were evaporated to provide 8 mg of a crude residue. The residue was dissolved in THF (0.5 mL), and 0.5 M KOH (132 µL)/water (250 µL) was added. The solution was stirred at room temperature for 1.25 h and the pH was adjusted to 3-4 with IR120 ion exchange resin. The resin was filtered and was stirred with 1M HCl. After filtration, the resin was subjected to the same treatment with 1M HCl until the acidic washes no longer tested positive for amine with ninhydrin. The combined resin washings were evaporated and the residue was purified on C-18 reverse phase silica eluting with water to afford after lyophilization, amino acid 151 (1.8 mg, 15%) as a white solid: ¹H NMR (300 MHz, D₂O) δ 2.09 (s, 3H), 2.48-2.59 (app qt, 1H), 2.94 (dd, 1H, *J* = 5.7,17.4), 3.61 (m, 1H), 4.14-4.26 (m, 2H), 6.86 (br s, 1H).

### Example 41

**Amino acid allyl ether 153:** To a solution of azide **6** (16 mg, 0.054 mmol) in THF (0.50 mL) and H₂O (35 µL) was added polystyrene supported PPh₃ (50 mg). The reaction was stirred at ambient temperature for 24 h, filtered through a sintered glass funnel and washed with hot methanol. Concentration *in vacuo* gave the crude amino ester which was dissolved in THF (1.0 mL) and treated with aqueous KOH (220 µL of a 0.5 M solution). After stirring at ambient temperature for 2 h Amberlite IR-120 (plus) acidic resin was added until the solution attained pH = 45 . The resin was filtered and washed with ethanol and H₂O. Concentration *in vacuo* gave a pale orange solid which was purified by reverse phase C₁₈ chromatography using H₂O as an eluent. Fractions containing the desired product were pooled and lyophilized to give the amino acid as a white powder. ¹H NMR (D₂O, 300 MHz): δ 6.51 (br t, 1H); 6.05-5.80 (m, 1H, -CH=, allyl); 5.36-5.24 (m, 2H, =CH₂, allyl); 4.35-4.25 (m, 1H); 4.25 - 4.05 (m, 2H, -CH₂-, allyl); 4.02-3.95 (m, 1H); 3.81-3.70 (m, 1H); 2.86-2.77 (apparent dd, 1H); 2.35-2.24 (complex m, 1H); 2.09 (s, 3H).

### Example 42

**Epoxide 161:** MCPBA (690 mg) was added to a solution of olefin **160** (532 mg, 1.61 mmol, prepared by Example 14, crude mesylate was filtered through silica gel using 30% EtOAc/Hexanes prior to use) in dichloromethane (15 mL) cooled to 0°C. The mixture was warmed to room temperature and stirred overnight. The bulk of the solvent was removed under vacuum and the mixture diluted with ethyl acetate. The organic layer was washed with aqueous sodium bisulfite, saturated sodium bicarbonate, brine and dried over MgSO₄. Concentration *in vacuo* followed by flash column chromatography of the residue (30% hexanes in ethyl acetate) gave 437 mg (78%) of **161** as a pale oil. ¹H NMR (CDCl₃, 300 MHz): [1:1 mixture of diastereomers] δ [4.75 (dd, *J* = 3.9, 8.2 Hz) & 4.71 (dd, *J* = 3.9, 8.4 Hz), 1H total]; 4.37 (m, 1H); 4.25-4.00 (m, 2H); 3.78 (s, 3H); [3.68 (dd, *J* = 5.7, 11.7 Hz) & 3.51 (dd, *J* = 6.6, 11.7 Hz), 1H total]; [3.17 (s) & 3.16 (s), 3H total]; [2.99 (m) & 2.93 (m), 1H total]; [2.83 (t, *J* = 4.1 Hz) & 2.82 (t, *J* = 4.5 Hz), 1H total]; 2.70-2.60 (m, 1H); 2.45-2.30 (m, 1H).

### Example 43

**Diol 162:** The epoxide **161** (437 mg, 1.23 mmol) was gently reluxed for 1 h in THF (20 mL) and H₂O (10 mL) containing 5 drops of 70% HClO₄. Solid NaHCO₃ was added and the mixture concentrated *in vacuo*. The residue was dissolved in EtOAc, washed with brine and dried. Concentration *in vacuo* gave the crude diol **162** as a pale oil in quantitative yield. Used without any purification for the next reaction.

### Example 44

**Aldehyde 163:** Oxidation of diol **162** was carried out according to the procedure of Vo-Quang and co-workers, "Synthesis", 68 (1988). To a slurry of silica gel (4.3 g) in dichloromethane (30 mL) was added a solution of NaIO₄ (4.4 mL of a 0.65 M aqueous solution). To this slurry was added a solution of the crude diol **162** (520 mg) in EtOAc (5 mL) and dichloromethane (15 mL). After 1 h the solids were filtered and washed with 20% hexanes/EtOAc. Concentration gave an oily residue which was dissolved in EtOAc and dried over MgSO₄. Concentration *in vacuo* gave the aldehyde **163** as a pale oil which was used immediately for the next reaction. ¹H NMR (CDCl₃, 300 MHz): δ 9.69 (s, 1H); 6.98 (m, 1H); 4.72 (dd, 1H, *J* = 3.7, 9.1 Hz); 4.53 (d, 1H, *J* = 18.3 Hz); 4.45 (d, 1H, *J* = 18.3 Hz); 4.31 (m, 1H); 4.26-4.18 (m, 1H); 3.79 (s, 3H); 3.19 (s, 3H); 3.05 (dd, 1H, *J* = 5.7, 18.6 Hz); 2.20-2.45 (m, 1H).

### Example 45

**Alcohol 164:** The crude aldehyde **163** was treated with NaCNBH₃ according to the procedure of Borch and co-workers, "J. Amer. Chem. Soc.", 93:2897 (1971) to give **269** mg (65%) of the alcohol **164** after flash chromatography (40% hexanes in ethyl acetate). ¹H NMR (CDCl₃, 300 MHz): δ 6.91 (m, 1H); 4.75 (dd, 1H, *J* = 3.9, 8.7 Hz); 4.34 (br t, 1H, *J* = 4.1 Hz); 4.25-4.15 (m, 1H); 3.85-3.70 (m, 4H); 3.77 (s, 3H); 3.16 (s, 3H); 2.95 (dd, 1H, *J* = 5.7, 18.6 Hz); 2.37 (dd, 1H, *J* = 7.1, 18.6 Hz); 2.26 (br s, 1H).

### Example 46

**Aziridine 165:** The alcohol **164** (208 mg, 0.62 mmol) was acetylated in the usual manner (AcCl, pyridine, dichloromethane, cat. DMAP) to give the acetate (241 mg, 100%). The crude acetate (202 mg, 0.54mmol) was treated at room temperature with Ph₃P (155 mg) in THF (12 mL) for 2 h. H₂O (1.1 mL) and triethylamine (224 µL) were then added and the solution stirred overnight. The reaction mixture was concentrated and the residue partitioned between ethyl acetate and saturated bicarbonate/brine. The organic layer was dried, concentrated *in vacuo* and purified by flash chromatography (10% MeOH in EtOAc) to give 125 mg (90%) of aziridine **165** as a white solid. ¹H NMR (CDCl₃, 300 MHz): δ 6.80 (m, 1H); 4.44 (br s, 1H); 4.23 (t, 2H, *J* = 4.8 Hz); 3.82-3.65 (m, 2H); 3.74 (s, 3H); 2.85 (br d, 1H, *J* = 19.2 Hz); 2.65-2.40 (m, 3H); 2.09 (s, 3H); 1.25 (br s, 1H).

### Example 47

**N-Boc aziridine 166:** Boc anhydride (113 mg, 0.52 mmol) was added to a solution of aziridine **165** (125 mg, 0.49 mmol), triethylamine (70 µL), DMAP (cat. amount) in dichloromethane (7 mL). After 1 h the reaction was concentrated and the residue subjected to flash chromatography (40% EtOAc in hexanes) to give 154 mg (88%) of the N Boc aziridine **166** as a pale oil. ¹H NMR (CDCl₃, 300 MHz): δ 6.82 (m, 1H); 4.47 (br m, 1H); 4.23 (t, 2H, *J* = 4.7 Hz); 3.81 (t, 2H, *J* = 4.7 Hz); 3.75 (s, 3H); 3.00 (br d, 1H, *J* =18.0 Hz); 2.90-2.85 (m, 2H); 2.65-2.55 (m, 1H); 2.10 (s, 3H); 1.44 (s, 9H).

### Example 48

**Azido ester 167:** Aziridine **166** (154 mg, 0.43 mmol), sodium azide (216 mg), and ammonium chloride (223 mg) was heated at 100°C in DMF (5 mL) for 18 h. The cooled reaction mixture was partitioned between ethyl ether and brine. The ether layer was washed with H₂O, brine and dried over MgSO₄. Concentration gave a crude residue which was treated with 40% TFA in dichloromethane at room temperature. After 2 h the reaction was concentrated *in vacuo* to give a pale oil which was passed through a short column of silica gel eluting with EtOAc. The product was then acylated in the usual manner (AcCl, pyridine, dichloromethane, cat. DMAP) to give the azido ester **167** as a pale yellow oil 16 mg (11% for 3 steps) after flash chromatography (5% MeOH in chloroform). ¹H NMR (CDCl₃, 300 MHz): δ 6.85 (m, 1H); 5.80 (br d, 1H, *J* = 7.8 Hz); 4.55 (m, 1H); 4.25-4.10 (m, 3H); 3.90-3.85 (m, 2H); 3.78 (s, 3H); 3.55 (m, 1H); 2.90 (dd, 1H, *J* = 5.4,17.0 Hz); 2.45-2.25 (m, 1H); 2.10 (s, 3H); 2.05 (s, 3H).

### Example 49

**Amino acid 168:** To a solution of ester **167** (16 mg, 0.047 mmol) in THF (1 ml) cooled to 0 °C was added aq. KOH (208 µl of a 0.476 M solution). The reaction was then warmed to room temperature and stirred for 2 h. The reaction was then acidified to pH = 4.0 with Amberlite IR-120 (plus) acidic resin. The resin was then filtered and washed with ethanol and H₂O. Concentration *in vacuo* gave a 14 mg (100%) of the azido carboxylic acid as a white solid. The azido acid was dissolved in ethanol (2 mL) and treated with hydrogen gas (1 atm) over Lindlar's catalyst (15 mg) for 16 h according to the procedure of Corey and co-workers, "Synthesis", 590 (1975). The reaction mixture was filtered through a celite pad and washed with hot ethanol and H₂O. Concentration *in vacuo* gave a pale orange solid which was purified by a C₁₈ column chromatography eluting with H₂O. The fractions containing the product were pooled and lyophilzed to give 9.8 mg of **168** as a white powder. ¹H NMR (D₂O, 500 MHz): δ: 6.53 (br s, 1H); 4.28 (br m, 1H); 4.08 (dd, 1H, *J* = 11.0, 11.0 Hz); 3.80-3.65 (complex m, 4H); 3.44 (m, 1H); 2.84 (apparent dd, 1H); 2.46-2.39 (complex m, 1H); 2.08 (s, 3H).

### Example 50

**Epoxy MOM ether 19 (PG=methoxymethyl):** Prepared in 74% from epoxy alcohol **1** according to the procedure of Mordini and co-workers, "J. Org. Chem.", 59:4784 (1994). ¹H NMR (CDCl₃, 300 MHz): δ 6.73 (m, 1H); 4.87 (s, 2H); 4.59 (t, 1H, *J* = 2.4 Hz); 3.76 (s, 3H); 3.57 (m, 1H); 3.50-3.40 (m, 1H); 3.48 (s, 3H); 3.10(d, *J* = 19.5 Hz); 2.45 (m, 1H).

### Example 51

**Aziridine 170:** Prepared in 77% overall from epoxide **19 (PG=methoxymethyl)** according to the general protocol described in Examples 3 and 4: ¹H NMR (COCl₃, 300 MHz): δ 6.85 (m, 1H); 4.78 (s, 2H); 4.54 (m, 1H); 3.73 (s, 3H); 3.41 (s, 3H); 2.87 (d, 1H, *J* = 18.9 Hz); 2.70-2.45 (m, 3H).

### Example 52

**Azido ester 22 (PG=methoxymethyl):** The aziridine **170** (329 mg, 1.54 mmol), NaN₃ (446 mg) and NH₄Cl (151 mg) was heated at 65°C in DMF (20 mL) for 18 h. The cooled reaction mixture was partitioned between ethyl ether and brine. The ether layer was washed with H₂O, brine and dried over MgSO₄. Concentration *in vacuo* gave the crude azido amine as a pale oil which was taken up in CH₂Cl₂ (15 mL) and treated with pyridine (4 mL) and AcCl (150 µL). Aqueous work up followed by flash chromatography of the residue gave 350 mg (76%) of azido ester **22 (PG=methoxymethyl)** as a pale oil. ¹H NMR (CDCl₃, 300 MHz): δ 6.78 (s, 1H); 6.39 (br d, 1H, *J* = 7.8 Hz); 4.72 (d, 1H, *J* = 6.9 Hz); 4.66 (d, 1H, *J* = 6.9 Hz); 4.53 (br d, 1H, *J* = 8.4 Hz); 4.00-3.90 (m, 1H); 3.80-3.65 (m, 1H); 3.75 (s, 3H); 3.37 (s, 3H); 2.85 (dd, 1H, *J* = 5.4,17.7 Hz); 2.35-2.20 (m, 1H); 2.04 (s, 3H).

### Example 53

**Amino acid 114:** The azide **22** (PG=methoxymethyl) (39 mg, 0.131 mmol) was treated with hydrogen gas at 1 atmosphere over Lindlar's catalyst (39 mg) in ethanol for 2.5 h according to the procedure of Corey and co-workers, "Synthesis", 590 (1975). The reaction mixture was filtered through a celite pad, washed with hot ethanol and concentrated to give the crude amine 33 mg (92%) as a pale foam. The amine in THF (1 mL) was treated with aq. KOH (380 µL of a 0.476 M solution). After 1 h the reaction was acidified to pH = 4.0 with Amberlite IR-120 (plus) acidic resin. The resin was then filtered, washed with H₂O and concentrated to give a pale solid which was purified by a C₁₈ column chromatography eluting with H₂O. The fractions containing the product were pooled and lyophilzed to give 20 mg of 114 as a white powder. ¹H NMR (D₂O, 300 MHz): δ 6.65 (s, 1H); 4.87 (d, 1H, *J* = 7.5 Hz); 4.76 (d, 1H, *J* = 7.5 Hz); 4.47 (br d, 1H, *J* = 8.7 Hz); 4.16 (dd, 1H, *J* = 11.4, 11.4 Hz); 3.70-3.55 (m, 1H); 3.43 (s, 3H); 2.95 (dd, 1H, *J* = 5.7, 17.4 Hz); 2.60-2.45 (m, 1H); 2.11 (s, 3H).

### Example 54

**Amino acid 171:** To solid amino acid **114** (4 mg, 0.015 mmol) was added 40% TFA in CH₂Cl₂ (1 mL, cooled to 0°C prior to addition). After stirring at room temperature for 1.5 h the reaction mixture was concentrated to give a white foam. Co-evaporation from H₂O several times followed by lyophilization gave a white solid, 5.5 mg of **117** as the TFA salt. ¹H NMR (D₂O, 300 MHz): δ 6.85 (m, 1H); 4.45 (m, 1H); 4.05 (dd, 1H, *J* = 11.4, 11.4 Hz); 3.65-3.55 (m, 1H); 3.00-2.90 (m, 1H); 2.60-2.45 (m, 1H); 2.09 (s, 3H).

### Example 55

**Acetonide 180:** To a suspension of shikimic acid (25 g, **144** mmol, Aldrich) in methanol (300 mL) was added *p*-toluenesulfonic acid (274 mg, 1.44 mmol, 1 mol%) and the mixture was heated to reflux for 2h. After adding more *p*-toluenesulfonic acid (1 mol%) the reaction was refluxed for 26h and was evaporated. The crude methyl ester (28.17 g) was suspended in acetone (300 mL) and was treated with dimethoxypropane (35 mL, 288 mmol) and was stirred at room temperature for 6h and then was evaporated. The crude product was dissolved in ethyl acetate (400 mL) and was washed with saturated NaHCO₃ (3X125 mL) and saturated NaCl. The organic phase was dried (MgSO₄), filtered, and evaporated to afford crude acetonide **180** (∼29.4 g) which was used directly: ¹H NMR (CDCl₃) δ 6.91 (t, 1H, *J* = 1.1), 4.74 (t, 1H, *J* = 4.8), 4.11 (t, 1H, *J* = 6.9), 3.90 (m, 1H), 2.79 (dd, 1H, *J* = 4.5, 17.4), 2.25 (m, 2H), 1.44 (s, 3H), 1.40 (s, 3H).

### Example 56

**Mesylate 130:** To a solution of acetonide **180** (29.4 g, 141 mmol) in CH₂Cl₂, (250 mL) at 0°C was added triethylamine (29.5 mL, 212 mmol) followed by the addition of methanesulfonyl chloride (13.6 mL, 176 mmol) over a period of 10 min. The reaction was stirred at 0°C for 1 h and ice cold water (250 mL) was added. After transfer to a separatory funnel, the organic phase was washed with water, 5% citric acid (300 mL), saturated NaHCO₃ (300 mL) and was dried (MgSO₄), filtered, and evaporated. The crude product was filtered through a short plug of silica gel on a fritted glass funnel eluting with ethyl acetate. The filtrate was evaporated to afford mesylate **130** (39.5 g, 91%) as a viscous oil which was used directly in the next step: ¹H NMR (CDCl₃) δ 6.96 (m, 1H), 4.80 (m, 2H), 4.28 (dd, 1H, *J* = 6.6, 7.5), 3.79 (s, 3H), 3.12 (s, 3H), 3.01 (dd, 1H, *J* = 5,17.7), 2.56-2.46 (m, 1H).

### Example 57

**Diol 131:** To a solution of mesylate **130** (35.85 g, 117 mmol) in methanol (500 mL) was added *p*-toluenesulfonic acid (1.11 g, 5.85 mmol, 5 mol%) and the solution was refluxed for 1.5 h and was evaporated. The residue was redissolved in methanol (500 mL) and was refluxed an additional 4 h. The solvent was evaporated and the crude oil was triturated with diethyl ether (250 mL). After completing the crystallization overnight at 0°C, the solid was filtered and was washed with cold diethyl ether, and dried to afford diol 131 (24.76 g) as a white solid. Evaporation of the filtrate and crystallization of the residue from methanol/diethyl ether gave an additional 1.55 g. Obtained 26.3 g (85%) of diol 131: ¹H NMR (CD₃OD) δ 6.83 (m, 1H), 4.86 (m, 1H), 437 (t, 1H, *J* = 4.2), 3.87 (dd, 1H, *J* = 4.2, 8.4), 3.75 (s, 3H), 3.13 (s, 3H), 2.98-2.90 (m, 1H), 2.53-2.43 (m,1H).

### Example 58

**Epoxy alcohol 1:** A suspension of diol **131** (20.78g, 78 mmol) in tetrahydrofuran (400 mL) at 0°C was treated with 1, 8-diazabicyclo[5.4.0]undec-7-ene (11.7 mL, 78 mmol) and was stirred at room temperature for 9 h at which time the reaction was complete. The reaction was evaporated and the crude residue was dissolved in CH₂Cl₂ (200 mL) and was washed with saturated NaCl (300 mL). The aqueous phase was extracted with CH₂Cl₂ (2X200 mL). The combined organic extracts were dried (MgSO₄), filtered, and evaporated. The crude product was purified on silica gel (ethyl acetate) to afford epoxy alcohol **1** (12 g, 90%) as a white solid whose ¹H NMR spectrum was consistent with that reported in the literature: McGowan, D. A.; Berchtold, G. A., "J. Org. Chem.", 46:2381 (1981).

### Example 59

**Methoxymethyl ether 22 (PG=methoxymethyl):** To a solution of epoxy alcohol **1** (4 g, 23.5 mmol) in CH₂Cl₂ (100 mL) was added N, N'-diisopropylethylamine (12.3 mL, 70.5 mmol) followed by chloromethyl methyl ether (3.6 mL, 47 mmol, distilled from tech. grade). The solution was refluxed for 3.5 h and the solvent was evaporated. The residue was partitioned between ethyl acetate (200 mL) and water (200 mL). The aqueous phase was extracted with ethyl acetate (100 mL). The combined organic extracts were washed with saturated NaCl (100 mL), dried (MgSO₄), filtered, and evaporated to afford 4.9 g of a solid residue which was of suitable purity to use directly in the next step: mp 62-65°(crude); mp 64-66°C (diethyl ether/hexane); ¹H NMR (CDCl₃) δ 6.73 (m, 1H), 4.87 (s, 2H), 4.59 (m,1H), 3.75 (s, 3H), 3.57 (m, 1H), 3.48 (m overlapping s, 4H), 3.07 (dd, 1H, *J* = 1.2, 19.8), 2.47 (dq, 1H, *J* = 27,19.5).

### Ethyl Ester Analog of Compound 22:

To a solution of the corresponding ethyl ester of compound **1** ( 120g, 0.065 mol) in CH₂Cl₂ (277 mL) at room temperature was added diisopropylethyl amine (34.0 mL, 0.13 mol) followed by chloromethyl methyl ether (10.0 mL, 0.19 mol). The reaction mixture was then gently refluxed for 2 h, cooled, concentrated *in vacuo,* and partitioned between EtOAc and water. The organic layer was separated and washed successively with dil. HCl, saturated bicarb, brine and dried over MgSO₄. Concentration *in vacuo* followed by flash chromatography on silica gel (50% hexanes in EtOAc) gave 13.3 g (90%) of the corresponding ethyl ester of compound **22** as a colorless liquid. ¹H NMR(300 MHz, CDCl₃) δ 6.73-6.71 (m, 1H); 4.87 (s, 2H); 4.61-4.57 (m, 1H); 4.21 (q, 2H, *J* = 7.2 Hz); 3.60-3.55 (m, 1H); 3.50-3.45 (m, 1H); 3.48 (s, 3H); 3.12-3.05 (m, 1H); 2.52-2.42 (m, 1H); 1.29 (t, 3H, *J* = 7.2 Hz).

### Example 60

**Alcohol 181:** To a solution of methoxymethyl ether **22 (PG=methoxymethyl)** (4.9 g, 22.9 mmol) in 8/1-MeOH/H₂O (175 mL, v/v) was added sodium azide (7.44 g, 114.5 mmol) and ammonium chloride (2.69 g, 50.4 mmol) and the mixture was refluxed for 15 h. The reaction was diluted with water (75 mL) to dissolve precipitated salts and the solution was concentrated to remove methanol. The resulting aqueous phase containing a precipitated oily residue was diluted to a volume of 200 mL with water and was extracted with ethyl acetate (3X100 mL). The combined organic extracts were washed with saturated NaCl (100 mL), dried (MgSO₄), filtered and evaporated. The crude was purified on silica gel (1/1-hexane/ethyl acetate) to afford alcohol **181** (5.09 g, 86%) as a pale yellow oil. Subsequent preparations of alcohol **181** provided material which was of sufficient purity to use in the next step without further purification: ¹H NMR (CDCl₃) δ 6.86 (m, 1H), 4.79 (s, 2H), 4.31 (br t, 1H, *J* = 4.2), 3.90-3.75, 3.77 (m overlapping s, 5H), 3.43 (s, 3H), 2.92 (d, 1H, *J* = 6.6), 2.87 (dd, 1H, *J* = 5.4,18.6), 2.21-2.30 (m, 1H).

### Example 61

**Mesylate 184:** To a solution of alcohol **181** (6.47 g, 252 mmol) in CH₂Cl₂ (100 mL) at 0°C was added first triethyl amine (4.4 mL, 31.5 mmol) then methanesulfonyl chloride (2.14 mL, 27.7 mmol). The reaction was stirred at 0°C for 45 min then was warmed to room temperature stirring for 15 min. The reaction was evaporated and the residue was partitioned between ethyl acetate (200 mL) and water (100 mL). The organic phase was washed with water (100 mL), saturated NaHCO₃ (100 mL), saturated NaCl (100 mL). The water washes were extracted with a single portion of ethyl acetate which was washed with the same NaHCO₃/NaCl solutions. The combined organic extracts were dried (MgSO₄), filtered, and evaporated. The crude product was of suitable purity to be used directly in the next step: ¹H NMR (CDCl₃) δ 6.85 (m, 1H), 4.82 (d, 1H, *J* = 6,9), 4.73 (d, 1H, *J* = 6.9), 4.67 (dd, 1H, *J* = 3.9, 9.0), 4.53 (br t, 1H, *J* = 42), 3.78 (s, 3H), 3.41 (s, 3H), 3.15 (s, 3H), 2.98 (dd, 1H, *J* = 6.0, 18.6), 237 (m, 1H); ¹³C NMR (CDCl₃) δ 165.6, 134.3, 129.6, 96.5, 78.4, 69.6, 55.8, 55.7, 52.1, 38.2, 29.1.

### Example 62

**Aziridine 170:** To a solution of mesylate **184** (8.56 g, 25 mmol) in THF (150 mL) at 0°C was added Ph₃P (8.2 g, 31 mmol), initially adding a third of the amount while cooling and then after removing the ice bath adding the remainder of the Ph₃P over a period of 10-15 min. After complete addition of the Ph₃P the reaction was stirred at room temperature for 3 h with the formation of a white precipitate. To this suspension was added triethyl amine (5.2 mL, 37.5 mmol) and water (10 mL) and the mixture was stirred at room temperature for 12 h. The reaction was concentrated to remove THF and the residue was partitioned between CH₂Cl₂ (200 mL) and saturated NaCl (200 mL). The aqueous phase was extracted with several portions of CH₂Cl₂ and the combined organic extracts were dried (Na₂SO₄), filtered, and evaporated to afford a crude product which was purified on silica gel (10% MeOH/EtOAc) to afford aziridine **170** (4.18 g, 78%) as an oil which typically contained trace amounts of triphenylphosphine oxide impurity: ¹H NMR (CDCl₃) δ 6.81 (m, 1H), 4.78 (s, 2H), 4.54 (m, 1H), 3.73 (s, 3H), 3.41 (s, 3H), 2.87 (app dd, 1H), 2.64 (br s, 1H), 2.56-2.47 (m, 2H), NH signal was not apparent; ¹³C NMR (CDCl₃) δ 166.9, 132.5, 128.0, 95.9, 69.5, 55.2, 51.6, 31.1, 27.7, 24.1.

### Example 63

**Amine 182:** To a solution of aziridine **170** (3.2 g, 15 mmol) in DMF (30 mL) was applied a vacuum on a rotary evaporator (40°C) for several minutes to degas the solution. To the solution was added sodium azide (4.9 g, 75 mmol) and ammonium chloride (1.6 g, 30 mmol) and the mixture was heated at 65-70°C for 21 h. The reaction mixture was cooled to room temperature, diluted with ethyl acetate (∼100 mL) and was filtered. The filtrate was evaporated and the residue was partitioned between diethyl ether (100 mL) and saturated NaCl (100 mL). The organic phase was washed again with saturated NaCl (100 mL), dried (MgSO₄), filtered, and was evaporated. Additional crude product was obtained from the aqueous washings by extraction with ethyl acetate and treated in the same manner as described above. The crude product was purified on silica gel (5%MeOH/CH₂Cl₂) to afford amine **182** (2.95 g) as an oil which contained a small amount of triphenylphosphine oxide impurity from the previous step: ¹H NMR (CDCl₃) δ 6.82 (t, 1H, *J* = 2.3), 4.81 (d, 1H, *J* = 7.2), 4.77 (d, 1H, *J* = 6.9), 4.09-4.04 (m, 1H), 3.76 (s, 3H), 3.47 and 3.44 (m overlapping s, 4H), 2.94-2.86 (m, 2H), 2.36-2.24 (m, 1H); ¹³C NMR (CDCl₃) δ 165.9, 137.3, 128.2, 96.5, 79.3, 61.5, 55.7, 55.6, 51.9, 29.5.

### Example 64

**N-Trityl aziridine 183:** Amine **182** (2.59 g, 10.2 mmol) was dissolved in 5% HCl/MeOH (30 mL) and the solution was stirred for 3 h at room temperature. Additional 5% HCl/MeOH (10 mL) was added stirring 1 h and the solvent was evaporated to afford 2.52 g of the HCl salt as a tan solid after high vacuum. To a suspension of the HCl salt in CH₂Cl₂ (50 mL) at 0°C was added triethylamine (3.55 mL, 25.5 mmol) followed by the addition of solid trityl chloride (5.55 g, 12.8 mmol) in one portion. The mixture was stirred at 0°C for 1 h and then was warmed to room temperature stirring for 2 h. The reaction was cooled to 0°C, triethylamine (3.6 mL, 25.5 mmol) was added and methane sulfonyl chloride (0.97 mL, 12.5 mmol) was added, stirring the resulting mixture for 1 h at 0°C and for 22 h at room temperature. The reaction was evaporated and the residue was partitioned between diethyl ether (200 mL) and water (200 mL). The organic phase was washed with water (200 mL) and the combined aqueous phases were extracted with diethyl ether (200 mL). The combined organic extracts were washed with water (100 mL), saturated NaCl (200 mL) and were dried (Na₂SO₄), filtered, and evaporated. The crude product was purified on silica gel (1/1-hexane/CH₂Cl₂) to afford N-trityl aziridine **183** (3.84 g, 86%) as a white foam: ¹H NMR (CDCl₃) δ 7.4-7.23 (m, 16H), 432 (m, 1H), 3.81 (s, 3H), 3.06 (dt, 1H, *J* = 1.8, 17.1), 2.94-2.86 (m, 1H), 2.12 (m, 1H), 1.85 (t, 1H, *J* = 5.0).

### Example 65

**Compound 190:** A solution of N-trityl aziridine **183** (100 mg, 0.23 mmol), cyclohexanol (2 mL) and boron trifluoride etherate (42 µL, 0.35 mmol) was heated at 70°C for 1.25 h and was evaporated. The residue was dissolved in pyridine (2 mL) and was treated with acetic anhydride (110 µL, 1.15 mmol) and catalytic DMAP. After stirring for 3 h at room temperature the reaction was evaporated. The residue was partitioned between ethyl acetate and 5% citric acid. The aqueous phase was extracted with ethyl acetate and the combined organic extracts were washed with saturated NaHCO₃, and saturated NaCl. The organic phase was dried (MgSO₄), filtered, and evaporated. The crude product was purified on silica gel (1/1-hexane/ethyl acetate) to afford compound **190** (53 mg, 69%) as a solid: mp 105-107°C (ethyl acetate/hexane); ¹H NMR (CDCl₃) δ 6.78 (m, 1H), 6.11 (d, 1H, *J* = 7.4), 4.61 (m, 1H), 4.32-4.23 (m, 1H), 3.76 (s, 3H), 3.44-3.28 (m, 2H), 2.85 (dd, 1H, *J* = 5.7, 17.6), 2.28-2.17 (m, 1H), 2.04 (s, 3H), 1.88-1.19 (m, 10H).

### Example 66

**Compound 191:** To a solution of compound **190** (49 mg, 0.15 mmol) in THF was added triphenylphosphine (57 mg, 0.22 mmol) and water (270 µL) and the solution was heated at 50°C for 10 h. The reaction was evaporated and the residue was dissolved in ethyl acetate, dried (Na₂SO₄), filtered and evaporated. The crude product was purified on silica gel (1/1-methanol/ethyl acetate) to afford the amine (46 mg) as a pale yellow solid. The a solution of the amine in THF (1.5 mL) was added 1.039N KOH solution (217 µL) and water (200 µL). The mixture was stirred at room temperature for 1 h and was then cooled to 0°C and acidified to pH 6-6.5 with IR 120 ion exchange resin. The resin was filtered, washed with methanol and the filtrate was evaporated. The solid residue was dissolved in water and was passed through a column (4X1 cm) of C-18 reverse phase silica gel eluting with water and then 2.5% acetonitrile/water. Product fractions were combined and evaporated and the residue was dissolved in water and lyophilized to afford amino acid **191** (28 mg) as a white solid: ¹H NMR (D₂O) δ 6.47 (br s, 1H), 4.80 (br d, 1H), 4.00 (dd, 1H, *J* = 8.9, 11.6), 3.59-3.50 (m, 2H), 2.87 (dd, 1H, *J* = 5.5, 17.2), 2.06 (s, 3H), 1.90-1.15 (series of m, 10H); Anal. Calcd for C₁₅H₂₄N₂O₄•H₂O: C, 57.31; H, 8.34; N, 8.91. Found: C, 57.38; H, 8.09; N, 8.77.

### Example 67

**bis-Boc guanidino ester 201:** Treated according to the procedure of Kim and Qian, "Tetrahedron Lett.", 34:7677 (1993). To a solution of amine **200** (529 mg, 1.97 mmol, prepared by the method of Example **109,** bis-Boc thiourea (561 mg, 2.02 mmol) and Et₃N (930 µL) in dry DMF (5.0 mL) cooled to 0°C was added HgCl₂ (593 mg, 2.18 mmol) in one portion. The heterogeneous reaction mixture was stirred for 45 min at 0°C and then at room temperature for 15 min, after which the reaction was diluted with EtOAc and filtered through a pad of celite. Concentration *in vacuo* followed by flash chromatography of the residue on silica gel (10% hexanes in ethyl acetate) gave 904 mg (90%) of **201** as a pale oil. ¹H NMR (CDCl₃, 300 MHz): δ 11.39 (s, 1H); 8.63 (d, 1H, *J* = 7.8 Hz); 6.89 (t, 1H, *J* = 2.4 Hz); 6.46 (d, 1H, *J* = 8.7 Hz); 4.43-4.32 (m, 1H); 4.27-4.17 (m, 1H); 4.13-4.06 (m, 1H); 3.77 (s, 3H); 3.67-3.59 (m, 1H); 2.83 (dd, 1H, *J* = 5.1, 17.7 Hz); 2.45-2.33 (m, 1H); 1.95 (s, 3H); 1.65-1.50 (m, 2H); 1.45 (s, 18H); 0.90 (t, 3H, *J =* 7.5 Hz).

### Example 68

**Carboxylic acid 202:** To a solution of methyl ester **201** (904 mg, 1.77 mmol) in THF (10 mL) was added aqueous KOH (3.45 mL of a 1.039 N solution). The reaction mixture was stirred at room temperature for 17 h, cooled to 0°C and acidified to pH 4.0 with Amberlite IR-120 (H⁺) acidic resin. The resin was filtered and washed with water and methanol. Concentration *in vacuo* gave the free acid as a pale foam which was used without further purification in the next reaction.

### Example 69

**Guanidine carboxylic acid 203:** To a solution of bis-Boc guanidnyl acid **202** (crude from previous reaction) in CH₂Cl₂ (40 mL) cooled to 0°C was added neat trifluoroacetic acid (25 mL). The reaction mixture was stirred at 0°C for 1 h and then at room temperature for 2 h. Concentration *in vacuo* gave a pale orange solid which was purified by C₁₈ reverse phase chromatography eluting with water. Fractions containing the desired product were pooled and lyophilized to give 495 mg (68%, 2 steps) of the guanidine carboxylic acid 203 as the trifluoroacetic acid salt. ¹H NMR (D₂O, 300 MHz): δ 6.66 (s, 1H); 4.29 (bd, 1H, *J* = 9.0 Hz); 4.01 (dd, 1H, *J* = 10.8, 10.8 Hz); 3.87-3.79 (m, 1H); 3.76-3.67 (m, 1H); 3.60-3.50 (m, 1H); 2.83 (dd, 1H, *J* = 5.1, 17.4 Hz); 2.47-2.36 (m, 1H); 2.06 (s, 3H); 1.65-1.50 (m, 2H); 0.90 (t, 3H, *J* = 7.2 Hz). Anal. Calcd for C₁₅H₂₃O₆N₄F₃: C, 43.69; H, 5.62; N, 1359. Found: C, 43.29; H, 5.90; N, 13.78.

### Example 70

**Formamidine carboxylic add 204:** A solution of amino acid **102** (25 mg, 0.10 mmol, prepared by the method of Example **110**) in water (500 µL) at 0 - 5°C was adjusted to pH 8.5 with 1.0 N NaOH. Benzyl formimidate hydrochloride (45 mg, 0.26 mmol) was added in one portion and the reaction mixture was stirred for 3 h at this temperature while maintaining the pH at 8.5 - 9.0 with 1.0 N NaOH. The reaction was then concentrated *in vacuo* and purified by C₁₈ reverse phase chromatography eluting with water. Fractions containing the desired product were pooled and lyophilized to give 4.0 mg (13%) of the formamidine carboxylic acid **204.** ¹H NMR (D₂O, 300 MHz): δ 7.85 (s, 1H); 6.53 (bd, 1H, *J* = 7.8 Hz); 4.32-4.25 (bm, 1H); 4.10-3.97 (m, 1H); 3.76-3.67 (m, 2H); 3.57-3.49 (m, 1H); 2.86-2.81 (m, 1H); 2.55-2.40 (m, 1H); 2.04 (s, 3H); 1.65-1.50 (m, 2H); 0.90 (t, 3H, *J* = 7.4 Hz).

### Example 71

**Amino acid 206:** To a solution of amino methyl ester **205** (84 mg, 0.331 mmol, prepared by Example **107**) in THF (1.0 mL) was added aqueous KOH (481 µL of a 1.039 N solution). The reaction mixture was stirred at room temperature for 2.5 h and acidified to pH 6.5 with Amberlite IR-120 (H⁺) acidic resin. The resin was filtered and washed with water and methanol. Concentration *in vacuo* gave the amino acid as a white solid which was purified by C₁₈ reverse phase chromatography eluting with water. Fractions containing the desired product were pooled and lyophilized to give 59 mg (74%) of the amino acid **206.** ¹H NMR (CD₃OD, 300 MHz): δ 6.60 (bd, 1H, *J* = 1.8 Hz); 4.01-3.95 (m, 1H); 3.71-3.60 (m, 2H); 3.50-3.42 (m, 1H); 3.05-2.85 (m, 2H); 2.39-2.28 (m, 1H); 1.70-1.55 (m, 2H); 0.95 (t, 3H, *J* = 7.5 Hz).

### Example 72

**Trifluoroacetamide 207:** To a degassed solution of amino acid **206** (59 mg, 0.246 mmol) in dry methanol (1.0 mL) under argon was added Et₃N (35 µL) followed by methyl trifluoroacetate (35 µL). The reaction was stirred for one week at room temperature and concentrated. Analysis by ¹H NMR showed that reaction was 40% complete. The crude reaction product was redissolved in dry methanol (1.0 mL), methyl trifluoroacetate (1.0 mL) and Et₃N (0.5 mL) and stirred at room temperature for 5 days. The reaction was then concentrated *in vacuo* and dissolved in 50% aqueous THF (2.0 mL), acidified to pH 4 with Amberlite IR-120 (H⁺) acidic resin and filtered. Concentration gave the crude trifluoroacetamide carboxylic acid which was used without further purification for the next reaction.

### Example 73

**Amino acid 208:** A solution of azide **207** (crude from previous reaction) in THF (2.0 mL) and water (160 µL) was treated with polymer supported triphenyl phosphine (225 mg) at room temperature. After stirring for 20 h the polymer was filtered and washed with methanol. Concentration *in vacuo* gave a pale solid which was purified by C₁₈ reverse phase chromatography eluting with water. Fractions containing the desired product were pooled and lyophilized to give 6.5 mg (9 %) of the trifluoroacetamide amino acid **208.** ¹H NMR (D₂O, 300 MHz): δ 6.59 (bs, 1H); 4.40-4.30 (m, 1H); 4.26 (t, 1H, *J* = 10.1 Hz); 3.80-3.66 (m, 2H); 3.56-3.47 (m, 1H); 2.96 (bdd, 1H, *J* = 5.4,17.7 Hz); 2.58-2.45 (m, 1H); 1.62 - 1.50 (m, 2H); 0.89 (t, 3H, *J* = 7.5 Hz).

### Example 74

**Methylsulfonamide methyl ester 209:** Methanesulfonyl chloride (19 µL) was added to a solution of amine **205** (58 mg, 0.23 mmol, prepared by Example **107**), Et₃N (97 µL) and a catalytic amount of DMAP (few crystals) in CH₂Cl₂ (1.0 mL) at 0°C. After 30 min the reaction mixture was warmed to room temperature and stirred for an additional 1 h. Concentration *in vacuo* followed by flash chromatography of the residue on silica gel (50% hexanes in ethyl acetate) gave 61 mg (79%) of the sulfonamide **209.** ¹H NMR (CDCl₃, 300 MHz): δ 6.87 (t, 1H, *J* = 2.3 Hz); 5.08 (d, 1H, *J* = 7.5 Hz); 4.03-3.90 (m, 1H); 3.78 (s, 3H); 3.75-3.45 (m, 4H); 3.14 (s, 3H); 2.95 (dd, 1H, *J* = 5.2, 17.3 Hz); 2.42-2.30 (m, 1H); 1.75-1.55 (m, 2H); 0.95 (t, 3H, *J* = 7.5Hz).

### Example 75

**Amino ester 210:** A solution of azide **209** (61 mg, 0.183 mmol) in THF (2.0 mL) and water (118 µL) was treated with polymer supported triphenyl phosphine (170 mg) at room temperature. After stirring for 17.5 h the polymer was filtered and washed with methanol. Concentration *in vacuo* followed by flash chromatography of the residue through a short silica gel column (100% methanol) gave 45 mg (80%) of the amino ester 210 as a pale foam. ¹H NMR (CDCl₃, 300 MHz): δ 6.85 (s, 1H); 3.94 (bd, 1H, *J* = 7.8 Hz); 3.77 (s, 3H); 3.74-3.60 (m, 2H); 3.55-3.45 (m, 1H); 3.25-3.15 (m, 1H); 3.11 (s, 3H); 2.94-2.85 (m, 1H); 2.85 (bs, 2H); 2.22-2.10 (m, 1H); 1.70-1.56 (m, 2H); 0.94 (t, 3H, *J* = 7.5 Hz).

### Example 76

**Amino acid 211:** A solution of methyl ester **210** (21 mg, 0.069 mmol) in THF (200 µL) was treated with aqueous KOH (135 µL of a 1.039 N solution). The reaction mixture was stirred at room temperature for 40 min and neutralized to pH 7.0 with Amberlite IR-120 (H⁺) acidic resin. The resin was filtered and washed with water and methanol. Concentration *in vacuo* gave the amino acid as a pale solid which was purified by C₁₈ reverse phase chromatography eluting with water. Fractions containing the desired product were pooled and lyophilized to give 3.5 mg (17%) of the amino acid **211**. ¹H NMR (D₂O, 300 MHz): δ 6.60 (d, 1H, *J* =1.8 Hz); 4.30-4.20 (m, 1H); 3.84-3.75 (m, 1H); 3.68-3.58 (m, 1H); 3.60-3.40 (m, 2H); 3.20 (s, 3H); 2.96-2.88 (m, 1H); 2.55-2.45 (m, 1H); 1.72-1.59 (m, 2H); 0.93 (t, 3H, *J* = 7.4 Hz).

### Example 77

**Bis-Boc guanidino ester 212:** Treated according to the procedure of Kim and Qian, "Tetrahedron Lett." **34**:7677 (1993). To a solution of amine **210** (31 mg, 0.101 mmol), bis-Boc thiourea (28.5 mg, 0.103 mmol) and Et₃N (47 µL) in dry DMF (203 µL) cooled to 0°C was added HgCl₂ (30 mg, 0.11 mmol) in one portion. The heterogeneous reaction mixture was stirred for 30 min at 0°C and then at room temperature for 30 min, after which the reaction was diluted with EtOAc and filtered through a pad of celite. Concentration *in vacuo* followed by flash chromatography of the residue on silica gel (40% hexanes in ethyl acetate) gave 49 mg (89%) of **212** as a pale oil. ¹H NMR (CDCl₃, 300 MHz): δ 11.47 (s, 1H); 8.66 (d, 1H, *J* = 8.4 Hz); 6.87 (s, 1H); 6.01 (bs, 1H); 4.50-4.35 (m, 1H); 4.04 (bd, 1H, *J* = 8.4 Hz); 3.76 (s, 3H); 3.70-3.60 (m, 1H); 3.53-3.45 (m, 2H); 3.02 (s, 3H); 2.85 (dd, 1H, *J* = 53,17.3 Hz); 2.42-2.30 (m, 1H); 1.66-1.55 (m, 2H); 1.49 (s, 9H); 1.48 (s, 9H); 0.93 (t, 3H, *J* = 73 Hz).

### Example 78

**Carboxylic acid 213:** To a solution of methyl ester **212** (49 mg, 0.090 mmol) in THF (1.0 mL) was added aqueous KOH (260 µL of a 1.039 N solution). The reaction mixture was stirred at room temperature for 16 h, cooled to 0°C and acidified to pH 4.0 with Amberlite IR-120 (H⁺) acidic resin. The resin was filtered and washed with water and methanol. Concentration *in vacuo* gave the free acid as a pale foam which was used without further purification in the next reaction.

### Example 79

**Guanidine carboxylic acid 214:** To a solution of bis-Boc guanidnyl acid 213 (crude from previous reaction) in CH₂Cl₂ (2.0 mL) cooled to 0°C was added neat trifluoroacetic acid (2.0 mL). The reaction mixture was stirred at 0°C for 1 h and then at room temperature for 1 h. Concentration *in vacuo* gave a pale orange solid which was purified by C₁₈ reverse phase chromatography eluting with water. Fractions containing the desired product were pooled and lyophilized to give 10 mg (25%, 2 steps) of the guanidine carboxylic acid **214**. ¹H NMR (D₂O, 300 MHz): δ 6.60 (bs, 1H); 4.22 (bd,1H, *J* = 9.0 Hz); 3.82-3.66 (m, 2H); 3.65-3.54 (m, 1H); 3.43 (bt, 1H, *J* = 9.9 Hz); 3.15 (s, 3H); 2.82 (dd, 1H, *J* = 5.0, 17.5 Hz); 2.48-2.30 (m, 1H); 1.71-1.58 (m, 2H); 0.93 (t, 3H, *J* = 73 Hz).

### Example 80

**Propionamide methyl ester 215:** Propionyl chloride (96 µL, 1.1 mmol) was added to a solution of amine **205** (178 mg, 0.70 mmol, prepared by Example **107**) and pyridine (1.5 mL) in CH₂Cl₂ (2.0 mL) cooled to 0°C. After 30 min at 0°C the reaction was concentrated and partitioned between ethyl acetate and brine. The organic layer was separated and washed sequentially with saturated sodium bicarbonate, brine and dried over MgSO₄. Concentration *in vacuo* followed by flash chromatography of the residue on silica gel (40% hexanes in ethyl acetate) gave 186 mg (86%) of the propionamide methyl ester **215** as a pale yellow solid. ¹H NMR (CDCl₃, 300 MHz): δ 6.86 (t, 1H, *J* = 2.3 Hz); 5.72 (bd, 1H, *J* = 7.8 Hz); 4.52-4.49 (m, 1H); 4.25-4.15 (m, 1H); 3.77 (s, 3H); 3.65-3.37 (complex m, 3H); 2.87 (dd, 1H, *J* = 5.7,17.7 Hz); 2.28 (q, 2H, *J* = 7.5 Hz); 2.25-2.20 (m, 1H); 1.65-1.50 (m, 2H); 1.19 (t, 3H, *J* = 7.5 Hz); 0.92 (t, 3H, *J* = 7.5 Hz).

### Example 81

**Amino methyl ester 216:** A solution of azide **215** (186 mg, 0.60 mmol) in THF (5.0 mL) and water (400 µL) was treated with polymer supported triphenyl phosphine (560 mg) at room temperature. After stirring for 21 h the polymer was filtered and washed with methanol. Concentration *in vacuo* gave the crude amino ester **216** which was used without any further purification for the next step.

### Example 82

**Amino acid 217:** A solution of methyl ester **216** (crude from previous reaction) in THF (500 µL) was treated with aqueous KOH (866 µL of a 1.039 N solution). The reaction mixture was stirred at room temperature for 3 h and neutralized to pH 7.0 with Amberlite IR-120 (H⁺) acidic resin. The resin was filtered and washed with water and methanol. Concentration *in vacuo* gave the amino acid as a pale solid which was purified by C₁₈ reverse phase chromatography eluting with water. Fractions containing the desired product were pooled and lyophilized to give 49 mg (31% 2 steps) of the amino acid **217.** ¹H NMR (D₂O, 300 MHz): δ 6.54 (s, 1H); 4.25 (bd, 1H, *J* = 8.7 Hz); 4.13 (dd, 1H, *J* = 9.0, 11.3 Hz); 3.74-3.60 (m, 1H); 3.61-3.40 (m, 2H); 2.85 (dd, 1H, *J* = 5.9,17.1 Hz); 2.55-2.40 (m, 1H); 2.35 (q, 2H, *J* = 7.5 Hz); 1.65-1.45 (m, 2H); 1.13 (t, 3H, *J* = 7.5 Hz); 0.88 (t, 3H, *J* = 7.5 Hz).

### Example 83

**(mono methyl) bis-Boc guanidino ester 218:** To a solution of amine **200** (51 mg, 0.19 mmol) and mono methyl bis-Boc thiourea (36 mg, 0.19 mmol) in dry DMF (1.0 mL) , was added 1-(3-Dimethylaminopropyl)-3-ethylcarbodiimide hydrochloride (38 mg) and Et₃N (56 µL) at room temperature. After 1.5 h at room temperature HgCl₂ (∼75 mg, excess) was added in one portion. The heterogeneous reaction mixture was stirred for 45 min, diluted with ethyl acetate and filtered through a pad of celite. The filtrate was diluted with additional ethyl acetate and washed with dilute HCl, saturated sodium bicarbonate, brine and dried over MgSO₄. Concentration *in vacuo* followed by flash chromatography of the residue on silica gel (10% methanol in ethyl acetate) gave 13 mg (16%) of the (mono methyl) bis-Boc guanidino ester **218** as a colorless foam. ¹H NMR (CDCl₃, 300 MHz): δ 6.84 (s, 1H); 6.20 (bd, 1H, *J* = 5.1 Hz); 5.45 (bs, 1H); 4.25-4.40 (bm, 1H); 4.20-4.05 (bm, 2H); 3.76 (s, 3H); 3.60-3.50 (m, 1H); 3.43-3.30 (m, 1H); 2.90 (dd, 1H, *J* = 5.4,17.7 Hz); 2.77 (d, 3H, *J* = 4.8 Hz); 2.35-2.25 (m, 1H); 1.96 (s, 3H); 1.60-1.50 (m, 2H); 1.47 (s, 9H); 0.91 (t, 3H, *J* = 7.2 Hz).

### Example 84

**(mono methyl) bis-Boc guanidino acid 219:** To a solution of methyl ester **218** (13 mg, 0.031 mmol) in THF (500 µL) was added aqueous KOH (60 µL of a 1.039 N solution). The reaction mixture was stirred at room temperature for 1 h and then gently refluxed for 1 h. The reaction was cooled to 0°C and acidified to pH 6.0 with Amberlite IR-120 (H⁺) acidic resin. The resin was filtered and washed with water and methanol. Concentration *in vacuo* gave the free acid **219** which was used without further purification in the next reaction.

### Example 85

**(mono methyl) guanidino amino acid 220:** To a solution of (mono methyl) bis-Boc guanidnyl acid **219** (crude from previous reaction) in CH₂Cl₂ (1.0 mL) cooled to 0°C was added neat trifluoroacetic acid (1.0 mL). The reaction mixture was stirred at 0°C for 1 h and then at room temperature for 1 h. Concentration *in vacuo* gave a pale solid which was purified by C₁₈ reverse phase chromatography eluting with water. Fractions containing the desired product were pooled and lyophilized to give 4.4 mg (33%, 2 steps) of the guanidine carboxylic acid **220.** ¹H NMR (D₂O, 300 MHz): δ 6.52 (bs, 1H); 4.27 (bd, 1H, *J* = 8.4 Hz); 4.01 (dd, 1H, *J* = 9.2, 10.3 Hz); 3.86-3.75 (m, 1H); 3.75-3.67 (m, 1H); 3.60-3.49 (m, 1H); 2.85 (s, 3H); 2.80 (dd, 1H, *J* = 5.1, 17.7 Hz); 2.47-2.37 (m, 1H); 2.04 (s, 3H); 1.64-1.50 (m, 2H); 0.90 (t, 3H, *J* = 7.2 Hz).

### Example 86

**(R)-methyl propyl ester 221:** BF₃•Et₂O (63 µL, 0.51 mmol) was added to a solution of N-trityl aziridine **183** (150 mg, 0.341 mmol) in (R)-(-)-2-butanol (1.2 mL) under argon with stirring at room temperature. The pale solution was heated at 70 °C for 2 h and then concentrated *in vacuo* to give a brown residue which was dissolved in dry pyridine (2.0 mL) and treated with acetic anhydride (225 µL) and a catalytic amount of DMAP (few crystals) at 0°C. The reaction was allowed to warm to room temperature and stirred for 2 h, concentrated *in vacuo* and partitioned between ethyl acetate and brine. The organic layer was separated and washed sequentially with dilute HCl, saturated sodium bicarbonate, brine and dried over MgSO₄. Concentration *in vacuo* followed by flash chromatography of the residue on silica gel (50% hexanes in ethyl acetate) gave 75 mg (72%) of the (R)-methyl propyl ester **221** as a pale solid. ¹H NMR (CDCl₃, 300 MHz): δ 6.79 (t, 1H, *J* = 2.2 Hz); 6.14 (d, 1H, *J* = 7.3 Hz); 4.55 (bd, 1H, *J* = 8.7 Hz); 4.33-4.23 (m, 1H); 3.77 (s, 3H); 3.56-3.45 (m, 1H); 3.40-3.27 (m, 1H); 2.85 (dd, 1H, *J* = 5.5, 17.5 Hz); 2.30-2.15 (m, 1H); 2.04 (s, 3H); 1.5901.40 (m, 2H); 1.10 (d, 3H, *J* = 6.0 Hz); 0.91 (t, 3H, *J* = 7.4 Hz).

### Example 87

**(R)-methyl propyl amino ester 222:** Ph₃P (95 mg, 0.36 mmol) was added in one portion to a solution of azide **221** (75 mg, 0.24 mmol) and water (432 µL) in THF (3.0 mL). The pale yellow solution was then heated at 50°C for 10 h, cooled and concentrated *in vacuo* to give a pale solid. Purification by flash chromatography on silica gel (50% methanol in ethyl acetate) gave 66 mg (97%) of the amino ester **222** as a pale solid.

### Example 88

**Amino acid 223:** A solution of methyl ester **222** (34 mg, 0.12 mmol) in THF (1.0 mL) was treated with aqueous KOH (175 µL of a 1.039 N solution). The reaction mixture was stirred at room temperature for 3 h and acidified to pH 6.0 with Amberlite IR-120 (H⁺) acidic resin. The resin was filtered and washed with water and methanol. Concentration *in vacuo* gave the amino acid as a pale solid which was purified by C₁₈ reverse phase chromatography eluting with water. Fractions containing the desired product were pooled and lyophilized to give 11.5 mg (36%) of the amino acid **223**. ¹H NMR (D₂O, 300 MHz): δ 6.52 (bs, 1H); 4.28 (bd, 1H, *J* = 8.7 Hz); 4.04 (dd, 1H, *J* = 8.8,11.5 Hz); 3.74-3.65 (m, 1H); 3.50-3.60 (m, 1H); 2.90 (dd, 1H, *J* = 5.5, 17.2 Hz); 2.50-2.40 (m, 1H0; 2.10 (s, 3H); 1.60-1.45 (m, 2H); 1.14 (d, 3H, *J* = 6.2 Hz); 0.91 (t, 3H, *J* = 7.4 Hz).

### Example 89

**bis-Boc guanidino ester 224:** Treated according to the procedure of Kim and Qian, "Tetrahedron Lett.", 34:7677 (1993). To a solution of amine 222 (32 mg, 0.113 mmol), bis-Boc thiourea (32 mg, 0.115 mmol) and Et₃N (53 µL) in dry DMF (350 µL) cooled to 0°C was added HgCl₂ (34 mg, 0.125mmol) in one portion. The heterogeneous reaction mixture was stirred for 45 min at 0°C and then at room temperature for 1 h, after which the reaction was diluted with EtOAc and filtered through a pad of celite. Concentration *in vacuo* followed by flash chromatography of the residue on silica gel (20% hexanes in ethyl acetate) gave 57 mg (96%) of **224** as a colorless foam. ¹H NMR (CDCl₃, 300 MHz): δ 11.40 (s, 1H); 8.65 (d, 1H, *J* = 7.8 Hz); 6.82 (s, 1H); 6.36 (d, 1H, *J* = 8.7 Hz); 4.46-4.34 (m, 1H); 4.20-4.10 (m, 1H); 4.10-3.95 (m, 1H); 3.76 (s, 3H); 2.79 (dd, 1H, *J* = 5.4,17.7 Hz); 2.47-2.35 (m, 1H); 1.93 (s, 3H); 1.60-1.45 (m, 2H); 1.49 (s, 18H); 1.13 (d, 3H, *J* = 6.0 Hz); 0.91 (t, 3H, *J* = 7.5 Hz).

### Example 90

**Carboxylic acid 225:** To a solution of methyl ester **224** (57 mg, 0.11 mmol) in THF (1.5 mL) was added aqueous KOH (212 µL of a 1.039 N solution). The reaction mixture was stirred at room temperature for 16 h, cooled to 0°C and acidified to pH 4.0 with Amberlite IR-120 (H⁺) acidic resin. The resin was filtered and washed with water and methanol. Concentration *in vacuo* gave the free acid as a pale foam which was used without further purification in the next reaction.

### Example 91

**Guanidine carboxylic acid 226:** To a solution of bis-Boc guanidnyl acid **225** (crude from previous reaction) in CH₂Cl₂ (4.0 mL) cooled to 0°C was added neat trifluoroacetic acid (4.0 mL). The reaction mixture was stirred at 0°C for 1 h and then at room temperature for 2 h. Concentration *in vacuo* gave a pale orange solid which was purified by C₁₈ reverse phase chromatography eluting with water. Fractions containing the desired product were pooled and lyophilized to give 18.4 mg (40%, 2 steps) of the guanidine carboxylic acid **226.** ¹H NMR (D₂O, 300 MHz): δ 6.47 (s. 1H); 4.28 (bd, 1H, *J* = 8.4 Hz); 3.93-3.74 (m, 2H); 3.72-3.63 (m, 1H); 2.78 (dd, 1H, *J* = 4.8, 17.4 Hz); 2.43-2.32 (m, 1H); 1.58-1.45 (m, 2H); 1.13 (d, 3H, *J* = 6.0 Hz); 0.90 (t, 3H, *J* = 7.4 Hz).

### Example 92

**(Diethyl) methyl ether ester 227:** BF₃•Et₂O (6.27 mL, **51** mmol) was added to a solution of N-trityl aziridine 183 (15 g, 34 mmol) in 3-pentanol (230 mL) under argon with stirring at room temperature. The pale solution was heated at 70-75°C for 1.75 h and then concentrated *in vacuo* to give a brown residue which was dissolved in dry pyridine (2.0 mL) and treated with acetic anhydride (16 mL, 170 mmol) and a catalytic amount of DMAP 200 mg. The reaction was stirred at room temperature for 18 h, concentrated *in vacuo* and partitioned between ethyl acetate and 1M HCl. The organic layer was separated and washed sequentially with saturated sodium bicarbonate, brine and dried over MgSO₄. Concentration *in vacuo* followed by flash chromatography of the residue on silica gel (50% hexanes in ethyl acetate) gave 7.66 g of the (Diethyl) methyl ether ester which was recrystallized from ethylacetate/hexane to afford **227** (7.25 g, 66%) as colorless needles: ¹H NMR (CDCl₃, 300 MHz): δ 6.79 (t, 1H, *J* = 2.1 Hz); 5.92 (d, 1H, *J* = 75 Hz); 4.58 (bd, 1H, *J* = 8.7 Hz); 4.35-4.25 (m, 1H); 3.77 (s, 3H); 3.36-3.25 (m, 2H); 2.85 (dd, 1H, *J* = 5.7, 17.4 Hz); 2.29-2.18 (m, 1H); 2.04 (s, 3H); 1.60-1.45 (m, 4H); 0.91 (t, 3H, *J* = 3.7 Hz); 0.90 (t, 3H, *J* = 7.3 Hz).

### Example 93

**(Diethyl) methyl ether amino ester 228:** Ph₃P (1.21 g, 4.6 mmol) was added in one portion to a solution of azide **227** (1 g, 3.1 mmol) and water (5.6 mL) in THF (30 mL). The pale yellow solution was then heated at 50°C for 10 h, cooled and concentrated *in vacuo*. The aqueous oily residue was partitioned between EtOAc and saturated NaCl. The organic phase was dried (MgSO₄), filtered, and evaporated. Purification by flash chromatography on silica gel (50% methanol in ethyl acetate) gave 830 mg (90%) of the amino ester 228 as a pale white solid. ¹H NMR (CDCl₃, 300 MHz): δ 6.78 (t, 1H, *J* = 2.1 Hz); 5.68 (bd, 1H, *J* = 7.8 Hz); 4.21-4.18 (m, 1H); 3.75 (s, 3H); 354-3.45 (m, 1H); 3.37-3.15 (m, 2H); 2.74 (dd, 1H, *J* = 5.1, 17.7 Hz); 2.20-2.07 (m, 1H); 2.03 (s, 3H); 1.69 (bs, 2H, -NH₂); 1.57-1-44 (m, 4H); 0.90 (t, 3H, *J* = 7.5 Hz); 0.89 (t, 3H, *J* = 7.5 Hz).

### Example 94

**Amino acid 229:** A solution of methyl ester **228** (830 mg, 2.8 mmol) in THF (15 mL) was treated with aqueous KOH (4 mL of a 1.039 N solution). The reaction mixture was stirred at room temperature for 40 min and acidified to pH 5.5-6.0 with Dowex 50WX8 acidic resin. The resin was filtered and washed with water and methanol. Concentration *in vacuo* gave the amino acid as a pale solid which was purified by C₁₈ reverse phase chromatography eluting with water and then with 5% CH₃CN/water. Fractions containing the desired product were pooled and lyophilized to give 600 mg (75%) of the amino acid **229.** ¹H NMR (D₂O, 300 MHz): δ 6.50 (t, 1H, *J* = 2.1 Hz); 4.30-4.26 (m, 1H); 4.03 (dd, 1H, *J* = 9.0,11.7 Hz); 3.58-3.48 (m, 2H); 2.88 (dd, 1H, *J* = 5.4,16.8 Hz); 2.53-2.41 (m, 1H); 1.62-1.40 (m, 4H); 0.90 (t, 3H, *J* = 7.5 Hz); 0.85 (t, 3H, *J* = 7.5 Hz).

### Example 95

***t*****-amyl ether ester 230:** BF₃•Et₂O (43 µL, 0.35 mmol) was added to a solution of N-trityl aziridine **183** (104 mg, 0.24 mmol) in *t*-amyl alcohol (2.5 mL) under argon with stirring at room temperature. The pale solution was heated at 75°C for 3 h and then concentrated *in vacuo* to give a brown residue which was dissolved in dry pyridine (2.0 mL) and treated with acetic anhydride (250 µL) and a catalytic amount of DMAP (few crystals). The reaction was stirred at room temperature for 1.5 h, concentrated *in vacuo* and partitioned between ethyl acetate and brine. The organic layer was separated and washed sequentially with dilute HCl, saturated sodium bicarbonate, brine and dried over MgSO₄. Concentration *in vacuo* followed by flash chromatography of the residue on silica gel (50% hexanes in ethyl acetate) gave 27 mg (35%) of the *t*-amyl ether ester 230 as a pale orange oil. ¹H NMR (CDCl₃, 300 MHz): δ 6.72 (t, 1H, *J* = 2.1 Hz); 5.83 (d, 1H, *J* = 7.2 Hz); 4.71 (bd, 1H, *J* = 8.1 Hz); 4.45-4.35 (m, 1H); 3.75 (s, 3H); 3.27-3.17 (m, 1H); 2.84 (dd, 1H, *J* = 5.7, 17.4 Hz); 2.27-2.15 (m, 1H); 2.05 (s, 3H); 1.57-1.47 (m, 2H); 1.19 (s, 3H); 1.15 (s, 3H); 0.90 (t, 3H, *J* = 7.5 Hz).

### Example 96

***t*****-amyl ether amino ester 231:** Ph₃P (35 mg, 0.133 mmol) was added in one portion to a solution of azide **230** (27 mg, 0.083 mmol) and water (160 µL) in THF (1.5 mL). The pale orange solution was then heated at 50°C for 10 h, cooled and concentrated *in vacuo* to give a pale solid. Purification by flash chromatography on silica gel (50% methanol in ethyl acetate) gave 20 mg (82%) of the amino ester **231** as a pale oil.

### Example 97

**Amino acid 232:** A solution of methyl ester **231** ( 20 mg, 0.068 mmol) in THF (1.0 mL) was treated with aqueous KOH (131 µL of a 1.039 N solution). The reaction mixture was stirred at room temperature for 2.5 h and acidified to pH 5.0 with Amberlite IR-120 (H⁺) acidic resin. The resin was filtered and washed with water and methanol. Concentration *in vacuo* gave the amino acid as a pale solid which was purified by C₁₈ reverse phase chromatography eluting with water. Fractions containing the desired product were pooled and lyophilized to give 8.6 mg (45%) of the amino acid **232.** ¹H NMR (D₂O, 300 MHz): δ 6.47 (bs, 1H); 4.42 (bd, 1H, *J* = 8.1 Hz); 3.97 (dd, 1H, *J* = 8.4, 11.4 Hz); 3.65-3.54 (m, 1H); 2.88 (dd, 1H, *J* = 5.5,17.3 Hz); 2.51-2.39 (m, 1H); 2.08 (s, 3H); 1.61-1.46 (m, 2H); 1.23 (s, 3H); 1.18 (s, 3H), 0.86 (t, 3H, *J* = 7.5 Hz).

### Example 98

***n*****-Propyl thio ether ester 233:** BF₃•Et₂O (130 µL, 1.06 mmol) was added to a solution of N-trityl aziridine **183** (300mg, 0.68 mmol) in 1-propanethiol (8.0 mL) under argon with stirring at room temperature. The pale solution was then heated at 65°C for 45 min, concentrated and partitioned between ethyl acetate and brine. The organic layer was separated and washed with saturated sodium bicarbonate, brine and dried over MgSO₄. Concentration *in vacuo* followed by flash chromatography of the residue on silica gel (30% hexanes in ethyl acetate) gave **134** mg (73%) of the *n*-propyl thio ether ester **233** as a pale oil. ¹H NMR (CDCl₃, 300 MHz): δ 6.87 (t, 1H, *J* = 2.4 Hz); 3.77 (s, 3H); 3.48-3.38 (m, 1H); 3.22-3.18 (m, 1H), 2.93 (dd, 1H, *J* = 5.4,17.4 Hz); 2.80 (t, 1H, *J* = 9.9 Hz); 2.51 (t, 2H, *J* = 7.2 Hz); 2.32-2.20 (m, 1H); 1.96 (bs, 2H, -NH₂), 1.69-1.56 (m, 2H); 1.00 (t, 3H, *J* = 7.2 Hz).

### Example 99

***n*****-Propyl thio ether azido ester 234:** To a solution of amine **233** (134 mg, 0.50 mmol) in pyridine (1.5 mL) cooled to 0°C was added neat acetyl chloride (60 µL, 0.84 mmol). After stirring for 1 h the reaction mixture was warmed to room temperature and stirred for an additional 15 min. The reaction was concentrated and partitioned between ethyl acetate and brine and washed sequentially with dilute HCl, water, saturated sodium bicarbonate, brine and dried over MgSO₄. Concentration *in vacuo* followed by flash chromatography of the residue on silica gel (30% hexanes in ethyl acetate) gave 162 mg (100%) of the *n*-Propyl thio ether azido ester **234** as a pale yellow solid. ¹H NMR (CDCl₃, 300 MHz): δ 6.90 (t, 1H, *J* = 2.7 Hz); 5.87 (bd, 1H, *J* = 7.8 Hz); 4.07-3.98 (m, 1H); 3.77 (s, 3H); 3.65-3.55 (m, 1H); 2.95-2.85 (m, 1H); 2.60-2.45 (m, 2H); 2.30-2.18 (m, 1H); 2.08 (s, 3H); 1.65-1.53 (m, 2H); 0.98 (t, 3H, *J* = 7.2 Hz).

### Example 100

***n*****-Propyl thio ether amino ester 235:** The azide **234** (130 mg, 0.416 mmol) in ethyl acetate (10 mL) was hydrogenated (1 atmosphere) over Lindlar's catalyst (150 mg) for 18 h at room temperature. The catalyst was then filtered through a celite pad and washed with hot ethyl acetate and methanol. Concentration *in vacuo* followed by flash chromatography of the orange residue gave 62 mg (53%) of the n-propyl thio ether amino ester 235. ¹H NMR (CDCl₃, 300 MHz): δ 6.88 (t, 1H, *J* = 2.7 Hz); 5.67 (bd, 1H, *J* = 8.7 Hz); 3.76 (s, 3H); 3.75-3.65 (m, 1H); 3.45-3.35 (bm, 1H); 3.05-2.95 (m, 1H); 2.87-2.78 (m, 1H); 2.56-2.40 (m, 2H); 2.18-2.05 (m, 1H); 2.09 (s, 3H); 1.65-1.50 (m, 2H); 1.53 (bs, 2H, -NH₂); 0.98 (t, 3H, *J* = 7.2 Hz).

### Example 101

**Compound 240:** A suspension of Quinic acid **(103** g), 2,2-dimethoxypropane (200 mL) and toluenesulfonic acid (850 mg) in acetone (700 mL) was stirred at room temperature for 4 days. Solvents and excess reagents were removed under reduced pressure. Purification by flash column chromatography (Hexanes/EtOAc = 2/1-1.5/1) gave lactone **240** (84 g, 73%): ¹H NMR (CDCl₃) δ 4.72 (dd, J = 2.4, 6.1 Hz, 1 H), 4.50 (m, 1 H), 4.31 (m, 1 H), 2.67 (m, 2 H), 2.4-2.2 (m, 3 H), 1.52 (s, 3 H), 1.33 (s, 3 H). Performing the reaction at reflux temperatures for 4 h afforded lactone **240** in 71% yield after aqueous work-up (ethyl acetate/water partition) and recrystallization of the crude product from ethyl acetate/hexane.

### Example 102

**Compound 241:** To a solution of lactone **240** (43.5 g, 203 mmol) in methanol (1200 mL) was added sodium methoxide (4.37 M, 46.5 ml, 203 mmol) in one portion. The mixture was stirred at room temperature for 3 hrs, and quenched with acetic acid (11.62 mL). Methanol was removed under reduced pressure. The mixture was diluted with water, and extracted with EtOAc (3x). The combined organic phase was washed with water (1x) and brine (1x), and dried over MgSO₄. Purification by flash column chromtography (Hexanes/EtOAc = 1/1 to 1/4) gave diol (43.4g, 87%): ¹H NMR (CDCl₃) δ 4.48 (m, 1 H), 4.13 (m, 1 H), 3.99 (t, J = 6.4 Hz, 1 H), 3.82 (s, 3 H), 3.34 (s, 1 H), 2.26 (d, J = 3.8 Hz, 2 H), 2.08 (m, 1 H), 1.91 (m, 1 H), 1.54 (s, 3 H), 1.38 (s, 3 H). Alternatively, treatment of lactone **240** with catalytic sodium ethoxide (1 mol%) in ethanol gave the corresponding ethyl ester in 67% after crystallization of the crude product from ethyl acetate/hexane. The residue obtained from the mother liquor (consisting of starting material and product) was subjected again to the same reaction conditions, affording additional product after recrystallization. Overall yield was 83%.

### Example 103

**Compound 242:** To a solution of diol **241** (29.8 g, 121 mmol) and 4-(N,N-dimethylamino)pyridine (500 mg) in pyridine (230 mL) was added tosyl chloride (27.7 g, 145 mmol). The mixture was stirred at room temperature for 3 days, and pyridine was removed under reduced pressure. The mixture was diluted with water, and extracted with EtOAc (3x). The combined organic phase was washed with water (2x) and brine (1x), and dried over MgSO₄. Concentration and purification by flash column chromatography (Hexanes/EtOAc = 2/1-1/1) gave tosylate **242** (44.6 g, 92%): ¹H NMR (CDCl₃) δ 7.84 (d, J = 8.4 Hz, 2 H), 7.33 (d, J = 8.1 Hz, 2 H), 4.76 (m, 1 H), 4.42 (m, 1 H), 4.05 (dd, J = 5.5, 7.5 Hz, 1 H), 3.80 (s, 3 H), 2.44 (s, 3 H), 2.35 (m, 1 H), 2.24 (m, 2 H), 1.96 (m, 1 H), 1.26 (s, 3 H), 1.13 (s, 3 H). The corresponding ethyl ester of compound **241** was treated with methanesulfonyl chloride and triethylamine in CH₂Cl₂ at 0°C to afford the mesylate derivative in quantitative yield after aqueous work-up. The mesylate was used directly without any further purification.

### Example 104

**Compound 243:** To a solution of tosylate **242** (44.6 g, 111.5 mmol) in CH₂Cl₂ (450 mL) at -78°C was added pyridine (89 mL), followed by slow addition of SO₂Cl₂ (26.7 mL, 335 mmol). The mixture was stirred at -78°C for 5 hrs, and methanol (45 mL) was added dropwise. The mixture was warmed to room temperature and stirred for 12 hrs. Ethyl ether was added, and the mixture was washed with water (3x) and brine (1x), and dried over MgSO₄. Concentration gave the intermediate as a oil (44.8 g). To a solution of the intermediate (44.8 g, 111.5 mmol) in MeOH (500 mL) was added TsOH (1.06 g, 5.6 mmol). The mixture was refluxed for 4 hrs. The reaction mixture was cooled to room temperature, and methanol was removed under reduced pressure. Fresh methanol (500 mL) was added, and the whole mixture was refluxed for another 4 hrs. The reaction mixture was cooled to room temperature, and methanol was removed under reduced pressure. Purification by flash column chromatography (Hexanes/EtOAc = 3/1-1/3) gave a mixture of the two isomers (26.8 g). Recrystalization from EtOAc/Hexanes afforded the pure desired product 243 (20.5 g, 54%): ¹H NMR (CDCl₃) δ 7.82 (d, J = 8.3 Hz, 2 H), 7.37 (d, J = 8.3 Hz, 2 H), 6.84 (m, 1 H), 4.82 (dd, J = 5.8, 7.4 Hz, 1 H), 4.50 (m, 1 H), 3.90 (dd, J = 4.4, 8.2 Hz, 1 H), 3.74 (s, 3 H), 2.79 (dd, J = 5.5,18.2 Hz, 1 H), 2.42 (dd, J = 6.6,18.2 Hz, 1 H). The corresponding mesylate-ethyl ester derivative of compound **242** was treated in the same manner as described. Removal of the acetonide protecting group was accomplished with acetic acid in refluxing ethanol to afford the diol in 39% yield by direct precipitation with ether from the crude reaction mixture.

### Example 105

**Compound 1:** To a solution of diol **243** (20.0 g, 58.5 mmol) in THF (300 mL) at 0°C was added DBU (8.75 mL, 58.5 mmol). The reaction mixture was warmed to room temperature, and stirred for 12 hrs. Solvent (THF) was removed under reduced pressure. Purification by flash column chromatography (Hexanes/EtOAc = 1/3) gave epoxide 1 (9.72 g, 100%): ¹H NMR (CDCl₃) δ 6.72 (m, 1 H), 4.56 (td, J = 2.6, 10.7 Hz, 1 H), 3.76 (s, 3 H), 3.56 (m, 2 H), 3.0 (d, J = 21 Hz, 1 H), 2.50 (d, J = 20 Hz, 1 H), 2.11 (d, 10.9 Hz, 1 H). The corresponding mesylate-ethyl ester derivative of compound **243** was treated in the same manner as described, affording the epoxide in nearly quantitative yield.

### Example 106

**Aziridine 244:** A solution of allyl ether **4** (223 mg, 1.07 mmol) and Lindlar's catalyst (200 mg) in absolute ethanol (8.0 mL) was treated with hydrogen gas (1 atmosphere) at room temperature for 50 min. The catalyst was then filtered through a celite pad and washed with hot methanol. Concentration *in vacuo* gave ∼230 mg of **244** as pale yellow oil which was used for the next reaction without any further purification.

### Example 107

**Azido amine 205:** Crude aziridine **244** (230 mg ), sodium azide (309 mg, 4.75 mmol) and ammonium chloride (105 mg, 1.96 mmol) in dry DMF (10 mL) was heated at 70°C for 16 h under an argon atmosphere. The reaction was cooled, filtered through a fritted glass funnel to remove solids and partitioned between ethyl acetate and brine. The organic layer was separated and dried over MgSO₄. Concentration *in vacuo* followed by flash chromatography of the residue on silica gel (10% hexanes in ethyl acetate) gave 154 mg (57%, 2 steps) of **205** as a yellow viscous oil of sufficient purity for the next reaction.

### Example 108

**N-acetyl azide 245:** Acetyl chloride (70 µl, 0.98 mmol) was added to a solution of amine **205** (154 mg, 0.61 mmol) and pyridine (1.3 mL) in CH₂Cl₂ (4.0 mL) cooled to 0°C. After 1.5 h at 0°C the reaction was concentrated and partitioned between ethyl acetate and brine. The organic layer was separated and washed sequentially with saturated sodium bicarbonate, brine and dried over MgSO₄. Concentration *in vacuo* followed by flash chromatography of the residue on silica gel (ethyl acetate) gave 167 mg (93%) of **245** as a pale yellow solid.

### Example 109

**Amino ester 200**: Triphenyl phosphine (1.7 g, 6.48 mmol) was added in several portions to a solution of **245** (1.78 g, 6.01 mmol) in THF (40 mL) and water (1.5 mL). The reaction was then stirred at room temperature for 42.5 h. Volatiles were removed under vaccum and the crude solid absorbed onto silica gel and purified by flash chromatography on silica gel (100% ethyl acetate then 100% methanol) to give 1.24 g (77%) of **200** as a pale solid.

### Example 110

**Amino acid 102:** To a solution of methyl ester **200** (368 mg, 1.37 mmol) in THF (4.0 mL) cooled to 0°C was added aqueous NaOH (1.37 mL of a 1.0 N solution). The reaction mixture was stirred at 0°C for 10 min, room temperature for 1.5 h and then acidified to pH 7.0-7.5 with Amberlite IR-120 (H⁺) acidic resin. The resin was filtered and washed with water and methanol. Concentration *in vacuo* gave the amino acid as a white solid which was purified by C₁₈ reverse phase chromatography eluting with water. Fractions containing the desired product were pooled and lyophilized to give 290 mg (83%) of amino acid **102.**

### Example 111

**Amine hydrochloride 250:** Amine **228** (15.6 mg, 0.05 mmol) was treated with 0.1 N HCl and was evaporated. The residue was dissolved in water and was filtered through a small column of C-18 reverse phase silica gel. The hydrochloride salt **250** (12 mg) was obtained as a solid after lyophilization: ¹H NMR (D₂O) δ 6.86 (s, 1H), 4.35 (br d, *J* = 9.0), 4.06 (dd, 1H, *J* = 9.0,11.6), 3.79 (s, 3H), 3.65-3.52 (m, 2H), 2.97 (dd, 1H, *J* = 5.5, 17.2), 2.58-2.47 (m, 1H), 2.08 (s, 3H), 1.61-1.41 (m, 4H), 0.88 (t, 3H, *J* = 7.4), 0.84 (t, 3H, *J* = 7.4).

### Example 112

**Bis-Boc-guanidine 251:** To a solution of amine **228** (126 mg, 0.42 mmol), *N,N'*-bis-*tert*-butoxycarbonylthiourea (127 mg, 0.46 mmol), and triethylamine (123 µL, 0.88 mmol) in DMF (4 mL) at 0°C was added HgCl₂ (125 mg, 0.46 mmol). The mixture was stirred at 0°C for 30 min and at room temperature for 1.5 h. The reaction was diluted with ethyl acetate and filtered through celite. The solvent was evaporated and the residue was partitioned between ethyl acetate and water. The organic phase was washed with saturated NaCl, dried (MgSO₄), filtered and the solvent was evaporated. The crude product was purified on silica gel (2/1, 1/1-hexane/ethyl acetate) to afford bis-Boc-guanidine **251** (155 mg, 69%) as a solid: ¹H NMR (CDCl₃) δ 11.40 (s, 1H), 8.66 (d, 1H, *J* = 7.9), 6.8 (s, 1H), 6.22 (d, 1H, *J* = 8.9), 4.43-4.34 (m, 1H), 4.19-4.08 (m, 1H), 4.03 (m, 1H), 3.76 (s, 3H), 3.35 (m, 1H), 2.79 (dd, 1H, *J* = 5.4, 17.7), 2.47-2.36 (m, 1H), 1.92 (s, 3H), 1.50, 1.49 (2s, 18H), 0.89 (m, 6H).

### Example 113

**Guanidino-acid 252:** To a solution of bis-Boc-guanidine **251** (150 mg, 0.28 mmol) in THF (3 mL) was added 1.039N KOH solution (337 µL) and water (674 µL). The mixture was stirred for 3 h, additional 1.039N KOH solution (67 µL) was added and stirring was continued for 2 h. The reaction was filtered to remove a small amount of dark precipitate. The filtrate was cooled to 0°C and was acidified with IR 120 ion exchange resin to pH 4.5-5.0. The resin was filtered and washed with methanol. The filtrate was evaporated to a residue which was dissolved in CH₂Cl₂ (3 mL), cooled to 0°C, and was treated with trifluoroacetic acid (3 mL). After stirring 10 min. at 0°C, the reaction was stirred at room temperature for 2.5 h. The solvents were evaporated and the residue was dissolved in water and was chromatographed on a short column (3X1.5 cm) of C-18 reverse phase silica gel eluting initially with water and then 5% acetonitrile/water. Product fractions were combined and evaporated. The residue was dissolved in water and lyophilized to afford guanidino-acid **252** (97 mg, 79%) as a white solid.

### Example 114

**Azido acid 260:** To a solution of methyl ester **227** (268 mg, 0.83 mmol) in THF (7.0 mL) was added aqueous KOH (1.60 mL of a 1.039 N solution) at room temperature. After stirring for 19 h at room temperature the reaction was acidified to pH 4.0 with Amberlite IR-120 (H⁺) acidic resin. The resin was filtered and washed with water and ethanol. Concentration *in vacuo* gave the crude azido acid **260** as a pale orange foam which was used for the next reaction without any further purification.

### Example 115

**Azido ethyl ester 261:** To a solution of carboxylic acid **260** (crude from previous reaction, assume 0.83 mmol), ethyl alcohol (150 µL), and catalytic DMAP in CH₂Cl₂ (6.0 mL) was added DCC (172 mg, 0.83 mmol) in one portion at room temperature. After several minutes a precipitate formed and after an additional 1 h of stirring the reaction was filtered and washed with CH₂Cl₂. Concentration *in vacuo* afforded a pale solid which was purified by flash chromatography on silica gel (50% hexanes in ethyl acetate) to give **272** mg (96%, small amount of DCU impurity present) of **261** as a white solid. When DCC was replaced by diisopropyl carbodiimide than the yield of 261 was 93% but the chromatographic purification eliminated urea impurities present when DCC was used.

### Example 116

**Amino ethyl ester 262:** Triphenyl phosphine (342 mg, 1.30 mmol) was added in one portion to a solution of **261** (272 g, 0.80 mmol) in THF (17 mL) and water (1.6 mL). The reaction was then heated at 50°C for 10 h, cooled and concentrated *in vacuo* to give a pale white solid. Purification of the crude solid by flash chromatography on silica gel (50% methanol in ethyl acetate) gave **242** mg (96%) of the amino ethyl ester **262** as a pale solid. The amino ethyl ester is dissolved in 3N HCl and lyophilized to give the corresponding water soluble HCl salt form. ¹H NMR (D₂O, 300 MHz): δ 6.84 (s, 1H); 4.36-4.30 (br m, 1H); 4.24 (q, 2H, *J* = 7.2 Hz); 4.05 (dd, 1H, *J* = 9.0,11.7 Hz); 3.63-3.50 (m, 2H); 2.95 (dd, 1H, *J* = 5.7,17.1 Hz); 2.57-2.45 (m, 1H); 1.60-1.39 (m, 4H); 1.27 (t, 3H, *J* = 7.2 Hz); 0.89-0.80 (m, 6H).

### Example 117

**bis-Boc guanidino ethyl ester 263:** Treated according to the procedure of Kim and Qian, "Tetrahedron Lett." 34:7677 (1993). To a solution of amine **262** (72 mg, 0.23 mmol), bis-Boc thiourea (66 mg, 0.24mmol) and Et₃N (108 µL) in dry DMF (600 µL) cooled to 0°C was added HgCl₂ (69 mg, 0.25mmol) in one portion. The heterogeneous reaction mixture was stirred for 1 h at 0°C and then at room temperature for 15 min, after which the reaction was diluted with EtOAc and filtered through a pad of celite. Concentration *in vacuo* followed by flash chromatography of the residue on silica gel (20% hexanes in ethyl acetate) gave 113 mg (89%) of **263** as a colorless foam. ¹H NMR (CDCl₃, 300 MHz): δ 11.41 (s, 1H); 8.65 (d, 1H, *J* = 8.1 Hz); 6.83 (s, 1H); 6.22 (d, 1H, *J* = 9.0 Hz); 4.46-4.34 (m, 1H); 4.21 (q, 2H, *J* = 6.9 Hz); 4.22-4.10 (m, 1H); 4.04-4.00 (m, 1H); 3.36 (quintet, 1H, *J* = 5.7 Hz); 2.78 (dd, 1H, *J* = 5.4, 17.7 Hz); 2.46-2.35 (m, 1H); 1.94 (s, 3H); 1.60-1.40 (m, 4H); 1.49 (s, 9H); 1.50 (s, 9H); 1.30 (t, 3H, *J* = 6.9 Hz); 0.93-0.84 (m, 6H).

### Example 118

**Guanidino ethyl ester 264:** To a solution of bis-Boc guanidnyl ethyl ester **263** (113 mg, 0.20 mmol) in CH₂Cl₂ (5.0 mL) cooled to 0°C was added neat trifluoroacetic acid (5.0 mL). The reaction mixture was stirred at 0°C for 30 min and then at room temperature for 1.5 h. The reaction was then concentrated *in vacuo* to give a pale orange solid which was purified by C₁₈ reverse phase chromatography eluting with water. Fractions containing the desired product were pooled and lyophilized to give 63 mg (66%) of the guanidine ethyl ester **264** as white solid. ¹H NMR (D₂O, 300 MHz): δ 6.82 (s, 1H); 4.35-4.31 (m, 1H); 4.24 (q, 2H, *J* = 7.1 Hz); 3.95-3.87 (m, 1H); 3.85-3.76 (m, 1H); 3.57-3.49 (m, 1H); 2.87 (dd, 1H, *J* = 5.1,17.7 Hz); 2.46-2.34 (m, 1H); 2.20 (s, 3H); 1.60-1.38 9M, 4H); 1.28 (t, 3H, *J* = 7.1 Hz); 0.90-0.80 (m, 6H).

### Example 122

Each of the reactions shown in **Table 50** were preformed according to **Scheme 50.** The preformed reactions are indicated with a " 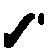 ". Unless otherwise indicated in **Table 50,** steps AA, AB and AC were preformed according to Examples 92, 93 and 94, respectively, and step AD was preformed according to the combination of Examples 112 and 113.

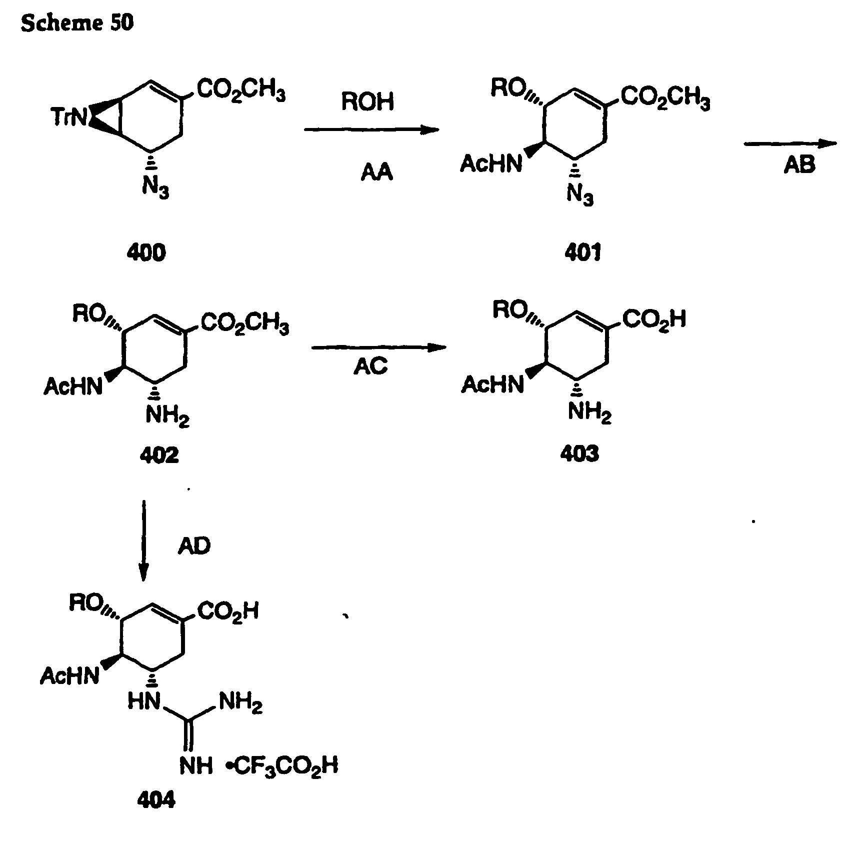

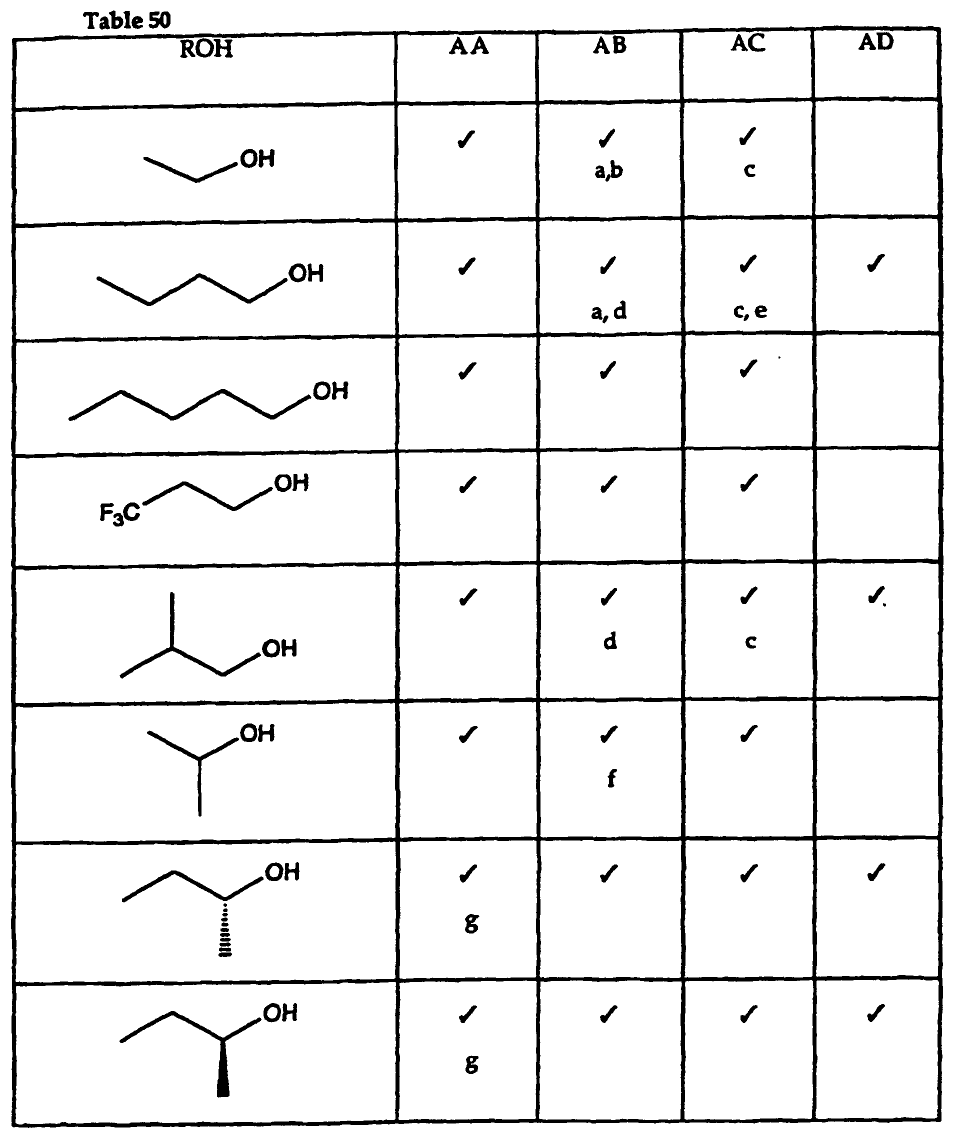

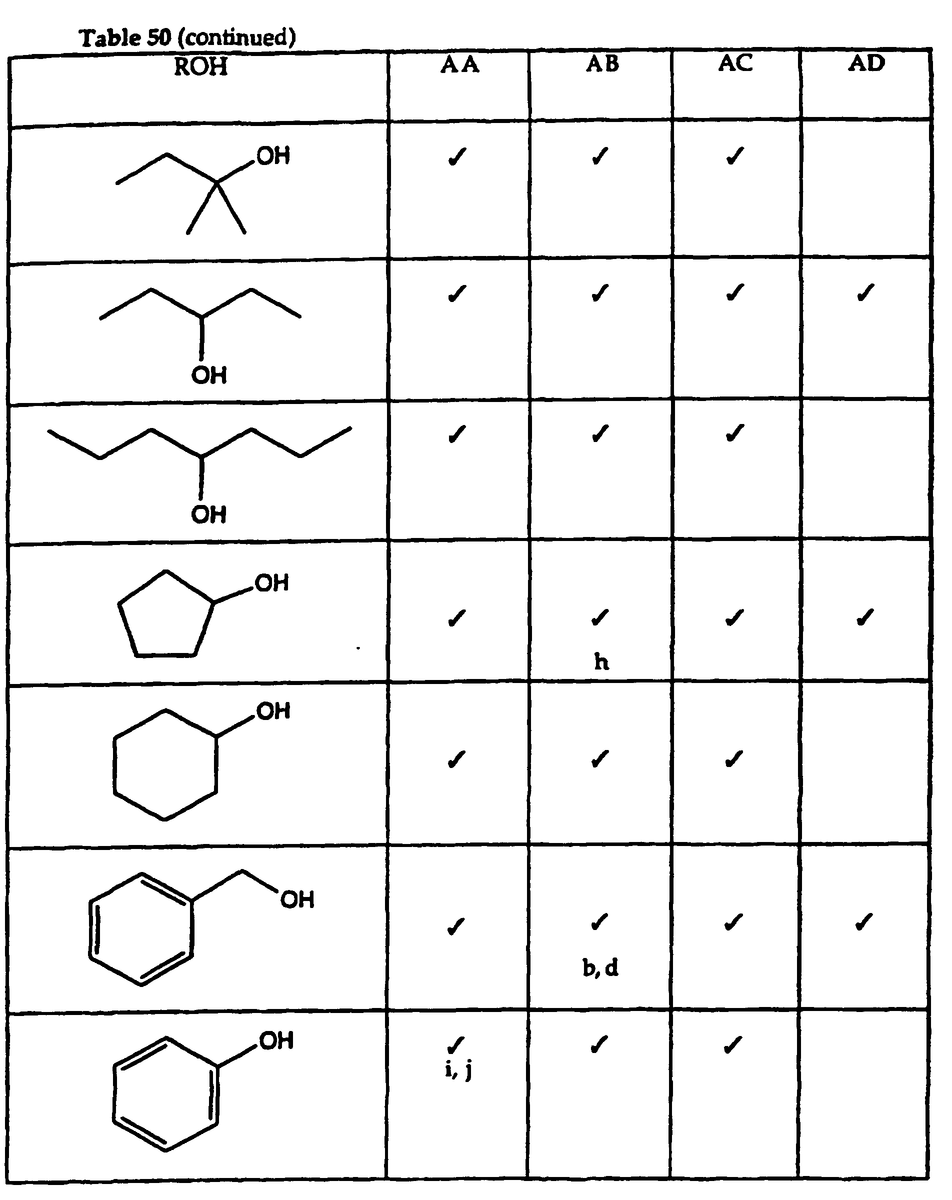

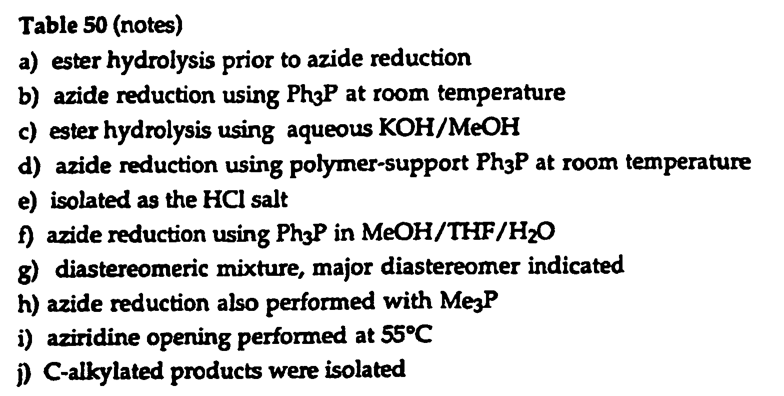

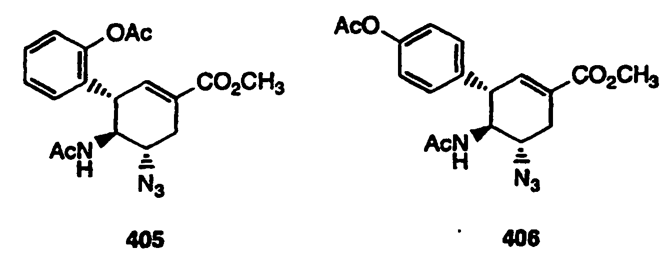

### Example 123

**Trifluroacetamide 340:** To a solution of amine **228** (100 mg, 0.34 mmol) in CH₂Cl₂ (3.5 mL) at 0 °C was added pyridine (41 µL, 0.51 mmol) and trifluroacetic anhydride (TFAA) (52 µL, 0.37 mmol) and the solution was stirred for 45 min at which time additional TFAA (0.5 eq) was added. After 15 min the reaction was evaporated under reduced pressure and the residue was partitioned between ethyl acetate and 1M HCL The organic phase was washed with saturated NaHCO₃, saturated NaCl, and was dried (MgSO₄), filtered, and evaporated. The residue was chromatographed on silica gel (2/1-hexane/ethyl acetate) to afford trifluoroacetamide **340** (105 mg, 78%): ¹H NMR (CDCl₃) δ 8.64 (d, 1H, *J* = 7.7), 6.81 (s, 1H), 6.48 (d, 1H, *J* = 8.2), 4.25-4.07 (m, 3H), 3.75 (s, 3H), 3.37 (m, 1H), 2.76 (dd, 1H, *J* = 4.5, 18.7), 2.54 (m, 1H), 1.93 (s, 3H), 1.48 (m, 4H), 0.86 (m, 6H).

### Example 124

**N-Methyl trifluoroacetamide 341:** To a solution of trifluroacetamide **340** (90 mg, 0.23 mmol) in DMF (2 mL) at 0 °C was added sodium hydride (10 mg, 60% dispersion in mineral oil, 0.25 mmol). After 15 min at 0 °C, methyl iodide (71 µL, 1.15 mmol) was added and the reaction was stirred for 2 h at 0 °C and for 1 h at room temperature. Acetic acid (28 µL) was added was the solution was evaporated. The residue was partitioned between ethyl acetate and water. The organic phase was washed with saturated NaCl, dried (MgSO₄), filtered, and evaporated. The residue was chromatographed on silica gel (1/1-hexane/ethyl acetate) to afford N-methyl trifluoroacetamide 341 (81 mg, 87%) as a colorless glass: ¹H NMR (CDCl₃) δ 6.80 (s, 1H), 6.26 (d, 1H, *J* = 9.9), 4.67 (m, 1H), 432 (m, 1H), 4.11 (m, 1H), 3.78 (s, 3H), 3.32 (m, 1H), 3.07 (br s, 3H), 2.60 (m, 2H), 1.91 (s, 3H), 1.48 (m, 4H), 0.87 (m, 6H).

### Example 125

**N-Methyl amine 342:** To a solution of N-methyl trifluoroacetamide **341** (81 mg, 0.20 mmol) if THF (3 mL) was added 1.04 N KOH (480 µL, 0.50 mmol) and the mixture was stirred at room temperature for 14 h. The reaction was acidified with IR 120 ion exchange resin to pH-4. The resin was filtered, washed with THF, and the filtrate was evaporated. The residue was dissolved in 10% TFA/water (5 mL) and was evaporated. The residue was passed through a column (1.5X2.5 cm) of C-18 reverse phase silica gel eluting with water. Product fractions were pooled and lyophilized to afford N-methyl amine **342** (46 mg, 56%) as a white solid: ¹H NMR (D₂O) δ 6.80 (s, 1H), 4.31 (br d, 1H, *J* = 8.8), 4.09 (dd, 1H, *J* = 8.9, 11.6), 3.53 (m, 2H), 2.98 (dd, 1H, *J* = 5.4, 16.9), 2.73 (s, 3H), 2.52-2.41 (m, 1H), 2.07 (s, 3H), 1.61-1.39 (m, 4H), 0.84 (m, 6H).

### Example 126

**Compound 346:** To a solution of epoxide **345** (13.32 g, 58.4 mmol) in 8/1-MeOH/H₂O (440 mL, v/v) was added sodium azide (19.0 g, 292.0 mmol) and ammonium chloride (2.69 g, 129.3 mmol) and the mixture was refluxed for 15h. The reaction was cooled, concentrated under reduced pressure and partitioned between EtOAc and H₂O. The organic layer was washed successively with satd. bicarb, brine and dried over MgSO₄. Concentration *in vacuo* followed by flash chromatography on silica gel (30% EtOAc in hexanes) gave 11.81 g (75%) of azido alcohol **346** as a viscous oil. ¹H NMR( 300 MHz, CDCl₃) δ 6.90-6.86 (m, 1H); 4.80 (s, 2H); 4.32 (bt, 1H, *J* = 4.2 Hz); 4.22 (q, 2H, *J* = 7.2 Hz); 3.90-3.74 (overlapping m, 2H); 3.44 (s, 3H); 2.90 (d, 1H, J = 6.9 Hz); 2.94-2.82 (m, 1H); 2.35-2.21 (m, 1H); 1.30 (t, 3H, *J* = 7.2 Hz).

### Example 127

**Compound 347:** To a solution of ethyl ester **346** (420 mg, 1.55 mmol) in dry THF (8.0 mL) cooled to -78 °C was added DIBAL (5.1 mL of a 1.0 M solution in toluene) dropwise via syringe. The bright yellow reaction mixture was stirred at -78 °C for 1.25 h and then slowly hydrolyzed with the slow addition of MeOH (1.2 mL). Volatiles were removed under reduced pressure and the residue partitioned between EtOAc and cold dilute HCl. The organic layer was separated and the aqueous layer back extracted with EtOAc. The organic layers were combined and washed successively with satd. bicarb, brine and dried over MgSO₄. Concentration *in vacuo* followed by flash chromatography on silica gel (20% hexanes in EtOAc) gave 127 mg (36%) of the diol **347** as a colorless viscous oil. ¹H NMR( 300 MHz, CDCl₃) δ 5.83-5.82 (m, 1H); 4.78 (s, 2H); 4.21 (bt, 1H, *J* = 4.4 Hz); 4.06 (bs, 2H); 3.85-3.65 (overlapping m, 2H); 3.43 (s, 3H); 3.18 (d, 1H, *J* = 8.1 Hz); 2.51 (dd, 1H, *J* = 5.5, 17.7 Hz); 2.07-1.90 (m, 1H); 1.92 (bs, 1H).

The following claims are directed to embodiments of the invention and shall be construed to cover in substantial variations thereof.

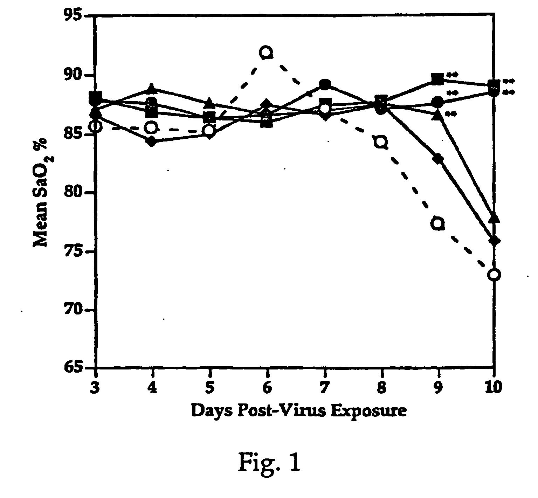

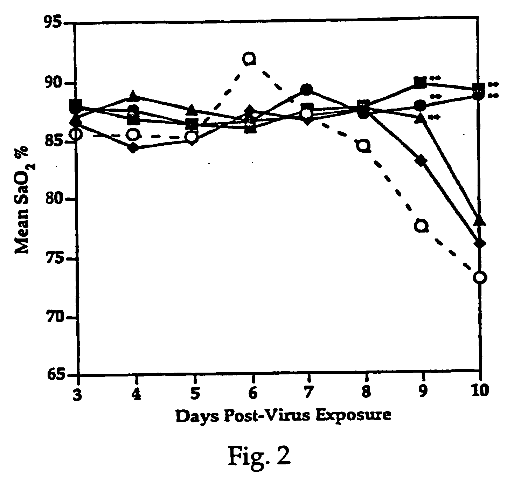

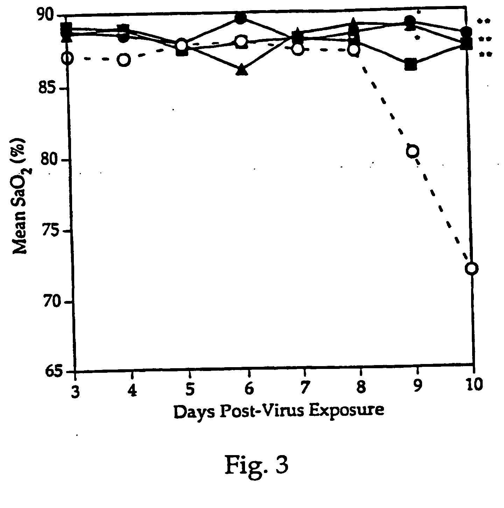

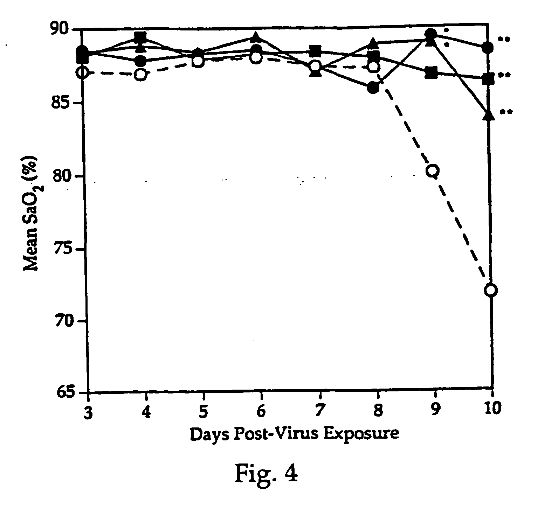

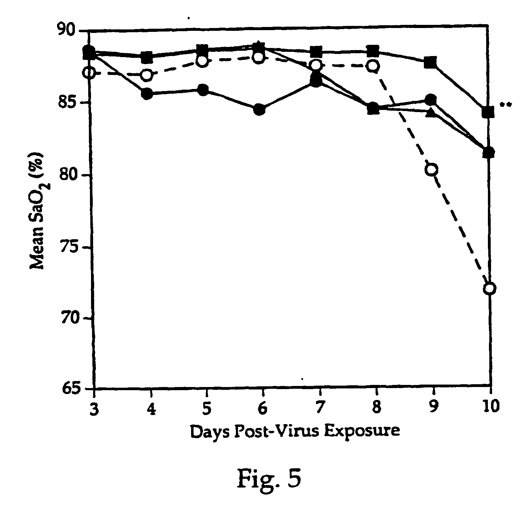

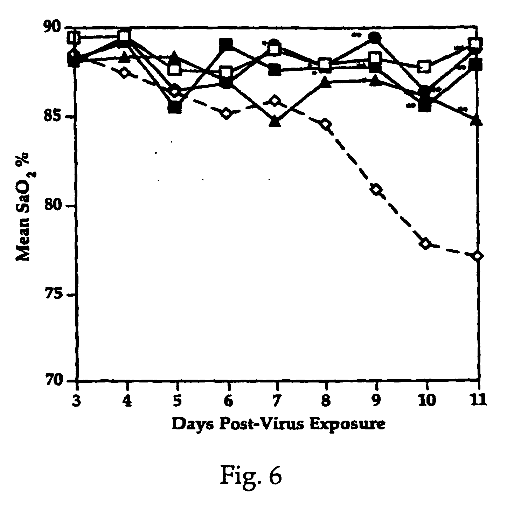

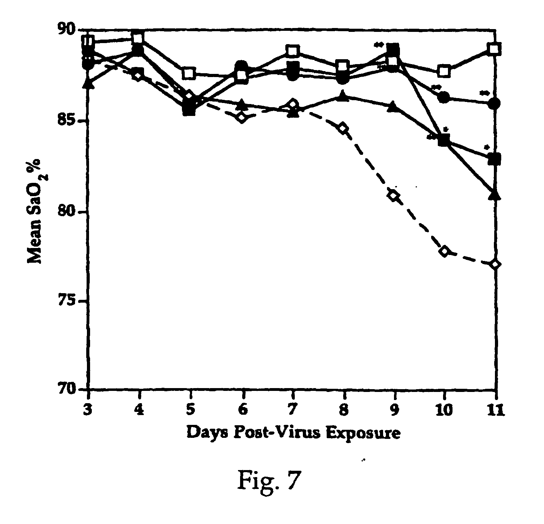

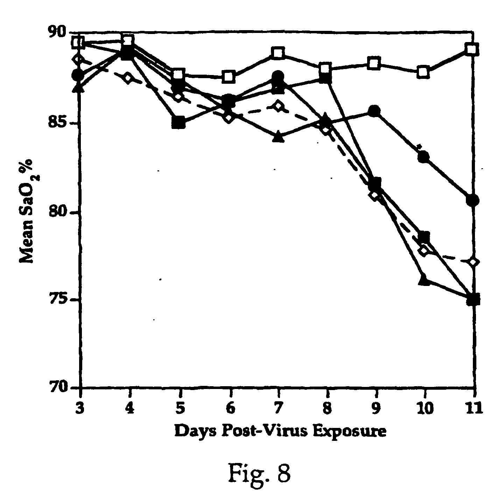

## Claims

1. A compound of the formula I: 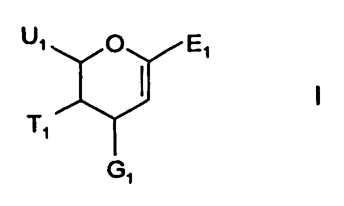 wherein
E₁ is COOH
G₁ is guanidino, amino, or guanidino or amino substituted with C₁-C₆alkyl;
T₁ is -NHCOCH₃, -NHCOCH₂F, -NHCOCHF₂, or -NHCOCF₃;
U₁ is -O-CH₂CH(R₁)W₇;
W₇ is CH₂OR₁; and
R₁ is C₄-C₁₂alkyl;
and the salts, solvates, resolved enantiomers and purified diastereomers thereof.

2. A pharmaceutical composition comprising a compound as defined in claim 1 and a pharmaceutically acceptable carrier.

3. A method of inhibiting the activity of neuraminidase comprising the step of contacting a sample suspected of containing neuraminidase with a compound as defined in claim 1.

4. The use of a compound as defined in claim 1 for preparing a pharmaceutical composition for the treatment or prophylaxis of influenza infection.

## Patentansprüche

1. Verbindung der Formel I: 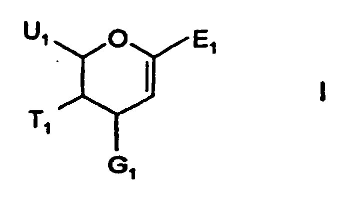 worin
E₁ für COOH steht,
G₁ für Guanidino, Amino oder für mit C₁-C₆-Alkyl substituiertes Guanidino oder Amino steht;
T₁ für -NHCOCH₃, -NHCOCH₂F, -NHCOCHF₂ oder -NHCOCF₃ steht;
U₁ für -O-CH₂CH(R₁)W₇ steht;
W₇ für CH₂OR₁ steht; und
R₁ für C₄-C₁₂-Alkyl steht;
und die Salze, Solvate, aufgetrennten Enantiomere und gereinigten Diastereomere davon.

2. Pharmazeutische Zusammensetzung, umfassend eine wie in Anspruch 1 definierte Verbindung und einem pharmazeutisch akzeptablen Träger.

3. Verfahren zur Inhibierung der Aktivität von Neuraminidase, umfassend den Schritt, bei dem man eine Probe, von der man annimmt, dass sie Neuraminidase enthält, mit einer wie in Anspruch 1 definierten Verbindung in Kontakt bringt.

4. Verwendung einer wie in Anspruch 1 definierten Verbindung zur Herstellung einer pharmazeutischen Zusammensetzung zur Behandlung oder Prophylaxe von Influenza-Infektionen.

## Revendications

1. Un composé de la formule I: 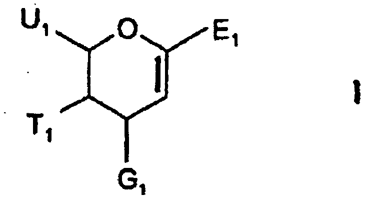 dans laquelle
E₁ est COOH
G₁ est guanidino, amino ou guanidino ou amino substitué avec alkyle en C₁-C₆;
T₁ est-NHCOCH₃, -NHCOCH₂F, -NHCOCHF₂, ou-NHCOCF₃;
U₁ est -O-CH₂CH(R₁)W_{7;} et
W₇ est CH₂OR₁; et
R₁ est alkyl en C₄-C₁₂;
ainsi que leurs sels, produits solvatés, énantiomères décomposés et diastéréomères purifiés.

2. Une composition pharmaceutique comprenant un composé tel que défini à la revendication 1 et un support pharmaceutiquement acceptable.

3. Un procédé pour inhiber l'activité de la neuraminidase comprenant l'étape consistant à mettre en contact un échantillon suspecté de contenir de la neuraminidase avec un composé tel que défini à la revendication 1.

4. L'utilisation d'un composé tel que défini à la revendication 1 pour préparer une composition pharmaceutique pour le traitement ou la prophylaxie d'une infection grippale.
